# EUROPEAN PATENT SPECIFICATION

(11) **EP 1 036 065 B1**
(45) Date of publication and mention of the grant of the patent: **12.05.2004**
(21) Application number: 98962883.9
(22) Date of filing: 03.12.1998
(51) Int. Cl.: C07D 233/56, A61K 31/415, C07D 401/06, C07D 403/06, C07D 409/06, C07D 405/06, C07D 233/84, C07D 413/12, C07D 495/04

(54) **SUBSTITUTED IMIDAZOLE DERIVATIVES HAVING AGONIST-LIKE ACTIVITY AT ALPHA 2B OR 2B/2C ADRENERGIC RECEPTORS**
SUBSTITUIERTE IMIDAZOLE DERIVATE MIT AGONISTISCHÄHNLICHER WIRKUNG AUF DIE ALPHA 2B ODER 2B/2C ADRENERGISCHEN REZEPTOREN
DERIVES IMIDAZOLES SUBSTITUES AYANT UNE ACTIVITE DE TYPE AGONISTE VIS A VIS DES RECEPTEURS ADRENERGIQUES ALPHA 2B OU 2B/2C

(30) Priority: 04.12.1997 US 985347
(43) Date of publication of application: 20.09.2000
(60) Divisional application: 04001074.6
(73) Proprietor: Allergan, Inc., Irvine, CA 92612 (US)
(72) Inventor: CHOW, Ken, Newport Coast, CA 92657 (US); GIL, Daniel, W., Corona Del Mar, CA 92625 (US); BURKE, James, A., Santa Ana, CA 92705 (US); HARCOURT, Dale, A., San Clemente, CA 92672 (US); GARST, Michael, E., Newport Beach, CA 92660 (US); WHEELER, Larry, A., Irvine, CA 92715 (US); MUNK, Stephen, A., Northville, MI 48167 (US)
(74) Representative: HOFFMANN - EITLE
(86) International application number: PCT/US1998/025669
(87) International publication number: WO 1999/028300

(56) References cited:
- EP-A- 0 194 984
- EP-A- 0 304 910
- WO-A-94/07866
- WO-A-97/12874
- WO-A-97/35858
- US-A- 5 621 113
- CHEMICAL ABSTRACTS, vol. 112, no. 15, 9 April 1990 Columbus, Ohio, US; abstract no. 139033n, page 721; XP002098179 & JP 01 242571 A (MITSUI PETROCHEMICAL INDUSTRIES LTD.) 27 September 1989

## Description

### 1. Field of the Invention

The present invention is directed to the use of compounds which are selective agonists of the α2B alone or α2B and α2C adrenergic receptor subtypes and which lack substantial activity at the α2A receptor subtype for the treatment of glaucoma or elevated intraocular pressure and other diseases with substantially reduced cardiovascular or sedative side effects by administering them to mammals including humans.

### 2. Brief Description of the Prior Art

Compounds which have adrenergic activity are well known in the art, and are described in numerous United States and foreign patents and in scientific publications. It is generally known and accepted in the art that adrenergic activity is useful for treating animals of the mammalian species, including humans, for curing or alleviating the symptoms and conditions of numerous diseases and conditions. In other words, it is generally accepted in the art that pharmaceutical compositions having an adrenergic compound or compounds as the active ingredient are useful for treating glaucoma, chronic pain, nasal congestion, high blood pressure, congestive heart failure and inducing anesthesia.

The two main families of adrenergic receptor are termed alpha adrenergic receptors and beta adrenergic receptors in the art, and each of these two families is known to have subtypes, which are designated by letters of the alphabet, such as α2A, α2B. See the article by Bylund et al, *Pharmacol Rev*. 46, pp. 121-136(1994).

### SUMMARY OF THE INVENTION

It has been discovered in accordance with the present invention that adrenergic compounds which act selectively, and preferably even specifically as agonists of the α2B or α2B / α2C (hereinafter referred to as α2B or α2B/2C) receptor subtypes in preference over the α2A receptor subtype, possess desirable therapeutic properties associated with adrenergics but without having one or more undesirable side effects such as changes in blood pressure or sedation. For the purposes of the present invention, a compound is defined to be a specific or at least selective agonist of the α2B or α2B/2C receptor subtype(s) if the compound is at least approximately ten times more potent as an agonist at either the α2B and α2C or both receptor subtypes than at the α2A receptor subtype, or if the difference in the compound's efficacy at the α2B and α2B/2C receptor relative to the α2A receptor is greater than 0.3 and its efficacy at the α2A receptor is ≤ 0.4.

Accordingly, the present invention relates to the use of a pharmaceutical composition comprising one or more specific or selective α2B or α2B/2C adrenergic agonist compounds as the active ingredient, for treatment of the many diseases or conditions against which alpha adrenergic compounds are useful, including glaucoma, reducing elevated intraocular pressure, chronic pain, diarrhea, and nasal congestion by administering the pharmaceutical composition to animals of the mammalian species, including humans. In addition, the compounds used in this invention are useful for treating muscle spasticity including hyperactive micturition, diarrhea, diuresis, withdrawal syndromes, pain including neuropathic pain, neurodegenerative diseases including optic neuropathy, spinal ischemia and stroke, memory and cognition deficits, attention deficit disorder, psychoses including manic disorders, anxiety, depression, hypertension, congestive heart failure, cardiac ischemia and nasal congestion.

The present invention particularly covers the use of the compounds of the invention for the treatment of diseases and conditions where adrenergic compounds are effective for treatment, but their use is limited because of their generally known side effects.

### DETAILED DESCRIPTION OF THE INVENTION

Compounds which are used in the present invention are selective or specific agonists of the α2B or α2B/2C adrenergic receptor subtypes, in preference over the α2A receptor subtype. In accordance with the present invention, a compound is considered a selective α2B or α2B/2C agonist if that compound's difference in efficacy as an agonist of the α2B or α2B/2C receptor subtype(s) compared to the α2A receptor subtype is greater than 0.3 and its efficacy at the α2A receptor subtype is ≤ 0.4 and/or it is at least approximately 10 times more potent Preferably, the compounds utilized in accordance with the present invention are specific agonists of the α2B or α2B/2C receptor subtypes. Specifically, in this regard, a specific agonist is defined in the sense that a specific α adrenergic agonist does not act as an agonist of the α2A receptor subtype to any measurable or biologically significant extent.

A set of agents has been discovered that are functionally selective for the α2B or α2B/2C - subtypes of said adrenergic receptors. This preferential activity can be determined in a variety of functional assays such as Cyclic AMP Production, Shimizu et al, *J*. *Neurochem*. **16**, pp. 1609-1619 (1969); R-SAT (Receptor Selection and Amplification Technology), Messier et al, *Pharmacol. Toxicol.* **76,** pp. 308-311(1995) and the Cytosensor microphysiometer, Neve et al, *J*. *Biol. Chem*. **267,** pp. 25748-25753, (1992) using cells that naturally express individual subtypes or have had one of the subtypes introduced. The cells or recombinant receptors used should be human or from a species that has been shown to have a similar pharmacology. In the study below, the RSAT assay on cells that have been transiently transfected with the human α2A (c10 gene), rat α2B (RNG gene) and human α2C (c4 gene) receptors was used. The rat α2B receptor has been shown to have a pharmacology that corresponds to the human α2B receptor (see, for example, Bylund et al., *Pharmocol, Rev*. **46,** pp. 127-129(1994)).

In the treatment of glaucoma, particularly, topical administration may be used. Any common topical formulation such as a solution, suspension, gel, ointment, or salve and the like may be applied to the eye in glaucoma and dermally to treat other indications. Preparation of such topical formulations are well described in the art of pharmaceutical formulations as exemplified, for example, by Remington's Pharmaceutical Science, Edition 17, Mack Publishing Company, Easton, Pennsylvania.

If the drug is to be administered systemically, it may be confected as a powder, pill, tablet or the like or as a syrup or elixir for oral administration. For intravenous, intraperitoneal, intrathecal or epidural administration, the compound will be prepared as a solution or suspension capable of being administered by injection. In certain cases, it may be useful to formulate these compounds in suppository or as an extended release formulation, including the dermal patch form, for deposit on or under the skin or for intramuscular injection.

Treatment of glaucoma or any other indications known or discovered to be susceptible to treatment by adrenergic compounds will be effected by administration of therapeutically effective dose of one or more compounds in accordance with the instant invention. A therapeutic concentration will be that concentration which effects reduction of the particular condition, or retards its expansion. In certain instances, the drug potentially could be used in a prophylactic manner to prevent onset of a particular condition. A given therapeutic concentration will vary from condition to condition and in certain instances may vary with the severity of the condition being treated and the patient's susceptibility to treatment. Accordingly, a given therapeutic concentration will be best determined at the time and place through routine experimentation. However, it is anticipated that in the treatment of, for example, glaucoma, that a formulation containing between 0.001 and 5 percent by weight, preferably about 0.01 to 3% will usually constitute a therapeutically effective concentration. If administered systemically, an amount between 0.001 and 50 mg per kg, preferably between 0.001 and 10 mg per kg body weight per day, but most preferably about 0.01 to 1.0 mg/kg, will effect a therapeutic result in most instances.

Because the α2B and α2B/2C specific selective agonist compounds lack substantial α2A side effects, treatments of diseases or conditions with such compounds in accordance with the present invention is advantageous, particularly when the treatment is directed to a human having cardiovascular problems.

The general structures of exemplary specific α2B and α2C agonist or selective α2B and α2B/2C agonist adrenergic compounds which are used in the present invention are provided by general Formulas, below.

In one aspect of the invention, a compound having selective agonist activity at the α2B or α2B/2C adrenergic receptor subtype(s) as compared to the 2A adrenergic receptor subtype is represented by the general formula 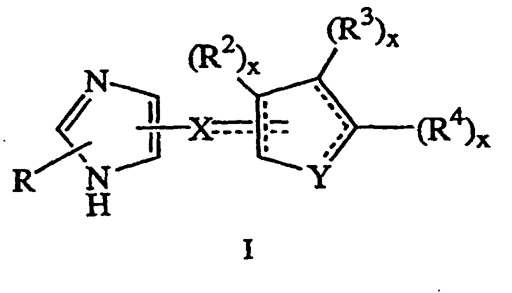 wherein the dotted lines represent optional bonds provided that two double bonds may not share a common carbon atom; R is H or lower alkyl; X is S or C(H)R¹, wherein R¹ is H or lower alkyl, but R¹ is absent when the bond between X and the ring represented by 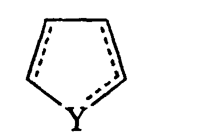 is a double bond; Y is O, N, S, (CR¹₂)_{y}, wherein y is an integer of from 1 to 3, -CH=CH- or -Y¹CH₂-, wherein Y¹ is O, N or S; x is an integer of 1 or 2, wherein x is 1 when R², R³ or R⁴ is bound to an unsaturated carbon atom and x is 2 when R², R³ or R⁴ is bonded to a saturated carbon atom; R² is H, lower alkyl, halogen, hydroxy or lower alkoxy, or, when attached to a saturated carbon atom, R₂ may be oxo; R₃ and R₄ are, each, H, lower alkyl, hydroxy, lower alkoxy, or phenyl or, together, are -(C(R²)x)z-; -Y¹(C(R²)x)z'-; - Y¹ (C(R²)x)y Y¹-; -(C(R²)x)- Y¹-(C(R²)x)-; - (C(R²)x)- Y¹-(C(R²)x)-(C(R²)x)- and - Y¹-(C(R²)x)- Y¹-(C(R²)x)- wherein z is an integer of from 3 to 5, z' is an integer of from 2 to 4 and x and y are as defined above, and further either end of each of these divalent moieties may attach at either R3 or R4 to form a condensed ring structure shown generally as 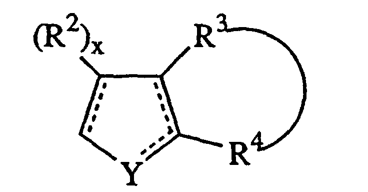 and the rings formed may be totally unsaturated, partially unsaturated, or totally saturated provided that a ring carbon has no more than 4 valences, nitrogen no more than three and O and S have no more than two.

In another aspect of the invention in the above compound is represented by the formula 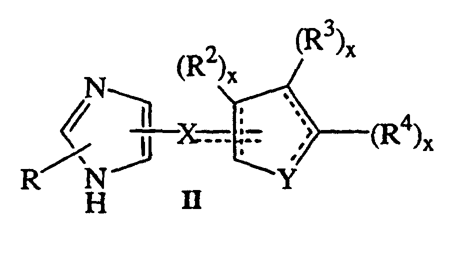 wherein X may be C(H)R¹ and R¹ is H.

In said compound of formula II, R₂ may be H and 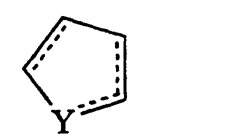 may represent a furanyl radical.

In such furanyl derivatives of Formula II, R³ and R⁴ together may be (CH)₄, or R³ may be H and R⁴ may be t-butyl, or R³ and R⁴ may be H, or R³ may be H and R⁴ may be methyl or ethyl.

Alternatively, in the compound of Formula I, R' may be methyl and 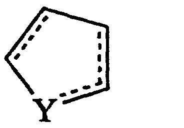 may represent a furanyl radical.

Alternatively, in said compounds of Formula II, R² may be H and 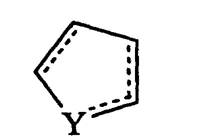 may represent a thienyl radical.

In such thienyl derivatives of Formula II, R³ and R⁴, together, may represent (CH₂)₄, or R³ may be phenyl and R⁴ may be H, or R³ and R⁴, together, may represent (CH₂)₃S, or R³ and R⁴ may be H, or R³ and R⁴, together, may represent (CH)₄, or may be R³ may be H and R⁴ may be methyl, or R³ may be bromo and R⁴ may be H, or R³ may be hydrogen and R⁴ may be chloro, or R³ may be methyl and R⁴ may be hydrogen.

Alternatively, in the compounds of Formula II 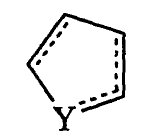 may represent a cyclohexyl radical.

In such cyclohexyl derivatives of Formula II, R² may be hydrogen and R³ and R⁴ may, together, represent (CH)₄, or R² may be oxo and R³ and R⁴, together, may be (CH)₄, or R² may be hydrogen or oxo and R³ and R⁴, together, may represent (CH)₂S, or R² may be hydrogen and R³ and R⁴ may, together, represent (CH₂)₄, forming an octahydronaphthalene, or R² may be oxo and R³ and R⁴ may, together, represent (CH₂)₄, or R² may be oxo and R³ and R⁴, together, may represent (CH)₂C(CH₃)(CH), or R² may be hydrogen and R³ and R⁴, together, may represent S(CH₂)₂, or R², R³ and R⁴ may be H, or R² may be oxo and R³ and R⁴, together, may represent (CH)₂C(OCH₃)CH, or R³ and R⁴ together may represent -Y¹-C(R₂)ₓ-C(R₂)ₓ-Y¹-wherein Y¹ is N, forming a tetrahydroquinoxaline wherein R² may be hydrogen or oxo. Alternatively, in the compounds of Formula II 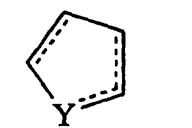 may represent a tetrahydroquinoline radical wherein R³ and R⁴ together are -Y¹-C(R₂)ₓ-C(R₂)ₓ-C(R₂)ₓ- wherein Y¹ is N. In such tetrahydroquinoline derivatives (R²)ₓ may be hydrogen or oxo; or may represent a tetrahydroisoquinoline radical wherein R³ and R⁴ together are -C(R₂)ₓ-Y¹-C(R₂)ₓ-C(R₂)ₓ-wherein Y¹ is N and (R²)ₓ may be hydrogen or oxo.

Alternatively, in the compounds of Formula II 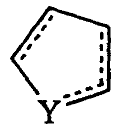 may represent a cyclopentyl radical.

In such cyclopentyl derivatives of Formula II, R² may be H and R³ and R⁴, together, may represent (CH)₄, or R² may be oxo and R³ and R⁴, together, may represent (CH)₄, or R² may be hydrogen and R³ and R⁴, together, may represent (CH₂)₃.

In another aspect of the invention, Y is (CH₂)₃ and X may be CH and R² may be oxo or X may be CH₂ and R² may be H. Alternatively, R³ and R⁴, together, may represent (CH)₄, Y may be CH₂C(CR¹₂)₂ wherein R¹ is hydrogen, or Y may be -CH2C(Me)- and R² may be hydrogen or oxo.

Finally, in the compounds of Formula II 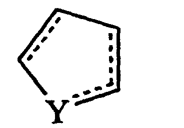 may represent a phenyl radical.

In such phenyl derivatives of Formula I, X may be CH₂, R maybe H or CH₃, R², R³ and R⁴ may be H, or R³ and R⁴, together, represent O(CR²)₂O to provide a 1,4-benzodioxan derivative, or alternatively, X may be S and R², R³ and R⁴ may be H.

In another aspect of the invention, said compound has the formula 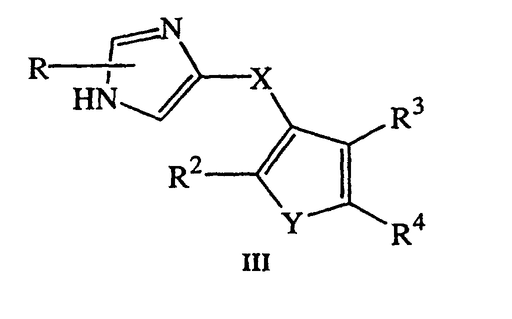 wherein Y is S or O.

In such compound of Formula III, X may be C(H)R¹, R, R¹, R², R³ and R⁴ may be H and Y may be O or S.

In another aspect of the invention, said compound has the formula 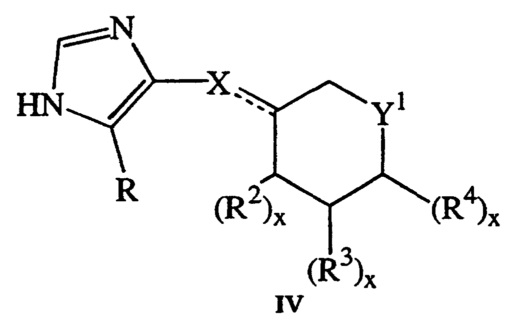 and R³ and R⁴, together, represent (CH)₄.

In such compounds of Formula IV, Y¹ may be O, R² may be oxo and X is CH or CH₂, or one of R² is hydroxy and the other may be H, or R² may be H.

In such compounds of Formula IV, Y¹ may be S, X may be CH₂ and R² may be oxo, or R² may be H and X may be CH and R² may be oxo.

In another aspect of the invention, the compound having selective activity at the 2B or 2B and 2C adrenergic receptor subtype(s) as compared to the 2A adrenergic receptor subtype is represented by the formula 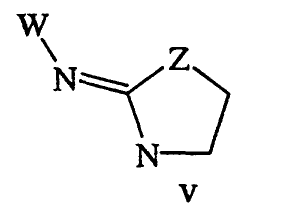 alternatively W is a bicyclic radical selected from the group consisting of 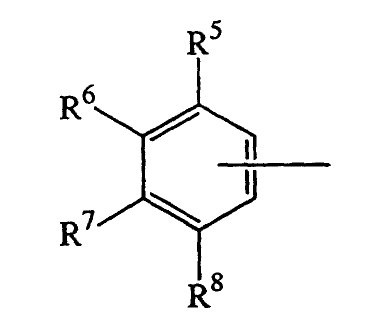 wherein R⁵, R⁶, R⁷ and R⁸ are selected from the group consisting of H and lower alkyl provided that at least one of R⁵ and R⁶ or R⁶ and R⁷ are OC(R⁹)C(R⁹)N(R) to form a condensed ring with 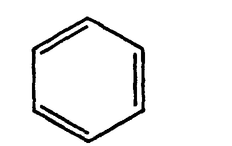 wherein R⁹ is H, lower alkyl or oxo;
and 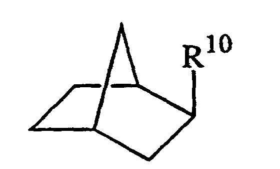 wherein R¹⁰ is H, lower alkyl, phenyl or lower alkyl substituted phenyl, and Z is O or NH. Compounds wherein W is norbornyl are disclosed and claimed in commonly assigned co-pending application 09/003902, filed on 7 January, 1998, which is hereby incorporated by reference in its entirety.

In one aspect of the invention Z may be O and W may be 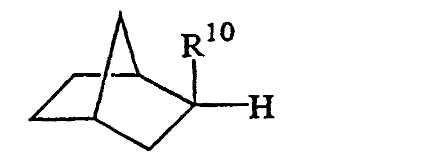 and R¹⁰ may be selected from the group consisting of H, phenyl and o-methylphenyl, e.g. R¹⁰ may be o-methylphenyl.

In another aspect of the invention W may be 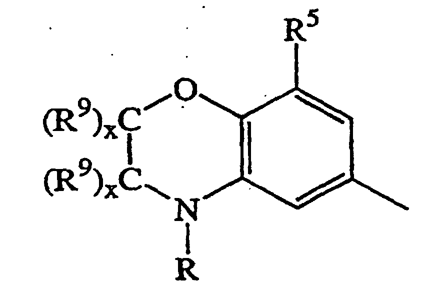 wherein Z may be NR, R may be methyl or hydrogen, one of (R⁹)ₓ may be H and R⁵ may be H.

Alternatively, W may be 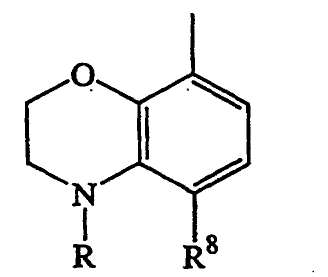 wherein R may be H and R⁸ may be methyl.

The invention is further illustrated by the following examples (including general synthetic schemes therefore).

### Example A

### Synthesis of 1-dimethylsulfamoyl-2-t-butyldimethylsilyl-5-imidazolecarboxaldehyde:

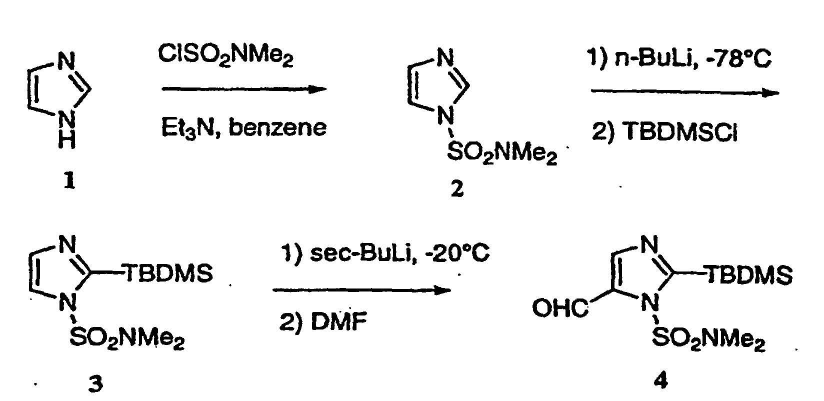

### Procedure -

Imidazole (1) (20.0g, 0.29 mol), triethylamine (41.0mL, 0.29 mol) and N,N-dimethylsulfamoyl chloride (31.6mL, 0.29 mol) were added to 320mL of benzene. The reaction was stirred for 48h at room temperature (rt) and then filtered. The filtrate was collected and concentrated under reduced pressure. Vacuum distillation of the crude product (∼0.5 mmHg, 115°-118°C) afforded 38.7g (76%) of a clear and colorless oil. Upon cooling the product solidifies to give white crystals (2). 1-(Dimethylsulfamoyl) imidazole (2) (18.8g, 0.11 mol) was added to 430mL of tetrahydrofuran (THF). The solution was cooled to 78° C. A solution of n-butyl lithium (n-BuLi) in hexane (1.6M, 70.9 mL, 0.11 mol) was added dropwise to the reaction flask. Upon completion, the reaction was stirred for 1h at -78°C. t-Butyldimethylsilylchloride (17.8g, 0.12 mol) in 50mL of THF was added via cannula to the reaction. After the addition was completed the reaction mixture was warmed slowly to rt and then stirred for 24h. The reaction was diluted with water and the organic layer separated. The organic phase was washed with brine and then dried over sodium sulfate. The mixture was filtered and the filtrate concentrated under reduced pressure. Column chromatography (20% ethyl acetate/ hexane as eluant) afforded a light yellow solid. Recrystallization from pentane gave 30g (94%) of white crystals (3).

1-Dimethylsulfamoyl-2-t-butyldimethylsilyl imidazole (3) (5.0g, 17.3 mmol) was added to 100mL of THF. The solution was cooled to -20°C. A solution of secondary butyl lithium (s-BuLi) in hexane (1.3M, 14.6mL, 19 mmol) was added dropwise to the reaction flask. Upon completion the reaction was stirred for 1h at -20°C. 8 mL of dimethylformamide (DMF) was added to the reaction and then stirred at rt for 3.5h. The reaction was diluted with water and the organic layer separated. The organic phase was washed with brine and then dried over sodium sulfate. The mixture was filtered and the filtrate concentrated under reduced pressure. Column chromatography (20% ethyl acetate/ hexane) afforded a light yellow oil. Upon cooling the product solidifies to give yellow crystals of 1-dimethylsulfamoyl-2-t-butyldimethylsilyl-5- imidazolecarboxaldehyde (4).

### Example B-1

### Procedure for Preparation of 4(5)-(7-methoxy-1,2,3,4-tetrahydronaphthalen-2-ylmethyl)-1H-imidazole, hydrogen chloride salt:

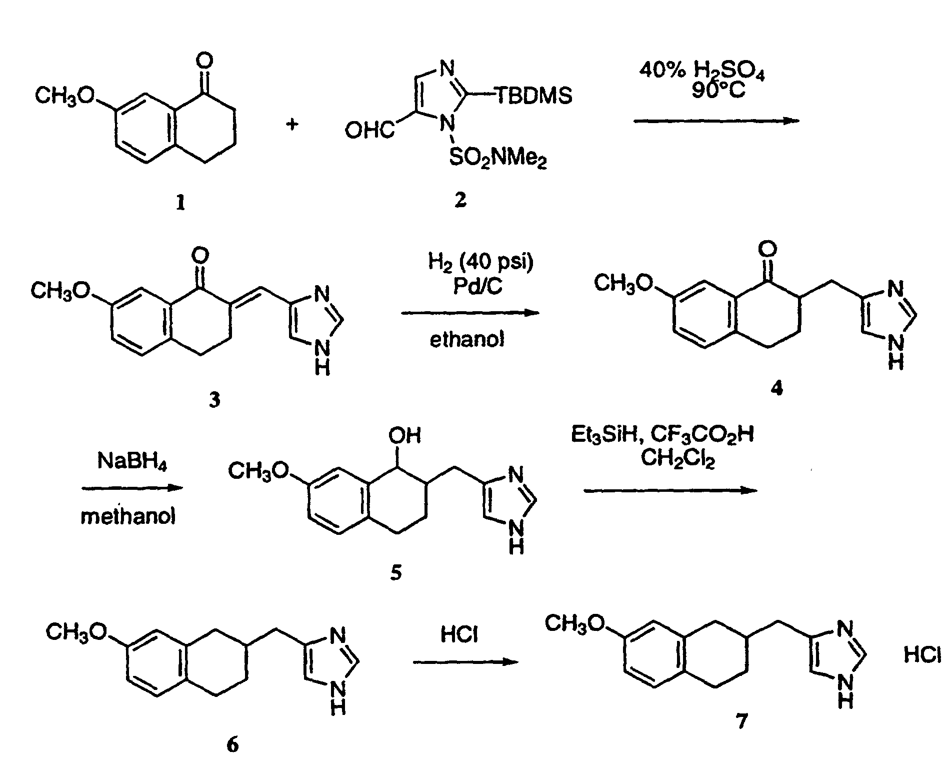

### Procedure -

7-Methoxy-1-tetralone (1) (1.5g, 8.5 mmol) and 1-dimethylsulfamoyl-2-t-butyldimethylsilyl-5- imidazolecarboxaldehyde (2) (2.7g, 8.5 mmol) were added to 8.5 mL of a 40% solution of sulfuric acid. The reaction was heated for 24h at 90°C. After cooling to rt, the reaction was made basic with excess concentrated ammonium hydroxide. The mixture was extracted twice with THF. The organic layers were combined and washed with brine. The organic layer was separated and dried over sodium sulfate. The mixture was filtered and the filtrate concentrated under reduced pressure to afford 2.7g of a yellow solid (3) comprising 3-(3H-imidazole-4(5)ylmethylene)-7-methoxy chroman-4-one. The crude product was suspended in 100mL of ethanol and a palladium on carbon catalyst (10%, 0.27g) added. The mixture was shaken in a Parr hydrogenator apparatus while under 40 psi of hydrogen. After 19h the reaction mixture was filtered through Celite and the filtrate concentrated under reduced pressure. Column chromatography with 7% methanol in chloroform afforded 1.05g (46%) of a tan color solid comprising 2-[3H-Imidazole-4(5)-ylmethyl]-7-methoxy-3,4-dihydro-2H-naphthalen-1-one (4)(B-1a). (4) (0.5g, 1.95 mmol) was added to 20mL of methanol. Sodium borohydride (74mg, 1.95 mmol) was added to the solution. After stirring for 2.5h at rt the reaction mixture was quenched with water. The reaction mixture was then extracted twice with ethyl acetate. The organic layers were combined and washed with brine. The organic layer was separated and dried over sodium sulfate. The mixture was filtered and the filtrate concentrated under reduced pressure to afford 0.5g of a white solid (5) comprising 2-[3H-Imidazole-4(5)-ylmethyl]-7-methoxy-3,4-dihydro-2H-naphthalen-1-ol. The crude product was dissolved in 26mL of dichloromethane. Triethylsilane (2.5mL, 15.6 mmol) and trifluoroacetic acid (4.8mL, 62.3 mmol) were added and the reaction stirred at rt for 22h. The reaction was made basic with 2N NaOH and the organic layer separated and washed with brine. The solution was dried over sodium sulfate. The mixture was filtered and the filtrate concentrated under reduced pressure. Column chromatography with 7% methanol in chloroform afforded 0.39g (83%) of a tan color oil (6). The product was dissolved in methanol and an excess of hydrogen chloride (HCl) in ether was added. The solution was concentrated under reduced pressure to yield 0.3g of a tan color solid. Column chromatography with 7% methanol in chloroform afforded 0.25g (46%) of 4(5)-(7-methoxy-1,2,3,4-tetrahydronaphthalen-2-ylmethyl)-1H-imidazole, hydrogen chloride salt (B-1) as white crystals (7) after recrystallization from a mixture of acetone and methanol.
^{**1**}**H NMR** (300 MHz, CD₃OD) 8.83 (s, 1H), 7.38 (s, 1H), 6.95 (d, 1H, J=8.5Hz), 6.66 (d, 1H, J=8.4Hz), 6.57 (s, 1H), 3.73 (s, 3H), 2.71-2.81 (m, 5H), 2.43-2.52 (m, 1H), 1.90-2.14 (m, 2H), 1.40-1.51 (m, 1H).

Following the procedure of Example B-1 various fused ring compounds are reacted to yield the imidazole derivatives listed below.

### Example B-2(a-d)

| | | |
|---|---|---|
| 4-chromanone | **(2a)** | 3-(3H-imidazol-4(5)-ylmethylene)chroman-4-one |
| | **(2b)** | 3-(3H-imidazol-4(5)-ylmethyl)chroman-4-one |
| | **(2c)** | 3-(3H-imidazol-4(5)-ylmethyl)chroman-4-ol |
| | **(2d)** | 4(5)-chroman-3-ylmethyl-1H-imidazole |

### Example B-3(a-b)

| | | |
|---|---|---|
| 1-tetralone | **(3a)** | 2-(3H-imidazol-4(5)-ylmethyl)-3,4-dihydro-2H-naphthalen-1-one |
| | **(3b)** | 4(5)-(1,2,3,4-tetrahydronaphthalen-2-ylmethyl)-1H-imidazole |

### Example B-4(a-b)

| | | |
|---|---|---|
| 4-methyl-1-tetralone | **(4a)** | 4(5)-(4-methyl-1,2,3,4-tetrahydronaphthalen-2-ylmethyl)-1H-imidazole |
| | **(4b)** | 2-(3H-imidazol-4(5)-ylmethyl)-4-methyl-3,4-dihydro-2H-naphthalen-1-one |

### Example B-5(a-b)

| | | |
|---|---|---|
| Thiochroman | **(5a)** | 3-(3H-imidazol-4(5)-ylmethylene)thiochroman-4-one |
| | **(5b)** | 3-(3H-imidazol-4(5)-ylmethyl)thiochroman-4-one |

### Example B-6

The hydrogen chloride salt of the previous compound is prepared by step 5 of the method of Example B-1, above.

| | |
|---|---|
| Thiochroman | 4(5)-thiochroman-3-ylmethyl-1H-imidazole |

### Example B-7(a-c)

| | | |
|---|---|---|
| 1-indanone | **(7a)** | 2-(3H-imidazol-4(5)-ylmethylene)indan-1-one |
| | **(7b)** | 2-(3H-imidazole-4(5)-ylmethyl)indan-1-one |
| | **(7c)** | 4(5)-indan-2-ylmethyl-1H-imidazole |

### Example B-8(a-b)

| | | |
|---|---|---|
| 7-methyl-1-tetralone | **(8a)** | 2-(3H-imidazol-4(5)-ylmethyl)-7-methyl- 3,4-dihydro-2H-naphthalen-1-one |
| | **(8b)** | 4(5)-(7-methyl-1,2,3,4-tetrahydronaphthalen-2-ylmethyl)-1H-imidazole |

The hydrogen chloride salt of this compound is prepared by the method of Example B-6.

### Example B-9(a-c)

| | | |
|---|---|---|
| 4-keto-4,5,6,7-tetrahydrothianaphthene | **(9a)** | 4(5)-(4,5,6,7-tetrahydrobenzo[b]thiophen-5-ylmethyl)-1H-imidazole |

The hydrogen chloride salt of this compound is prepared by the method of Example B-6.

| | |
|---|---|
| **(9b)** | 5-(3H-inudazol-4(5)-ylmethyl)-6,7-dihydro-5H-benzo[b]thiophen-4-one |

The hydrogen chloride salt of this compound is prepared by the method of Example B-6.

| | |
|---|---|
| **(9c)** | 5-(octahydrobenzo[b]thiophen-5-ylmethyl)-1H-imidazole |

### Example B-10

| | |
|---|---|
| 4,4-Dimethyl-1-tetralone | 4(5)-(4,4-dimethyl-1,2,3,4-tetrahydronaphthalen-2-ylmethyl)-1H-imidazole |

### Example B-11(a-b)

| | | |
|---|---|---|
| 1-Benzosuberone | **(11a)** | 4(5)-(6,7,8,9-tetrahydro-5H-benzocyclohepten-6-ylmethyl)-1H-imidazole |
| | **(11b)** | 6-(1H-imidazol-4(5)-ylmethylene)-6,7,8,9-tetrahydrobenzocyclohepten-5-one |

### Example C-1

Procedure for Preparation of 4(5)-thiophen-3-ylmethyl-1H-imidazole :

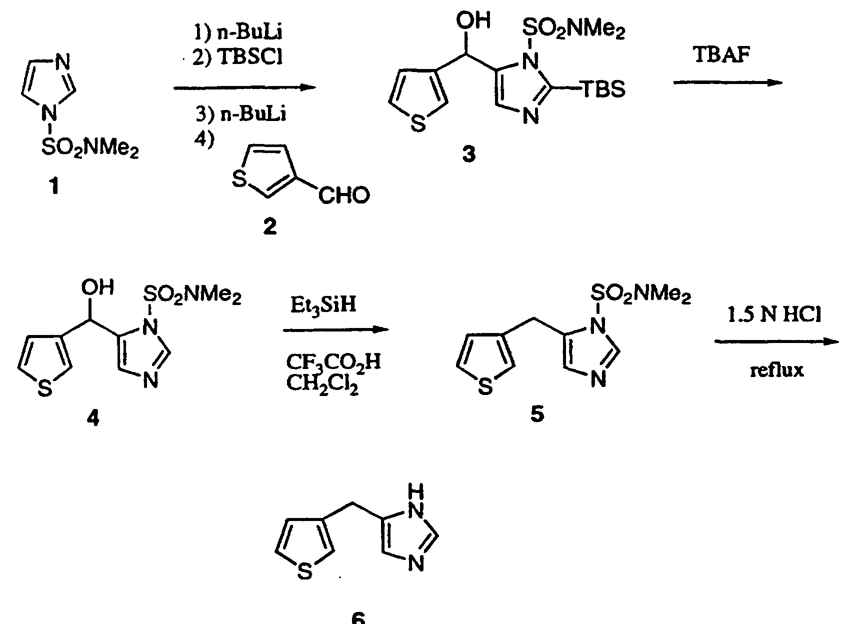

### Procedure -

1-(Dimethylsulfamoyl)imidazole (1) (2.0g, 11.4 mmol) is taken up in 42mL of anhydrous THF and cooled to -78°C. n-BuLi (6.6mL, 10.6 mmol) is added dropwise to the solution of (1). The resultant solution is stirred at -78°C for 30 min. Tert-butyldimethylsilylchloride (TBSCI) (1.6g, 10.6 mmol) in 8mL of THF is added to the reaction. The reaction is warmed to rt and stirred overnight. The next day the reaction is cooled to -20°C and 7.3mL (11.6 mmol) of n- BuLi added. After stirring at -20°C for 45 min, 3-thiophene carboxaldehyde (2) (1.0mL, 11.6 mmol) is added to the reaction mixture. Then reaction is warmed to rt and stirred overnight. The next day the reaction is quenched with water and diluted with ethyl acetate. The organic layer is washed with water followed by brine. The organic phase is dried over sodium sulfate and the solvent removed under reduced pressure. Flash chromatography (2:5 ethyl acetate/ hexane) affords 3.0g (7.5 mmol) of 2-(t-butyldimethylsilyl)-5-(hydroxythiophen-2-ylmethyl)imidazole-1-sulfonic acid dimethylamide (3). (3) (1.5g, 3.74 mmol) is taken up in 37mL of THF. A 1M solution of tetra-n-butylammonium fluoride (TBAF) in THF (4.1mL, 4.1 mmol) is added dropwise to the solution of (3). The reaction is stirred overnight at rt. The next day the reaction is quenched with water and then extracted with ethyl acetate. The organic layer is washed with water followed by brine. The organic phase is dried over sodium sulfate and the solvent removed under reduced pressure. 0.94g (3.3 mmol) of 5-(hydroxythiophen-2-ylmethyl)imidazole-1-sulfonic acid dimethylamide (4) is recovered. (4) (0.5g, 1.74 mmol) is taken up in 23mL of dichloromethane, to the solution is added 2.2 mL (13.9 mmol) of triethylsilane and 4.3 mL (55.7 mmol) of trifluoroacetic acid. The reaction is stirred at rt overnight and then quenched with water and neutralized with solid sodium bicarbonate. The organic layer is washed with water followed by brine. The organic phase is dried over sodium sulfate and the solvent removed under reduced pressure. Flash chromatography using a 1:1 mixture of ethyl acetate and hexane affords 0.42g (1.55 mmol) of 5-(thiophen-2-ylmethyl)imidazole-1-sulfonic acid dimethylamide (5). (5) (0.42g, 1.55 mmol) is taken up in 10mL of a 1.5N HCl solution and heated at reflux for 3h and then stirred at rt overnight. The reaction is diluted with ethyl acetate, neutralized with solid sodium bicarbonate and then made basic with 2N NaOH. The organic layer is washed with water followed by brine. The organic phase is dried over sodium sulfate and the solvent removed under reduced pressure. Flash chromatography using a 10:1 mixture of chloroform and methanol affords 0.17g (1.0 mmol) of 4(5)-thiophen-3-ylmethyl-1H-imidazole (6) (C-1).
^{**1**}**H NMR** (300 MHz, CD₃OD) 7.52 (s, 1H), 7.25-7.27 (m, 1H), 6.96-7.01 (m, 2H), 6.77 (s, 1H), 3.98 (s, 2H).

### Example C-2

The 2-carboxaldehyde isomer of 3-thiophene carboxaldehyde is substituted into the method of Example C-1 to yield 4(5)-thiophen-2-ylmethyl-1H-imidazole

### Example C-3

5-Methyl-2-thiophene carboxaldehyde of 3-thiophene carboxaldehyde is substituted into the method of Example C-1 to yield 4(5)-(5-methylthiophen-2-ylmethyl)-1H-imidazole

### Example C-4

5-Chloro-2-thiophene carboxaldehyde of 3-thiophene carboxaldehyde is substituted into the method of Example C-1 to yield 4(5)-(5-chlorothiophen-2-ylmethyl)-1H-imidazole

### Example C-5

2-Furan carboxaldehyde is substituted into the method of Example C-1 to yield 4 (5)-furan-2-ylmethyl-1H-imidazole

### Example C-6

3-Furan carboxaldehyde is substituted into the method of Example C-1 to yield 4(5)-furan-3-ylmethyl-1H-imidazole

### Example C-7

5-Methyl-2-furan carboxaldehyde is substituted into the method of Example C-1 to yield 4(5)-(5-methylfuran-2-ylmethyl)-1H-imidazole

### Example C-8

Benzaldehyde is substituted into the method of Example C-1 to yield 4(5)-benzyl-1H-imidazole

### Example C-9

2-Thianaphthene carboxaldehyde is substituted into the method of Example C-1 to yield 4(5)-benzo[b]thiophen-2-ylmethyl-1H-imidazole

### Example C-10

2-Benzofuran carboxaldehyde is substituted into the method of Example C-1 to yield 4(5)-benzofuran-2-ylmethyl-1H-imidazole

### Example C-11

5-Ethyl-2-furan carboxaldehyde is substituted into the method of Example C-1 to yield 4(5)-(5-ethylfuran-2-ylmethyl-1H-imidazole

### Example C-12

4-Bromo-2-thiophene carboxaldehyde is substituted into the method of Example C-1 to yield 4(5)-(4-bromothiophen-2-ylmethyl)-1H-imidazole

### Example C-13

4-Phenyl-2-thiophene carboxaldehyde is substituted into the method of Example C-1 to yield 4(5)-(4-phenylthiophen-2-ylmethyl)-1H-imidazole

### Example C-14

4-Methyl-2-thiophene carboxaldehyde is substituted into the method of Example C-1 to yield 4(5)-(4-methylthiophen-2-ylmethyl)-1H-imidazole,hydrochloride salt

### Example D-1

### Procedure for Preparation of oxazolidin-2-ylidene-(3-phenyl bicyclo[2.2.1]hept-2-yl) amine:

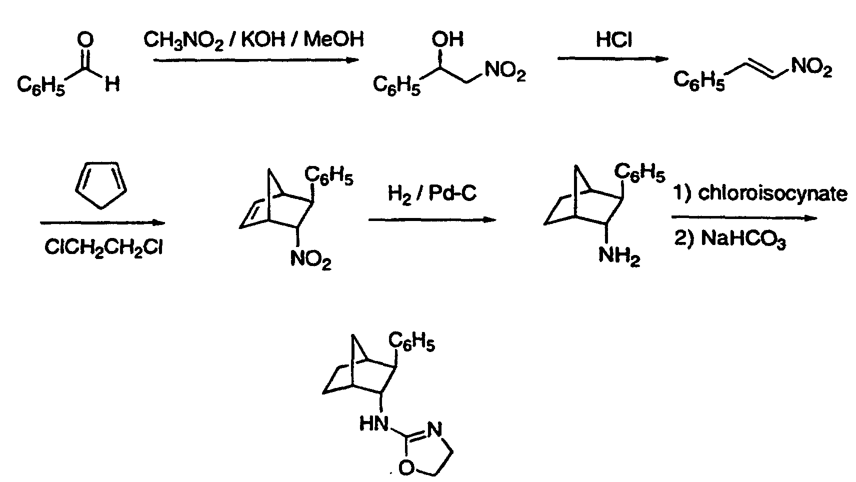

### Procedure -

The *endo exo* relative stereochemistry of the compound was prepared, by making the β-nitrostyrene as shown above. Treatment of a methanol solution of benzaldehyde (10g, 94.3 mmole) with nitromethane (51ml, 943 mmol) in the presence of sodium hydroxide (3N in methanol to pH=8) afforded the nitro alcohol in 60% yield. Dehydration of the alcohol was effected by treatment with methanesulfonyl chloride (3.56g, 31.1mmole) followed by triethylamine (6.3g, 62.2 mmol) in dichloromethane (35ml) to give 97% yield of product. Kugelrohr distillation was done to purify compound. Construction of the bicyclo[2.2.1]heptane skeleton was carried out in one step. The Diels-Alder reaction was conducted by warming the nitrostyrene (4.5g, 30.2 mmole) with cyclopentadiene (3.98g, 60.4 mmole) in 1, 2-dichloroethane (10ml). The Diels-Alder reaction proceeds in approximately a 3:1 endo:exo nitro ratio. Both the ratio and relative stereochemistry was demonstrated through x-ray analysis. Reduction of both the nitro group and the olefin was carried out under an atmosphere of hydrogen in the presence of 10% by weight palladium on charcoal. Separation of isomers was conveniently carried out at this stage using flash chromatography with 5% ammonia-saturated methanol in dichloromethane. The amine (0.7g, 3.74 mmole) was treated first with chloroethylisocyanate (0.38ml, 4.49mmole) to afford the chloroethylurea, which was then warmed in the presence of aqueous NaHCO₃ solution to afford oxazolidin-2-ylidene-(3-phenyl bicyclo[2.2.1] hept-2-yl) amine (D-1) in 51% yield.
^{**1**}**H NMR** (300 MHz, CDCl₃) d 1.36-1.80 (m, 6H), 2.14 (d, 1H, J=4.40Hz), 2.37 (s, 1H), 2.65 (s, 1H), 3.71-3.78 (m, 2H), 3.95-3.98 (m, 1H), 4.19-4.25 (t, 2H, J=17.15Hz, J=8.36Hz), 7.17-7.29 (m, 5H).

### Example D-2

Oxazolidin-2-ylidene-(3-o-tolyl bicyclo[2.21]hept-2-yl)amine is prepared by substituting o-methyl β-nitrostyrene in the method of D-1

### Example D-3

Bicyclo[2.2.1]hept-2-yl oxazolidin-2-ylidene amine is prepared by substituting nitroethene in the method of D-1

### Example E-1

### Procedure for Preparation of imidazolidin-2-ylidene-(4-methyl-3,4-dihydro-2H-benzo[1,4]oxazin-6-yl)amine:

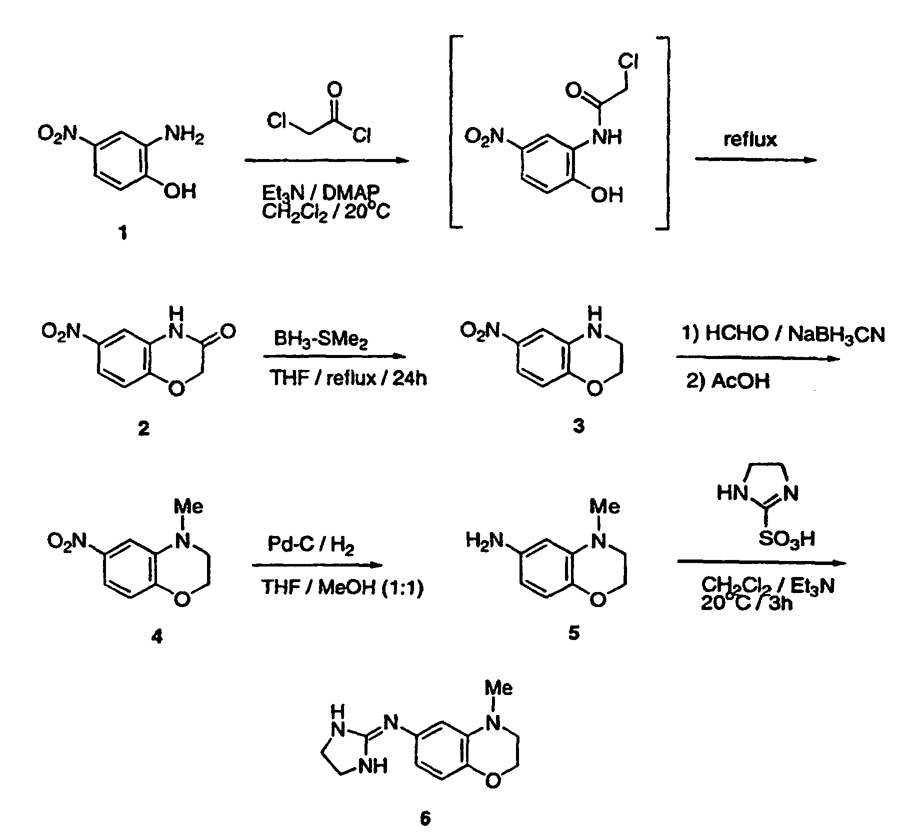

### Procedure -

To 2-amino-4-nitrophenol (1) (4.00g, 25.95 mmol), triethylamine (15.20mL, 109.0 mmol) and 4-dimethylaminopyridine (0.063g, 0.52 mmol) slurried in anhydrous CH₂Cl₂ (250mL) at 0°C under argon added chloroacetyl chloride (2.27 mL, 28.55mmol) via syringe. After refluxing for 72h pure product was filtered off and washed with water. The mother liquor was washed successively with phosphoric acid (0.5M), saturated sodium bicarbonate, water and brine and then dried over MgSO₄. This solution was adhered to silica and purified by flash chromatography on silica with hexane/ethyl acetate (4:6) to give additional product. The combined solids were dried in vacuo to give pure 6-nitro-4H-benzo[1,4]oxazin-3-one (2) (4.12g) in 82% yield. To a slurry of (2) (1.49g, 7.65 mmol) in anhydrous THF (40mL) under argon in a 2-neck round-bottom flask equipped with a reflux condenser was added borane-dimethyl sulfide complex (15.3mL, 30.62 mmol). The mixture was heated at reflux until starting material was no longer observed via thin layer chromatography (2h). The reaction mixture was cooled to rt and carefully quenched by the dropwise addition of methanol. The resulting mixture was then refluxed an additional 10 minutes. The crude reaction mixture was concentrated in vacuo and purified by flash chromatography on silica with hexane/ethyl acetate (8:2) to give pure 6-nitro-3,4-dihydro-2H-benzo[1,4]oxazine (3) (1.36g) as an orange solid in 99% yield. To (3) (0.032g, 0.178mmol) and formalin (37% in H₂O, 0.20 mL, 2.67 mmol) in anhydrous acetonitrile (1.5mL) at ambient temperature was added sodium cyanoborohydride (0.034g, 0.534 mmol). This solution was stirred for 30 min before adding glacial acetic acid (0.032mL, 0.534 mmol). The resulting mixture was stirred an additional 16h. The organics were taken up in diethyl ether and washed successively with NaOH (2N) and brine, dried over MgSO₄ and concentrated in vacuo. The resulting solids were purified by flash chromatography on silica with hexane/ethyl acetate (7:3) to give pure 4-methyl-6-nitro-3,4-dihydro-2H-benzo[1,4]oxazine (4) (0.031g) in 93% yield. To (4) (2.16g, 11.12 mmol) and 10% palladium on carbon (0.216g, 10 wt. %) under argon was added methanol (MeOH) (30mL) followed by THF (30mL). Hydrogen was bubbled thru the resulting slurry until no (4) remained visible by thin layer chromatography (2h). Celite was added and the mixture was filtered through a bed of celite followed by a MeOH wash. The resulting solution was concentrated in vacuo to give pure 4-methyl-3,4-dihydro-2H-benzo[1,4]oxazin-6-ylamine (5) (1.86g) as a pale purple oil in 100% yield which was carried on without further purification. To (5) (1.86g, 11.34 mmol) and imidazoline-2-sulfonic acid (1.84g, 12.24 mmol) in anhydrous acetonitrile (50mL) under argon at 0°C was added triethylamine (3.26mL, 23.36 mmol). This solution was gradually warmed to ambient temperature and stirred for 16h. At that time an additional amount of imidazoline-2-sulfonic acid (0.86g, 5.55 mmol) was added and the resulting mixture was stirred an additional 5h. This solution was concentrated in vacuo and the residues were taken up in H₂O. The organics were extracted into CH₂Cl₂ and washed twice with NaOH and then brine, dried over MgSO₄ and concentrated in vacuo. The resulting foam was purified by flash chromatography on silica with 20% methanol (saturated with ammonia) in chloroform to give pure imidazolidin-2-ylidene-(4-methyl-3,4-dihydro-2H-benzo[1,4]oxazin-6-yl)amine (6) (E-1) (0.905g) in 34% yield.
^{**1**}**H NMR** (CDCl₃): 2.81 (s, 3H); 3.26 (t, J=8.9 Hz, 2H); 3.60 (s, 4H); 4.26 (m, 2H); 4.60 (vbrs, 2H); 6.34 (dd, J=8.2 Hz, J=2.4 Hz, 1H); 6.39 (d, J=2.4 Hz, 1H); 6.68 (d, J= 8.2 Hz, 1H).

### Example F & G

Procedure for Preparation of 6-(imidazolidin-2-ylidene amino)-5-methyl-4H-benzo[1,4]oxazin-3-one (F) and Imidazolidin-2-ylidene-(5-methyl-3,4-dihydro-2H-benzo[1,4]oxazin-8-yl)amine (G):

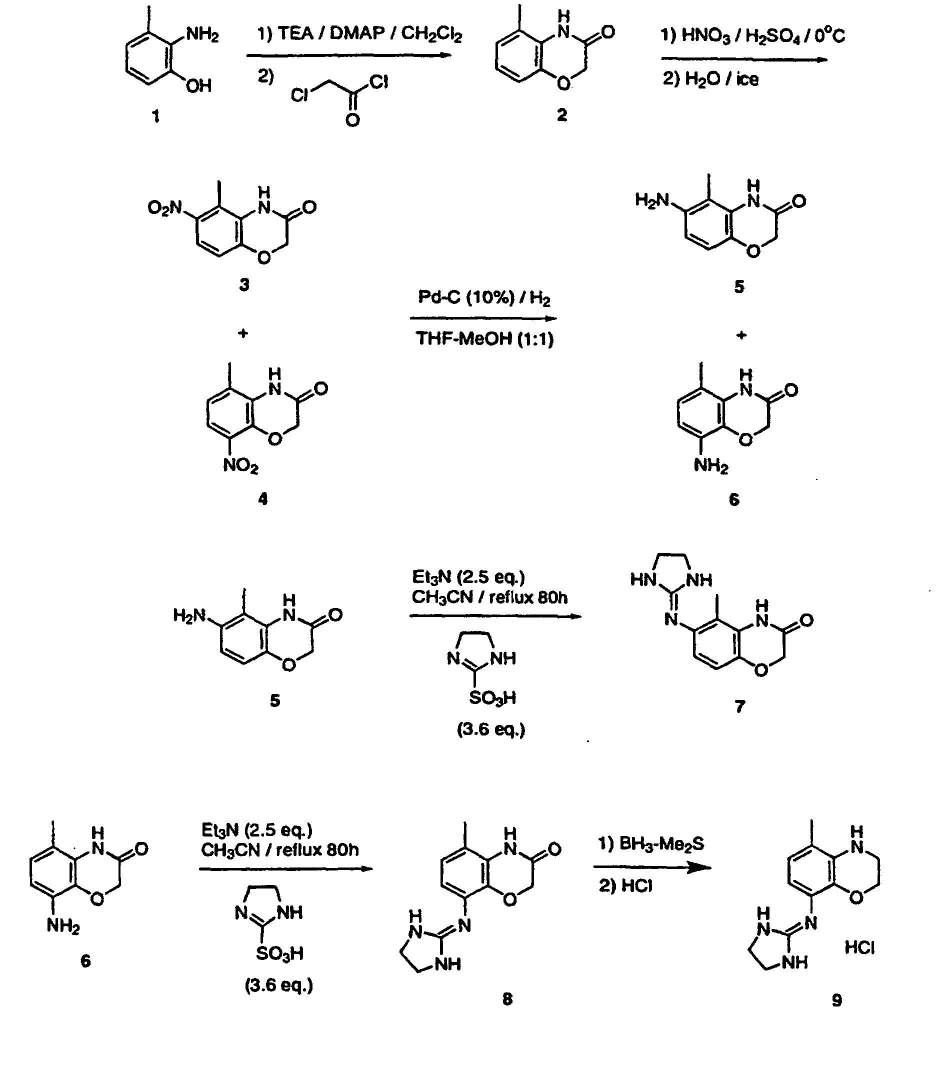

### Procedure -

To 2-amino-3-methylphenol (1) (14.72g, 0.120 mol), triethylamine (35.0mL, 0.251 mol) and 4-dimethylaminopyridine (0.29g, 2.39 mmol) in anhydrous CH₂Cl₂ (100mL) at 0°C under argon was added chloroacetyl chloride (10.0mL, 0.126 mol) dropwise via syringe. After the addition was complete the resulting solution was refluxed for 24h. The organics were washed successively with phosphoric acid (0.5M), saturated sodium bicarbonate, water and brine and then dried over MgSO₄. The resulting solution was concentrated and taken up in THF to which ether was added. The resulting crystals were filtered off to give pure 5-methyl-4H-benzo[1,4]oxazin-3-one (2) (12.30g) in 63% yield. To (2) (14.64g, 89.72 mmol) dissolved in concentrated H₂SO₄ (65 mL) at -10°C was added 70% concentrated HNO₃ (8.08g, 89.72 mmol) in concentrated H₂SO₄ (25mL) with rapid mechanical stirring at a rate whereby the internal temperature was maintained below -5°C. As soon as the addition was complete the mixture was poured onto crushed ice (500mL) and the resultant solids were filtered off and slurried in cold water (300 mL) while sufficient NaOH was added to adjust the pH to 7. The recovered yellow powder was dissolved in THF, adhered to silica and purified by flash chromatography with 60% hexane and ethyl acetate to give the nitrated product as a mixture of two regioisomers, i.e. the desired 6-substituted aromatic comprising 6-nitro-5-methyl-4H-benzo[1,4]oxazin-3-one (3) (55%) and the 8-substituted byproduct comprising 8-nitro-5-methyl-4H-benzo[1,4]oxazin-3-one (4) (22%). These isomers are separated with difficulty at this point and were carried on to the next step as a mixture. To a mixture of (3) (1.93 g, 9.27mmol) and (4) (0.48g, 2.32 mmol) dissolved in a solution of MeOH (300mL) and THF (300mL) under argon was added 10% palladium on carbon (1.20g). The resulting solution was subjected to H₂ at one atmosphere pressure. After 16h the catalyst was filtered off and the resulting solution was concentrated in vacuo and purified by flash chromatography on silica with 50% hexane and ethyl acetate to give 6-amino-5-methyl-4H-benzo[1,4]oxazin-3-one (5) (0.96 g) in 46% yield and 8-amino-5-methyl-4H-benzo[1,4]oxazin-3-one (6) (0.17 g) in 8% yield. (5) (1.20g, 6.74 mmol), imidazoline-2-sulfonic acid (2.02g, 13.48 mmol) and triethylamine (2.35mL, 16.85 mmol) were heated at reflux in anhydrous acetonitrile (50mL) under argon for 48h. At that time an additional amount of imidazoline-2-sulfonic acid (1.01g, 6.74 mmol) and triethylamine (1.41mL, 10.12 mmol) were added and the resulting mixture was stirred an additional 24h. This solution was concentrated in vacuo and the residues were taken up in a solution of CHCl₃/isopropyl alcohol (3:1) and washed successively with NaOH (1N) and brine, dried over MgSO₄ and concentrated in vacuo. The resulting foam was purified by flash chromatography on silica with 20% methanol saturated with ammonia in chloroform to give 6-(imidazolidin-2-ylideneamino)-5-methyl-4H-benzo[1,4]oxazin-3-one (7) (0.42g) as a foam in 27% yield along with 55% recovered starting material. The HCl salt was recrystallized from a mixture of ethanol and diethyl ether (EtOH/Et₂O) to give fine white needles.
^{**1**}**H NMR** (DMSO): 2.10 (s, 3H); 3.59 (s, 4H); 4.53 (s, 2H); 6.83 (d, J=8.6 Hz, 1H); 6.90 (d, J= 8.6 Hz, 1H); 8.07 (brs, 2H); 10.15 (vbrs, 1H); 10.42 (s, 1H).

(6) (0.222 g, 1.35mmol), imidazoline-2-sulfonic acid (0.223 g, 1.49mmol) and triethylamine (0.415 mL, 2.98mmol) were heated at 95°C in anhydrous acetonitrile (10 mL) in a sealed tube for 2h. At that time an additional amount of imidazoline-2-sulfonic acid (0.112 g, 0.75mmol) was added and the reaction was continued for an additional 16 h. This solution was concentrated in vacuo and the residues were taken up in a solution of CHCl₃/isopropyl alcohol (3:1) and washed successively with NaOH (2N) and brine, dried (MgSO₄) and concentrated in vacuo. The resulting oil was recrystalizied from CHCl₃ to give pure 6-(imidazolidin-2-ylideneamino)-5-methyl-4H-benzo[1,4]oxazin-3-one (8) (F) (0.048 g) as a white powder in 15% yield along with 35% recovered starting material. To a slurry of (8), (0.08 g, 0.321mmol) in anhydrous THF (50 mL) under argon in a 3-neck round-bottom flask equipped with reflux condenser was added borane-dimethyl sulfide complex (0.48 mL, 0.936mmol). The mixture was heated at reflux until starting material was no longer observed via thin layer chromatography (3 h). The reaction mixture was cooled to room temperature and carefully quenched by the dropwise addition of methanol. The crude reaction mixture was concentrated in vacuo and purified by flash chromatography on silica using 20% methanol saturated with ammonia/chloroform to give imidazolidin-2-ylidene-(5-methyl-3,4-dihydro-2H-benzo[1,4]oxazin-8-yl)amine (9) (G) (0.03 g) as the HCl salt in 37% yield.
^{**1**}**H NMR** (CDCl₃): 2.07 (s, 3H); 3.46 (t, J=4.3Hz, 2H); 3.55 (s, 4H); 4.24 (t, J=4.3Hz, 2H); 5.60 to 5.95 (vbrs, 2H); 6.44 (d, J=8.0 Hz, 1H); 6.57 (d, J=8.0 Hz, 1H).

### Example H

### Procedure for Preparation of 4(5)-phenylsulfanyl-1H-imidazole :

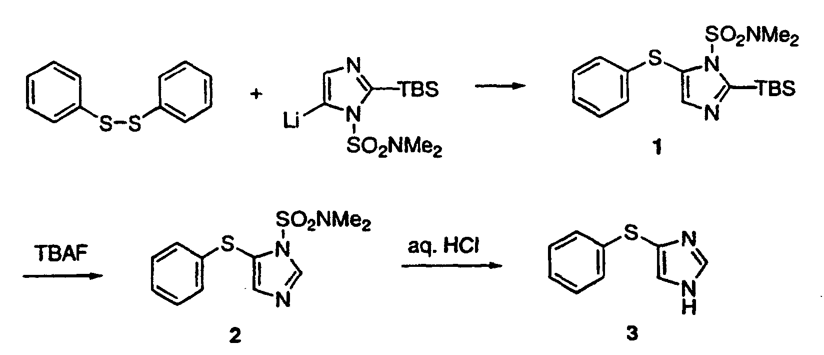

### Procedure -

1-(N,N-dimethylsulfamoyl)imidazole (1.5g, 8.6 mmol) was taken up in 28mL of THF. The solution was cooled to -78°C and n-BuLi (5.4mL, 8.6 mmol) added dropwise via syringe. After stirring at -78°C for 1h TBSCI (1.3g, 8.56 mmol) in 10mL of THF was added. The bath was removed and the reaction allowed to warm-up to rt. The reaction mixture was stirred overnight. The reaction mixture was cooled to -20°C and n-BuLi (5.4 mL, 8.6 mmol) added. After 45 min phenyldisulfide (1.9g, 8.6 mmol) in 8mL of THF was added. The reaction mixture was stirred at rt for 48h. The reaction mixture was quenched with saturated ammonium chloride and extracted with ethyl acetate. The organic layer was collected and washed with water and then brine. The solution was dried over sodium sulfate and the solvent removed under reduced pressure. Flash chromatography (2.5% EtOAc/hexane) afforded 2.8g (7.0 mmol) of 2-(t-butyldimethylsilyl)-5-phenylsulfanylimidazole-1-sulfonic acid dimethylamide (1) as a yellow color oil. The compound (1) (2.8g, 7.0 mmol) was dissolved in THF and the solution cooled to 0°C. TBAF (7.0mL, 7.0 mmol) was added dropwise to the solution. The reaction mixture was stirred overnight at rt. The next day the reaction was quenched with water and extracted with ethyl acetate. The organic layer was washed with water followed by brine. The solution was dried over sodium sulfate and the solvent removed under reduced pressure. Flash chromatography (50% EtOAc/hexane) afforded 474mg of 5-phenylsulfanylimidazole-1-sulfonic acid dimethylamide (2) and 290mg of 5-phenylsulfanyl-1H-imidazole (3) (H). The 478mg of (2) was added to 2N HCl and the solution heated at reflux for 2h. The reaction mixture was made basic with 2N NaOH and extracted with ethyl acetate. The organic layer was washed with water followed by brine. The solution was dried over sodium sulfate and the solvent removed under reduced pressure. Flash chromatography (EtOAc) afforded (3) as a white crystalline solid. A combined total of 360mg (2.0 mmol) of (3) is recovered.
^{**1**}**H NMR** (300 MHz, CD₃OD) 7.91 (s, 1H). 7.37 (s, 1H), 7.19-7.23 (m, 2H), 7.07-7.11 (m, 3H).

### Example I

Procedure for Preparation of 4(5)-(1,2,3,4-tetrahydronaphthalen-2-ylmethyl)-4,5-dihydro-1H-imidazole, methane sulfonic acid salt:

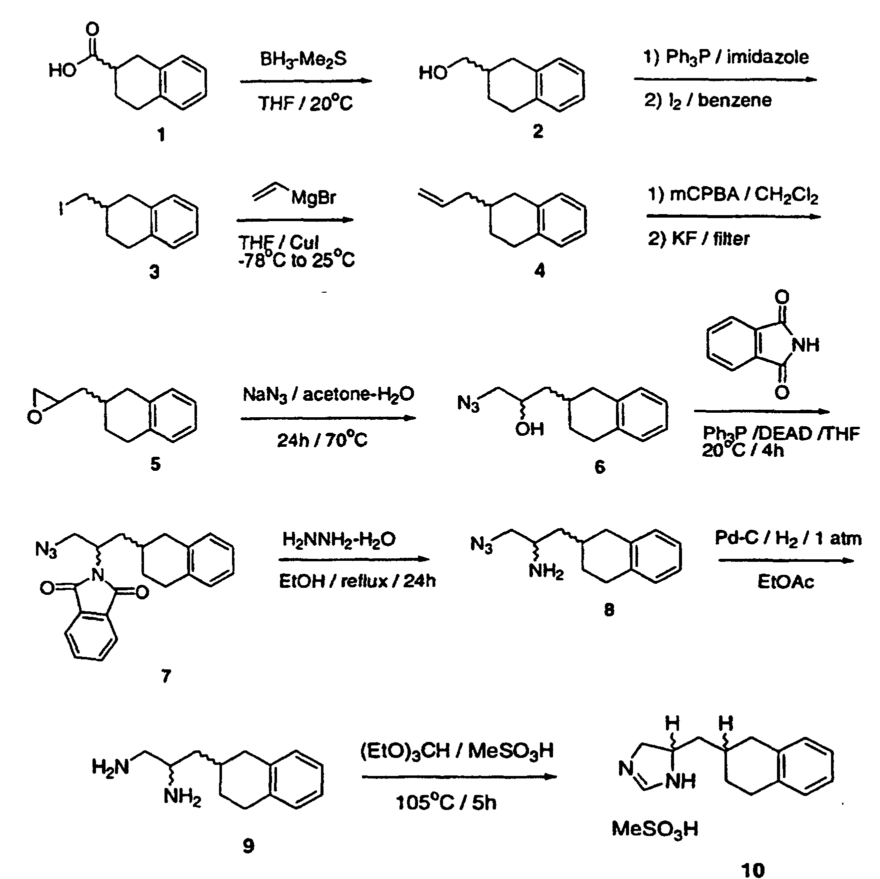

### Procedure -

To 1,2,3,4-tetrahydronaphthalene-2-carboxylic acid (1) (4.93g, 27.42 mmol in anhydrous THF (250mL) at 20°C under argon was added 3.26 mL (32.90 mmol) borane-dimethylsulfide (BH₃-Me₂S) via syringe. After stirring for 16h MeOH (4mL) was added and the mixture was warmed to 55°C until no more gas was evolved. The mixture was concentrated to an oil, taken up in Et₂O and washed successively with 2M phosphoric acid, saturated sodium bicarbonate, water and brine and then dried over MgSO₄ and reconcentrated. The resulting oil was purified by high vacuum Kugelrohr at 150°C to give pure alcohol (1,2,3,4-tetrahydronaphthalen-2-yl)methanol (2) (4.09g) in 93% yield. To triphenylphosphine (10.179g, 38.809 mmol) and imidazole (2.64g, 38.809 mmol) in anhydrous benzene (175mL) was added the iodine (8.60g, 33.865 mmol) in benzene (75mL) with rapid stirring followed by (2) in benzene (50mL). After 3h the solids were filtered off and the filtrate was reduced in vacuo to a volume of 50mL to which was added hexane (200mL). The resultant solids were filtered off and the filtrate was washed successively with water and brine, dried over MgSO₄ and concentrated in vacuo. The resulting oil was purified by flash chromatography on silica with hexane to give pure 2-iodomethyl-1,2,3,4-tetrahydronaphthalene (3) (6.239g) in 90% yield. To (3) (10.02 g, 36.85 mmol) and CuI (1.41g, 7.37 mmol) in anhydrous THF (50mL) at -78°C under argon was added vinylmagnesium bromide (1M in THF, 73.70mL, 73.70 mmol) slowly at a speed at which no color developed. This solution was allowed to warm to 0°C and stirred for 6h. The resulting mixture was recooled to -40°C and quenched by the careful addition of 2M phosphoric acid (35mL). This solution was diluted with 100mL water and extracted with hexanes. The organic fractions were washed successively with water and brine, dried over MgSO₄ and concentrated in vacuo. The resulting oil was purified by flash chromatography on silica with hexane to give 2-allyl-1,2,3,4-tetrahydronaphthalene (4) (5.618g) in 88% yield. (4) (5.615g, 32.645 mmol) and meta-chloroperbenzoic acid (m-CPBA) (14.08g, 81.613 mmol) were stirred in anhydrous methylene chloride (50mL) for 16h. The solids were filtered off and potassium flouride KF (5.11g, 88.142 mmol) was added and this mixture was stirred an additional hour. The solids were filtered off and the reaction was concentrated in vacuo. The resulting oil was purified by flash chromatography on silica with 5% ethyl acetate in hexane to give 2-(1,2,3,4-tetrahydronaphthalen-2-ylmethyl)oxirane (5) (5.41g) in 88% yield. To (5) (1.626g, 8.649 mmol) in a solution of acetone (20mL) and water (5mL) was added sodium azide (1.97g, 30.271 mmol). This solution was warmed to 85°C and stirred for 48h. The solution was concentrated in vacuo and the residues were taken up in CHCl₃ and washed successively with water and brine, dried over MgSO₄ and concentrated in vacuo. The resulting oil was purified by flash chromatography on silica with 30% ethyl acetate in hexane to give pure 1-azido-3-(1,2,3,4-tetrahydronaphthalen-2-yl)propan-2-ol (6) (1.762g) in 88% yield. A mixture of (6) (1.88g, 8.140 mmol), triphenylphosphine (2.67g, 10.173 mmol), phthalimide (1.50g, 10.173 mmol), diethyl azodicarboxylate (DEAD) (1.77g, 10.173 mmol) were stirred in anhydrous THF (50mL) for 4h. This solution was concentrated in vacuo, taken up in a solution of hexane (25mL) and ether (25mL) and stirred for 16h. The solids were filtered off and the filtrate was concentrated in vacuo. The resulting oil was purified by flash chromatography on silica with 20% ethyl acetate in hexane to give 2-[1-azidomethyl-2-(1,2,3,4-tetrahydronaphthalen-2-yl)ethyl]isoindole-1,3-dione (7) (2.487g) contaminated with a small amount of impurity which was carried on without further purification. A mixture of (7) (3.93g, 10.917 mmol) and hydrazine (0.680mL, 21.833 mmol) were heated in ethanol (60mL) at reflux for 16h. The solids were filtered off and the filtrate was concentrated in vacuo. The residues were purified by flash chromatography on silica with 5% MeOH in CH₂Cl₂ to give 1-azidomethyl-2-(1,2,3,4-tetrahydronaphthalen-2-yl)ethylamine (8) (2.057g) in 88% yield. A mixture of (8) ( 2.056g, 8.940 mmol) and 10% palladium on carbon (0.260 g) were stirred in MeOH (30mL) under 1 atmosphere of hydrogen for 16h. The solids were filtered off and the filtrate was concentrated in vacuo. The residues were purified by flash chromatography on silica with 10% ammonia saturated MeOH in CH₂Cl₂ to give 3-(1,2,3,4-tetrahydronaphthalen-2-yl)propane-1,2-dione (9) (1.557g) in 85% yield. A mixture of (9) (0.590g, 2.892 mmol) and methanesulfonic acid (0.980mL, 14.460 mmol) were heated in triethylorthoformate (10mL) at 105°C 3h. The reaction was concentrated in vacuo and the solids were filtered off. Subsequent recrystalization of these solids from a mixture of MeOH and ether gave pure 4(5)-(1,2,3,4-tetrahydronaphthalen-2-ylmethyl)-4,5-dihydro-1H-imidazole, methane sulfonic acid salt (I) (0.435g) in 48% yield.
^{**1**}**H NMR** (CDCl₃): 1.37 to 1.56 (m, 1H); 1.56 to 1.70 (m, 1H); 1.80 to 2.02 (m, 2H); 2.32 to 2.55 (m, 2H); 2.72 (s, 3H); 2.75 to 2.95 (m, 3H); 3.48 to 3.59 (m, 1H); 3.93 to 4.08 (m, 1H); 4.31 to 4.47 (m, 1H); 7.00 to 7.20 (m, 4H); 8.46 (s, 1H); 10.04 (s, 1H); 10.35 (brs, 1H).

### Example J-1

Procedure for Preparation of 4(5)-cyclohexylmethyl-1H-imidazole:

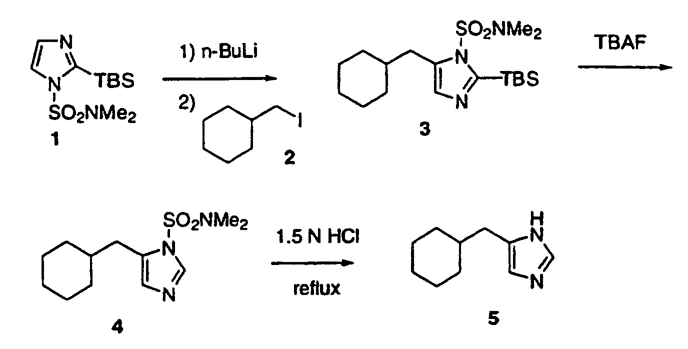

### Procedure -

2-Tert-butyldimethylsilyl-1-dimethyisulfamoyl imidazole (1) (4.1g, 14.2 mmol) is taken up in 47 mL of anhydrous THF and cooled to -20°C. n-BuLi (8.9 mL, 14.2 mmol) is added dropwise to the solution of (1). The resultant solution is stirred at -20°C for 45 min. Cyclohexylmethyl iodide (2) (3.14g, 14 mmol) is then added dropwise to the reaction mixture. Then reaction is warmed to rt and stirred overnight. The next day the reaction is quenched with saturated ammonium chloride and diluted with water. The mixture is extracted with ethyl acetate (3 x 100 mL). The organic layers are combined and washed with water followed by brine. The organic phase is dried over sodium sulfate and the solvent removed under reduced pressure. Flash chromatography (4:1 ethyl acetate/ hexane) affords 2.26g (5.6 mmol) of 5-cyclohexylmethyl-2-tert-butyldimethylsilyl-1-dimethylsulfamoyl imidazole (3). (3) (2.26g , 5.6 mmol) is taken up in 56 mL of THF and cooled to 0°C. A 1M solution of TBAF in THF (5.6 mL, 5.6 mmol) is added dropwise to the solution of (3). The reaction is warmed to rt and stirred overnight. The next day the reaction is quenched with water and then extracted with ethyl acetate. The organic layer is washed with water followed by brine. The organic phase is dried over sodium sulfate and the solvent removed under reduced pressure. Flash chromatography (1:1 ethyl acetate/ hexane) affords 1.2g (4.42 mmol) of 5-cyclohexylmethyl-1-dimethylsulfamoyl imidazole (4). (4) (1.2g, 4.42 mmol) is taken up in 25 mL of a 1.5N HCl solution and heated at reflux for 2h. The reaction is cool to rt and diluted with ethyl acetate. The mixture is brought to pH 13 with 2N NaOH and then extracted with chloroform (4 x 100 mL). The organic layers are combined and washed with water followed by brine. The organic phase is dried over sodium sulfate and the solvent removed under reduced pressure. Flash chromatography (9:1 chloroform/ methanol) affords 700 mg (4.27 mmol) of 4(5)-cyclohexylmethyl-1H-imidazole (5) (J-1).
^{**1**}**H NMR** (CDCl₃): 0.92 to 1.0 (m, 2H); 1.16 to 1.26 (m, 3H); 1.57 to 1.73 (m, 6H); 2.48 (d, J=6.9 Hz, 2H); 6.77 (s, 1H); 7.56 (s, 1H)

### Example J-2

(S)-2-iodomethyl-1,2,3,4-tetrahydronaphthalene is substituted into the method of Example J-1 to yield (S)-4(5)-(1,2,3,4-tetrahydronaphthalen-2-ylmethyl)-1H-imidazole, (S)-2-iodomethyl-1,2,3,4-tetrahydronaphthalene was prepared from (S)-1,2,3,4-tetrahydro-2-naphthoic acid. (S)-1,2,3,4-tetrahydro-2-naphthoic acid was prepared from the resolution of 1,2,3,4-tetrahydro-2-naphthoic acid (*J*. *Med*. *Chem*. **1983,** *26*, 328-334)

### Example J-3

(R)-2-iodomethyl-1,2,3,4-tetrahydronaphthalene is substituted into the method of Example J-1 to yield (R)-4(5)-(1,2,3,4-tetrahydronaphthalen-2-ylmethyl)-1H-imidazole. (R)-2-iodomethyl-1,2,3,4-tetrahydronaphthalene was prepared from (R)-1,2,3,4-tetrahydro-2-naphthoic acid. (R)-1,2,3,4-tetrahydro-2-naphthoic acid was prepared from the resolution of 1,2,3,4-tetrahydro-2-naphthoic acid (*J*. *Med. Chem*. **1983,** *26*, 328-334)

### Example K-1

Procedure for Preparation of 4(5)-(4,5,6,7-tetrahydrobenzo[b]thiophen-2-ylmethyl)-1H-imidazole:

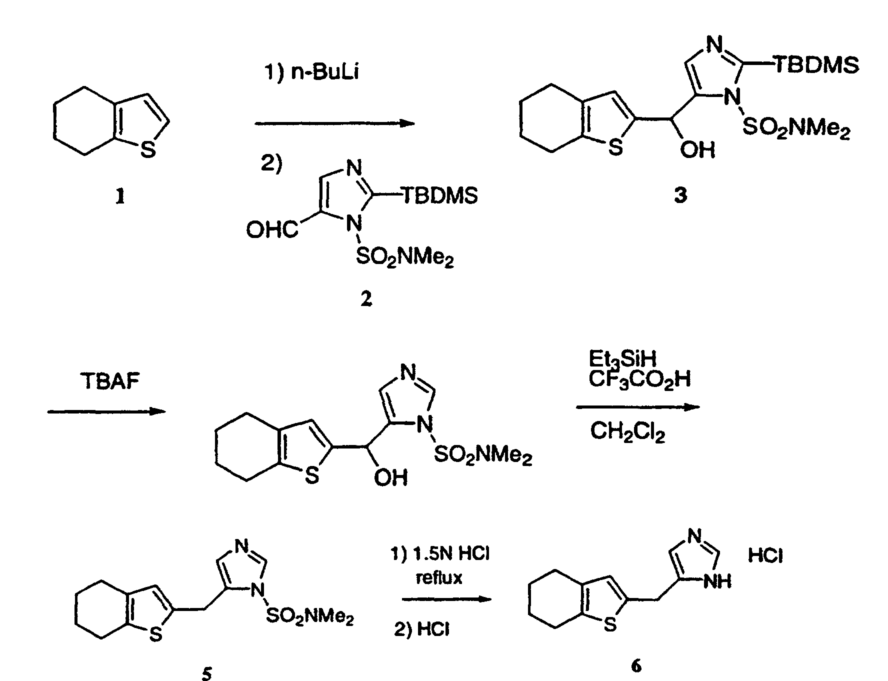

### Procedure -

4,5,6,7-tetrahydrobenzo[b]thiophene (1) (2.1g, 15 mmol) is taken up in 75mL of anhydrous THF and cooled to -78°C. n-BuLi (6.0mL, 15 mmol) is added dropwise to the solution of (1). The resultant solution is stirred at - 78°C for 60 min. 1-Dimethylsulfamoyl-2-t-butyldimethylsilyl-5-imidazolecarboxaldehyde (2) (4.8g, 15 mmol) in 25mL of THF is added to the reaction. The reaction is warmed to rt and stirred for 2h before being quenched with water and diluted with ethyl acetate. The organic layer is washed with water followed by brine. The organic phase is dried over sodium sulfate and the solvent removed under reduced pressure. Flash chromatography (1:3 ethyl acetate/ hexane) affords 5.2g (11 mmol) of 2-(tert-butyldimethylsilyl)-5-[hydroxy-(4,5,6,7-tetrahydrobenzo[b]thiophen-2-yl)methyl]imidazole-1-sulfonic acid dimethylamide (3), (3) (5.2g, 11.3 mmol) is taken up in 57mL of THF. A 1M solution of tetra-n-butylammonium fluoride (TBAF) in THF (11.3mL, 11.3 mmol) is added dropwise to the solution of (3). The reaction is stirred for 1h 15min reaction before being quenched with water and then extracted with ethyl acetate. The organic layer is washed with water followed by brine. The organic phase is dried over sodium sulfate and the solvent removed under reduced pressure. Recrystallization from hexane/ethyl acetate affords 5-[hydroxy-(4,5,6,7-tetrahydrobenzo[b]thiophen-2-yl)methyl)imidazole-1-sulfonic acid dimethylamide (4) (2.1g, 6.2 mmol). An additional 2g of the crude product is also recovered. (4) (2.0g, 5.9 mmol) is taken up in 78mL of dichloromethane, to the solution is added 7.5 mL (46.9 mmol) of triethylsilane and 14.4 mL (0.19 mol) of trifluoroacetic acid. The reaction is stirred at rt overnight and then quenched with water and neutralized with 2N NaOH. The organic layer is washed with water followed by brine. The organic phase is dried over sodium sulfate and the solvent removed under reduced pressure. Flash chromatography using a 1:1 mixture of ethyl acetate and hexane affords 0.75g (2.3 mmol) of 5-(4,5,6,7-tetrahydrobenzo[b]thiophen-2-ylmethyl)imidazole-1-sulfonic acid dimethylamide (5). (5) (0.42g, 1.55 mmol) is taken up in 15mL of a 1.5N HCl solution and heated at reflux for 2h and then stirred at rt overnight. The reaction is diluted with ethyl acetate, neutralized with 2N NaOH. The organic layer is washed with water followed by brine. The organic phase is dried over sodium sulfate and the solvent removed under reduced pressure. The crude product is dissolved in methanol and an excess of HCl in ether is added. Solvent is removed under reduced pressure to afford 0.6g (2.3 mmol) of 4(5)-(4,5,6,7-tetrahydrobenzo[b]thiophen-2-ylmethyl)-1H-imidazole (6) (K-1).
¹H NMR (CD₃OD): 8.80 (s, 1H); 7.34 (s, 1H); 6.57 (s, 1H); 4.18 (s, 2H); 2.65 to 2.69 (m, 2H); 2.51 to 2.55 (m, 2H); 1.74 to 1.83 (m, 4H)

### Example K-2

2-(Tert-butyl) furan is substituted into the method of Example K-1 to yield 4(5)-(5-tert-butylfuran-2-ylmethyl)-1H-imidazole

### Example K-3

5,6-Dihydro-4H-thieno[2,3-b]thiopyran is substituted into the method of Example K-1 to yield 4(5)-(5,6-dihydro-4H-thieno[2,3-b]thiopyran-2-ylmethyl)-1H-imidazole

### Example L

Procedure for Preparation of 4(5)-(1-furan-2-ylethyl)-1H-imidazole:

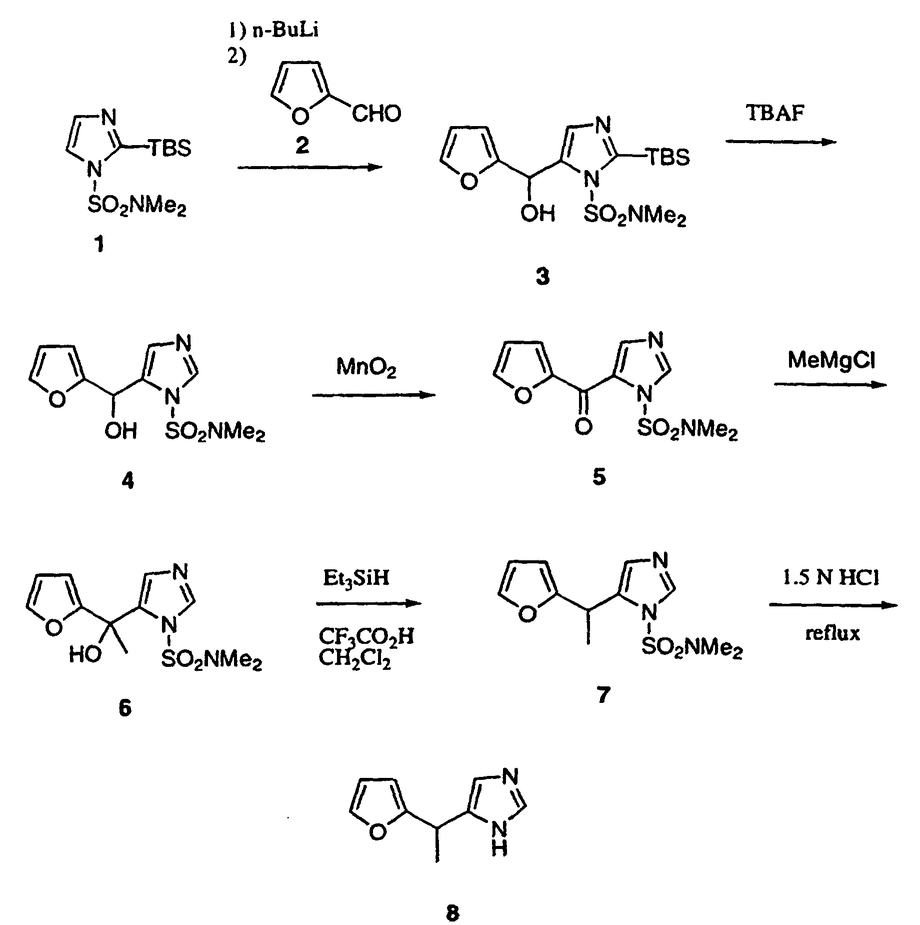

### Procedure -

2-(Tert-butyldimethylsilyl)-1-(dimethylsulfamoyl)imidazole (1) (3.3 g, 11.4 mmol) is taken up in 38mL of anhydrous THF and cooled to -78°C. n-BuLi (7.2mL, 11.4 mmol) is added dropwise to the solution of (1). The resultant solution is stirred at -78°C for 30 min. 2-Furfural (2) (0.94mL, 11.4 mmol) is added to the reaction. The reaction is warmed to rt and stirred overnight. The next day the reaction is quenched with saturated ammonium chloride and diluted with ethyl acetate. The organic layer is washed with water followed by brine. The organic phase is dried over sodium sulfate and the solvent removed under reduced pressure. Flash chromatography (4:1 ethyl acetate/ hexane) affords 4.4g (11.4 mmol) of 2-(t-butyldimethylsilyl)-5-(furan-2-ylhydroxy-methyl)imidazole-1-sulfonic acid dimethylamide (3). (3) (4.4g, 11.4 mmol) is taken up in 110mL of THF and cool to 0°C. A 1M solution of tetra-n-butylammonium fluoride (TBAF) in THF (11.4mL, 11.4 mmol) is added dropwise to the solution of (3). The reaction is stirred overnight at rt. The next day the reaction is quenched with water and then extracted with ethyl acetate. The organic layer is washed with water followed by brine. The organic phase is dried over sodium sulfate and the solvent removed under reduced pressure. 3.9g of crude 5-(furan-2-ylhydroxymethyl)imidazole-1-sulfonic acid dimethylamide (4) is recovered. (4) (1.0g, 3.7 mmol) is taken up in 37mL of dichloromethane, to the solution is added 1.6g (18.5 mmol) of manganese dioxide. The reaction is stirred at rt overnight and then filtered through celite. The eluent is collected and the solvent removed under reduced pressure. Flash chromatography using a 1:1 mixture of ethyl acetate and hexane affords 0.69g (2.6 mmol) of 5-(furan-2-ylcarbonyl)imidazole-1-sulfonic acid dimethylamide (5). (5) (0.69g, 2.6 mmol) is taken up in 26mL of THF. The solution is cool to -78°C. 1.7mL (5.1 mmol) of a 3M solution of methylmagnesium chloride is added. After stirring at -78° C for 1.5h reaction is warmed to rt and stirred for an additional hour. The reaction is quenched with water and then extracted with ethyl acetate. The organic layer is washed with water followed by brine. The organic phase is dried over sodium sulfate and the solvent removed under reduced pressure. Crystallization from ether/hexane affords 0.39g (1.4 mmol) of 5-(1-furan-2-yl-1-hydroxyethyl)imidazole-1-sulfonic acid dimethylamide (6). An additional 0.19g of (6) is recovered. (6) (0.58g, 2.0 mmol) is taken up in 27mL of dichloromethane, to the solution is added 2.6 mL (16.3 mmol) of triethylsilane and 5.5 mL (71.4 mmol) of trifluoroacetic acid. The reaction is stirred at rt overnight and then quenched with water and neutralized with solid sodium bicarbonate. The organic layer is washed with water followed by brine. The organic phase is dried over sodium sulfate and the solvent removed under reduced pressure.
Flash chromatography using a 2:1 mixture of ethyl acetate and hexane affords 0.53g (2.0 mmol) of 5-(1-furan-2-ylethyl)imidazole-1-sulfonic acid dimethylamide (7). (7) (0.34g, 1.3 mmol) is taken up in 10mL of a 1.5N HCl solution and heated at reflux for 30min and then stirred at rt overnight. The reaction is diluted with ethyl acetate and then made basic with 2N NaOH. The organic layer is washed with water followed by brine. The organic phase is dried over sodium sulfate and the solvent removed under reduced pressure. Flash chromatography (10:1 chloroform/methanol) affords 0.1g (0.62 mmol) of 4(5)-(1-furan-2-ylethyl)-1H-imidazole (8) (L).
^{**1**}**H NMR** (300 MHz, CDCl₃) 7.56 (m, 1H), 7.33-7.34 (m, 1H), 6.81 (m, 1H), 6.29-6.31 (m,1H), 6.06-6.07 (m,1H), 4.22 (q, J= 7.2 Hz, 1H), 1.63 (d, J= 7.2 Hz, 3H).

### Example M

### Procedure for Preparation of 4(5)-(2,3-dihydrobenzo[1,4]dioxin-6-ylmethyl)-4-methyl-1H-imidazole :

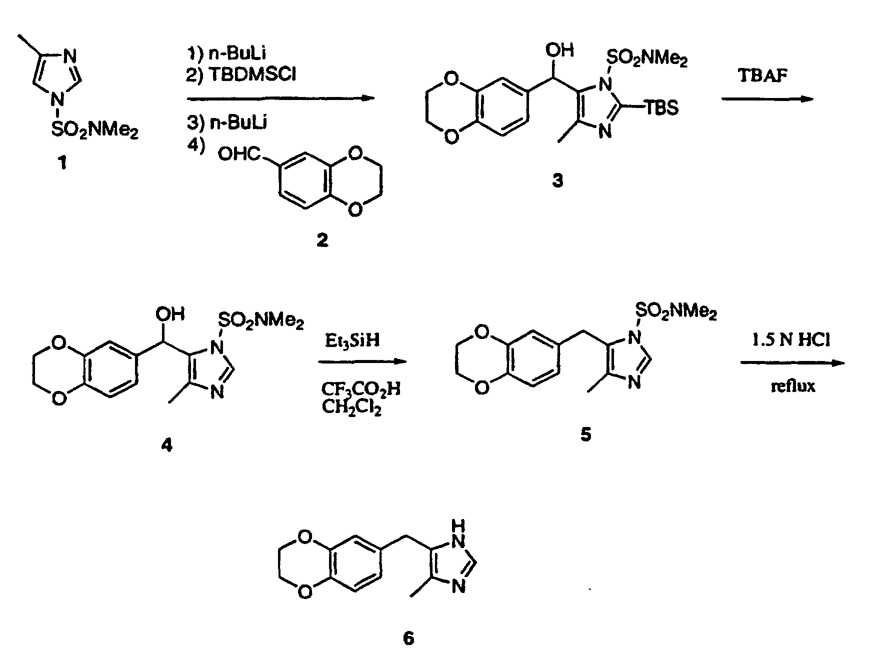

### Procedure -

4-Methyl-1-(dimethylsulfamoyl)imidazole (1) (2.0g, 10.6 mmol) is taken up in 42mL of anhydrous THF and cooled to -78°C. n-BuLi (6.6mL, 10.6 mmol) is added dropwise to the solution of (1). The resultant solution is stirred at -78°C for 30 min. Tert-butyldimethylsilylchloride (TBSCI) (1.6g, 10.6 mmol) in 10mL of THF is added to the reaction. The reaction is warmed to rt and stirred overnight. The next day the reaction is cooled to -20°C and 7.3mL (11.6 mmol) of n- BuLi added. After stirring at -20°C for 30 min, 1,4-benzodioxan-6-carboxaldehyde (2) (1.92g, 11.7 mmol) in 10mL of THF is added to the reaction mixture. Then reaction is warmed to rt and stirred for 3h. The reaction is quenched with water and diluted with ethyl acetate. The organic layer is washed with water followed by brine. The organic phase is dried over sodium sulfate and the solvent removed under reduced pressure. Flash chromatography (1:2 ethyl acetate/ hexane) affords 3.9g (8.4 mmol) of 2-(t-butyldimethylsilyl)-5-[(2,3-dihydro benzo[1,4]dioxin-6-yl)hydroxymethyl]-4-methylimidazole-1-sulfonic acid dimethylamide (3). (3) (1.0g, 2.14 mmol) is taken up in 21mL of THF. A 1M solution of tetra-n-butylammonium fluoride (TBAF) in THF (2.35mL, 2.35 mmol) is added dropwise to the solution of (3). The reaction is stirred for 30min at rt. The reaction is quenched with water and then extracted with ethyl acetate. The organic layer is washed with water followed by brine. The organic phase is dried over sodium sulfate and the solvent removed under reduced pressure. Flash chromatography using ethyl acetate as eluant affords 0.75g (2.12 mmol) 5-[(2,3-dihydrobenzo[1,4]dioxin-6-yl)hydroxymethyl]-4-methylimidazole-1-sulfonic acid dimethylamide (4). (4) (0.75g, 2.12 mmol) is taken up in 28mL of dichloromethane, to the solution is added 2.7mL (17.0 mmol) of triethylsilane and 5.2mL (67.8 mmol) of trifluoroacetic acid. The reaction is stirred at rt overnight and then quenched with water and neutralized with solid sodium bicarbonate. The organic layer is washed with water followed by brine. The organic phase is dried over sodium sulfate and the solvent removed under reduced pressure. Flash chromatography using a 3:1 mixture of ethyl acetate and hexane affords 0.63g (1.87 mmol) of 5-(2,3-dihydrobenzo[1,4]dioxin-6-ylmethyl)-4-methylimidazole-1-sulfonic acid dimethylamide (5). (5) (0.63g, 1.87 mmol) is taken up in 10mL of a 1.5N HCl solution and heated at reflux for. The reaction is diluted with ethyl acetate, neutralized with solid sodium bicarbonate. The organic layer is washed with water followed by brine. The organic phase is dried over sodium sulfate and the solvent removed under reduced pressure. Crystallization from ether/hexane affords 0.33g (1.43 mmol) of 4(5)-(2,3-dihydrobenzo[1,4]dioxin-6-ylmethyl)-4-methyl-1H-imidazole (**6**) (M).
^{**1**}**H NMR** (300 MHz, acetone-d⁶) 7.37 (s, 1H), 6.66-6.67 (m, 3H), 4.18 (s, 4H), 3.73 (s,1H), 2.13 (s, 3H)

### Example N

Procedure for Preparation of 2-(3H-imidazol-4(5)-ylmethyl)-3,4,5,6,7,8-hexahydro-2H-naphthalen-1-one (N-1), 4(5)-(2,3,4,4a,5,6,7,8-octahydronaphthlen-2-ylmethyl)-1H-imidazole (N-2) and 4(5)-(1,2,3,4,5,6,7,8-octahydronaphthalen-2-ylmethyl)-1H-imidazole (N-3):

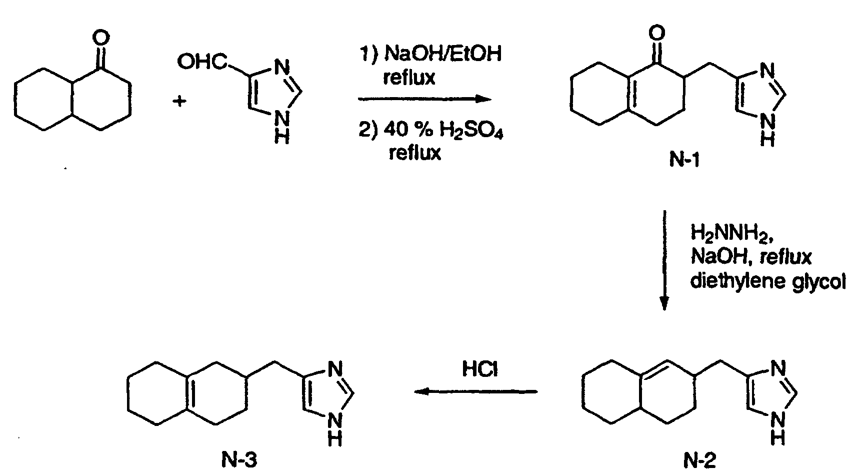

### Procedure :

1-Decalone (10.0g, 66 mmol) and 4(5)-imidazole carboxaldehyde (6.3g, 66 mmol) were added to 100 mL of ethanol. To the solution was added NaOH (5.2g, 130 mmol) in 20 mL of water. The reaction was heated at reflux for 5 days. The reaction was cooled to rt and made basic with aqueous HCl. The solution was extracted with THF/ethyl acetate. The organic layers were combined and washed with brine. The organic phase was dried over magnesium sulfate and the solvent removed under reduced pressure to afford the crude product. The crude product was heated at reflux in 40% H₂SO₄ for 1 day. The reaction was cooled to rt and made basic with saturated K₂CO₃. The solution was extracted with THF/ethyl acetate. The organic layers were combined and washed with brine. The organic phase was dried over magnesium sulfate and the solvent removed under reduced pressure. Purification by flash chromatography (15:1 CH₃Cl/MeOH) afforded N-1 (4.9g, 32% yield).
¹H NMR: 7.55 (s,1H), 6.77 (s, 1H), 3.08-3.14 (m, 2H), 1.52-2.46 (m, 13H).

The free base of the hydrochloride salt of N-1 (3.0g, 11 mmol) was generated with NaOH and then added to diethylene glycol (100mL). To the solution was added hydrazine hydrate (3.2 mL, 100 mmol) and the reaction was left to stir overnight at rt. NaOH (3.1g, 77 mmol) was added and the solution heated at reflux for 5 days. The reaction was cooled to rt and diluted with water. The solution was extracted with THF/ethyl acetate. The organic layers were combined and washed with brine. The organic phase was dried over magnesium sulfate and the solvent removed under reduced pressure. Purification by flash chromatography (8:1 CH₃Cl/MeOH) afforded N-2 (0.64g, 27% yield).
¹H NMR: 7.58 (s,1H), 6.76 (s, 1H), 5.24 (d, J= 4.3 Hz, 1H), 0.91-2.58 (m, 16H).

N-2 (1.0g, 4.6 mmol) was added to 10 mL of concentrated HCl. The solution was stirred at rt for 30 min and then neutralized with K₂CO₃. The solution was extracted with THF/ethyl acetate. The organic layers were combined and washed with brine. The organic phase was dried over magnesium sulfate and the solvent removed under reduced pressure. Purification by flash chromatography (15:1 CH₃Cl/MeOH) afforded N-3.
¹H NMR: 7.54 (s,1H), 6.74 (s, 1H), 2.45-2.52 (m, 3H), 1.46-1.97 (m, 14H).

### Example O

### Procedure for Preparation of 4(5)-octahydro pentalen-2-ylmethyl)-1H-imidazole, hydrochloride:

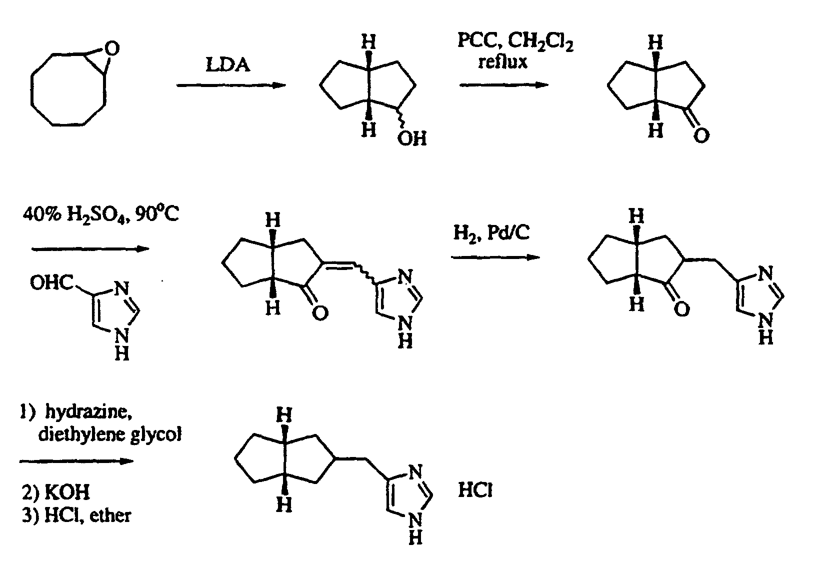

### Procedure-

A. Following the synthesis of White and Whitesell, *Synthesis* pp. 602-3 (1975), ether (10 mL) was added to a flame-dried flask cooled to 0°C and then kept under an argon atmosphere. Then n-butyl lithium (35 mL of 2.5 M solution in hexane, 2.2 equiv.) was added and subsequently diisopropyl amine (14 mL, 2.5 equiv.) was added slowly and the mixture was allowed to stir for 30 min. at 0°C. To this generated solution of lithium diisopropyl amide was added cyclooctene oxide (5.0 g, 1.0 equiv.). The mixture was stirred at rt for one day and then heated to reflux under argon atmosphere for 2 days. The reaction was quenched by addition of NH₄Cl. The solution was extracted with THF/EtOAc. The organic extracts were combined, washed with brine, dried over magnesium sulfate and concentrated in vacuo to afford a yellow brown oil which was the 1-hydroxyoctahydropentalene. The compound was used without further purification in the next step.
B. The alcohol thus obtained (5.0 g, 1 equiv.) was dissolved in dichloromethane (200 mL) and to this solution was added pyridinium chlorochromate (13 g, 1.5 equiv.) and the mixture was stirred at rt for one day. The solution was then filtered through a short column of SiO₂ using diethyl ether as eluent. The obtained solution was concentrated in vacuo to afford a pale green-yellow oil which was used without further purification in the next step.
C. The octahydro-pentalen-1-one (5.0 g, 1.0 equiv.) of the above step was added to 4(5)-imidazolecarboxaldehyde (3.8 g, 1.0 equiv.) and 40% H₂SO₄ (20 ml) and the mixture was maintained at 90°C for 3 days. The reaction was then quenched by addition of ammonium hydroxide and extracted with tetrahydrofuran/ethyl acetate. The organic extracts were combined, washed with brine, dried over magnesium sulfate. The resulting aqueous layer was neutralized with HCl/ NH₄Cl. The aqueous layer was re-extracted as above and the combined organic fractions were concentrated in vacuo to afford an orange solid.
D. This orange solid was dissolved in ethanol to which palladium on carbon (0.5 g) was added. The reaction flask was placed under 40 psi of hydrogen for one day. The reaction solution was filtered though celite with more ethanol used as eluent. The solution was concentrated in vacuo to afford a yellow brown oil. Purification by column chromatography using 17:1 chloroform/methanol afforded the ketone product in a somewhat impure state.
E. The ketone functionality was then removed by addition of the product of the step above (8.2 g, 1.0 equiv.) to diethylene glycol (80 mL)and hydrazine hydrate (13.0 g, 1.0 equiv.). This mixture was stirred overnight and then potassium hydroxide (11.0 g, 5.0 equiv.) was added and the solution was heated under reflux for one day. The reaction solution was cooled to rt and washed with water. The solution was extracted with THF/EtOAc and the combined fractions were washed with brine, dried over magnesium sulfate and concentrated in vacuo to afford a yellow oil. The monohyrdochloride salt was made by dissolving this oil in anhydrous ethanol saturated with HCl and heating.

### Example P

Procedure for the preparation of 7-(3H-imidazol-4(5)-ylmethyl)-6,7-dihydro-5H-isoquinolin-8-one (P-1) and 7-(3H-imidazol-4(5)-ylmethyl)-5, 6, 7, 8-tetrahydroisoquinoline (P-2)

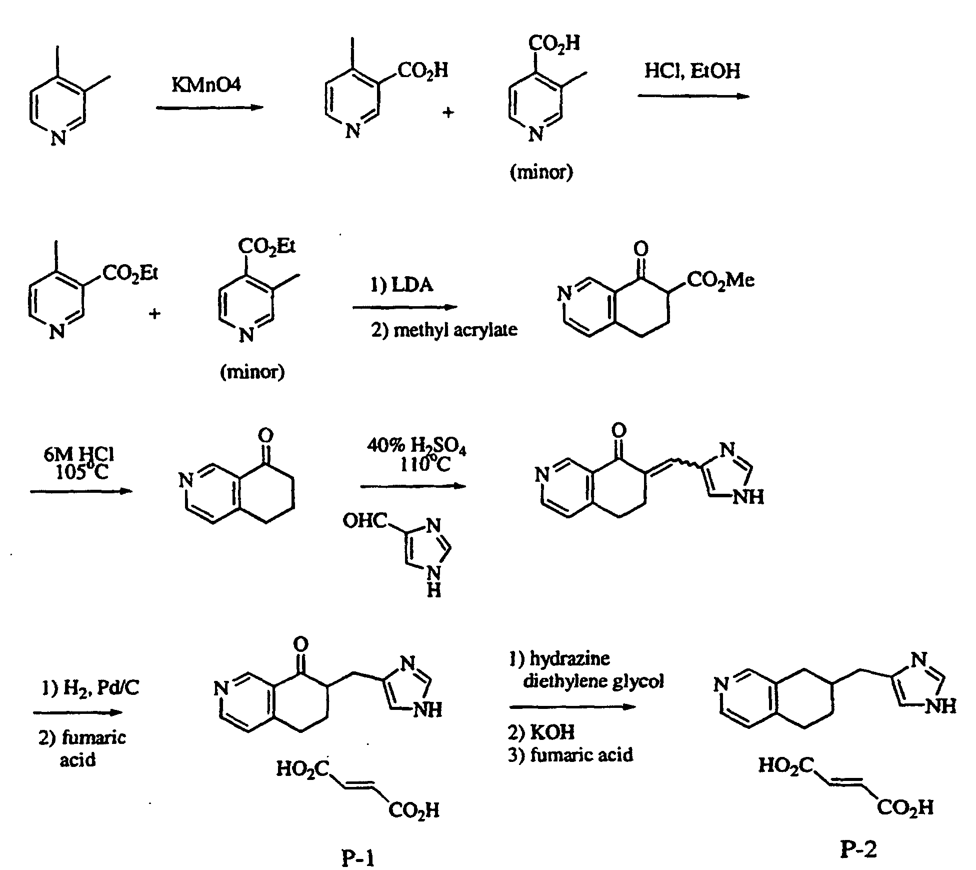

### Procedure:

A. 3,4-lutidine (21.4g, 1 equiv.) was dissolved in 200 mL of water at 20°C and potassium permanganate was added in 6.32g portions twice daily for 5 days (total 63.2g, 2 equiv.). After 5 days the solution was stored in the freezer, then thawed and filtered through celite. The resulting colorless solution was concentrated at 90°C on a rotary evaporator until a white solid was obtained. This solid was recrystallized from 5N HCl to give 9.56g of white crystals. NMR indicated a mixture of two regioisomers with the desired isomer being the major product.
B. These crystals were heated in anhydrous ethanol saturated with HCl gas under argon and at reflux for 6 h. Then ethanol was removed from the solution by rotary evaporation and the residue was taken up in 100 mL of water and the pH was adjusted to between 7 and 8 with solid sodium bicarbonate. The aqueous phase was extracted with diethyl ether (3X) and the combined organic fractions were washed with brine, dried over magnesium sulfate and then filtered and concentrated to give a colorless oil (3.56g , 10.8% yield).
C. Diisopropylamine 2.84g, 1.3 equiv.) was added to n-BuLi (11.21 mL, 1.3 equiv.) in 100 mL of anhydrous THF under argon at -78°C via syringe to produce lithium diisopropylamide in situ. To this solution was added the product of B above (3.56g, 1 equiv.) in 20 mL of tetrahydrofuran, via syringe and the mixture was stirred at -78°C for 20 min. At this point methyl acrylate (4.85 mL 2.5 equiv.) in 20 mL of tetrahydrofuran was added dropwise through a cannula. The solution was stirred another 2 h before quenching by addition of 40 mL of 10% potassium acetate. The solution was allowed to warm to 20°C and then was concentrated on a rotary evaporator. The aqueous residue was extracted three times with chloroform. The combined fractions were washed with brine and dried over magnesium sulfate, filtered and concentrated to a black solid, which was stored under high vacuum. Chromatography on silica gel with hexanes / ethyl acetate (7/3 → 6/4) afforded 2.41g (58.2%) of the desired product which was used without further purification in the next step.
D. The material from Step C (0.48g, 1 equiv.) was dissolved in 1 mL of 6M HCl and heated at 105°C for 16 h after which time the solution was concentrated to a solid by rotary evaporation at 80°C. The residue was taken up in 2 mL of water and neutralized with solid sodium bicarbonate. The neutralized solution was extracted with chloroform (3X) and the combined fractions were washed with brine, dried over magnesium sulfate and concentrated to a colorless oil. (0.456g 93.4%).
E. The isoquinolone (1.91 g, 1 equiv.) obtained in step D above was heated with 4(5)-imidazolecarboxaldehyde 1.25g, 1, equiv.) at 110°C in 15 mL of 40% sulfuric acid for 30 h. The reaction mixture was stored for several days at 0° C under argon. The solution was then diluted with 20 mL of water and basified to pH 8.9 with NH₄OH. Solids were collected by filtration and dried with high vacuum. The product was a yellow solid (2.81g, 96.1%) comprising a mixture of both positional isomers at the exo double bond.
F. The product of E, above, was dissolved in 150 mL of methanol and to this solution Pd/C (.412g, 0.15 wt. equiv.) was added. The methanolic solution was then saturated with H₂ by repeated evacuations and H₂back-fill iterations. The solution was stirred under 1 atm. pressure of H₂ for 20 h until TLC revealed that no unsaturated starting material remained. The solution was filtered through celite and concentrated to an oil. Chromatography on silica using dichloromethane and methanol (9/1) recovered pure product (1.853g 6504 %) as a white foam. This was taken up in methanol to which fumaric acid (0.4817g, 1.5 equiv.) was added with warming to dissolve the solids. The solution was cooled slowly and off-white crystals (0.826g, 74%) were obtained, which are represented as the compound P-1. P-2 was obtained by hydrazine reduction in the same manner as described in Step E of Example O above.

### Example Q

Procedure for the preparation of (Z)-6-(3H-imidazol-4(5)-ylmethylene)-7,8-dihydro-6H-quinolin-5-one (Q-1), (E)-6-(3H-imidazol-4(5)-ylmethylene)-7,8-dihydro-6H-quinolin-5-one (Q-2), 6-(3H-imidazol-4(5)-ylmethyl)-7,8-dihydro-6H-quinolin-5-one (Q-3), 6-(3H-imidazol-4(5)-ylmethyl)-5,6,7,8-tetrahydroquinoline, dihydrochloride (Q-4) and 6-(3H-imidazol-4(5)-ylmethyl)-octahydroquinolin-5-one (Q-5)

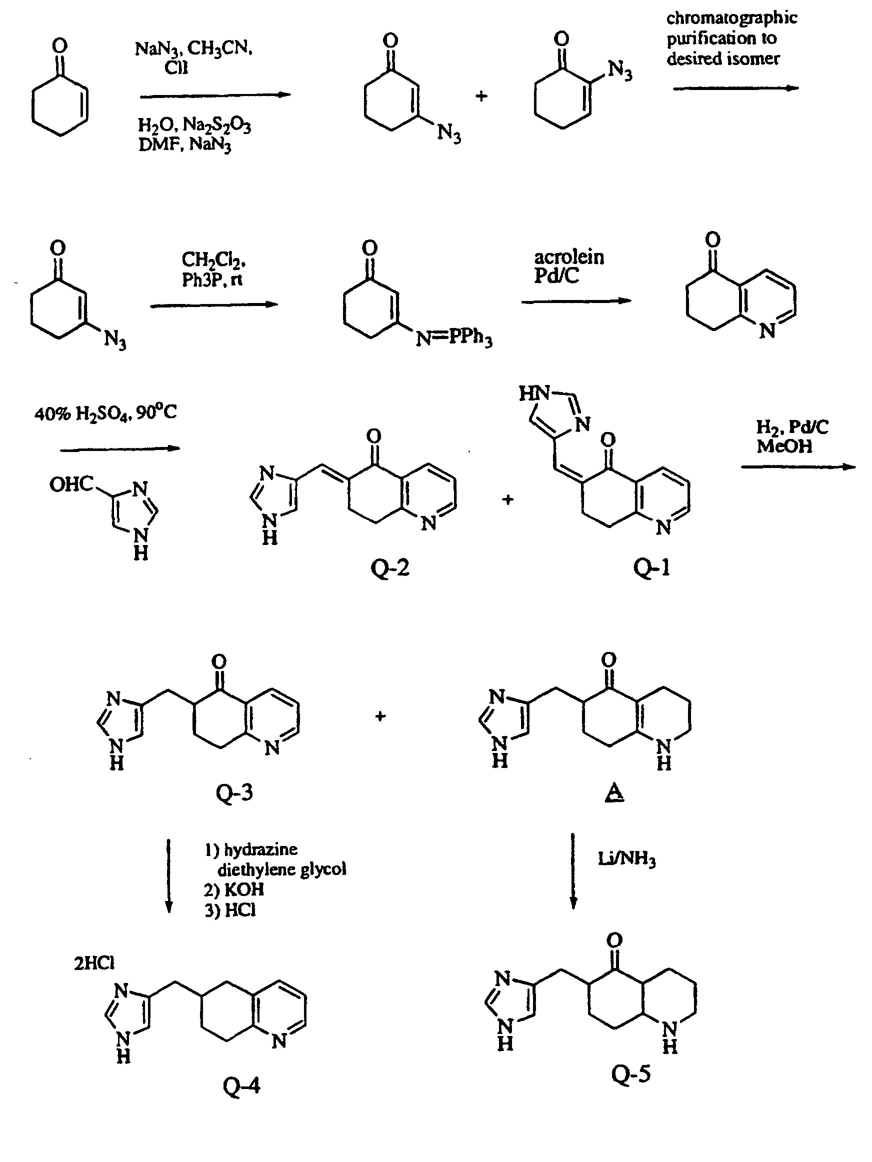

### Procedure:

A. The reactive azido reagent of the first step was generated in situ by addition of iodine monochloride (67.6 g, 1.15 equiv.) in 50 mL of acetonitrile dropwise through a dropping funnel to a stirred slurry of sodium azide (58.84 g, 2.5 equiv.) in 350 mL of anhydrous acetonitrile at -10°C and under argon. Addition was complete in 30 min, the mixture was stirred an additional 30 min and cyclohexenone (34.81 g, 1.0 equiv.) was added via a syringe and then stirred at 20°C for an additional 20 h. The mixture was then poured into a liter of water and extracted with three 200 mL portions of diethyl ether. The combined fractions were washed with 5% sodium thiosulfate solution and then brine. The organic phase was dried over magnesium sulfate, filtered and concentrated in vacuo at 20°C. The residues were taken up in 1 L of DMSO at 0°C and a second portion of NaN₃ was added and the mixture stirred while warming to ambient temperature. This mixture was then diluted with 2.5 L of ice water and extracted ten times with dichloromethane (10 X 250 mL). The combined organic fractions were concentrated on a rotovap to a volume of ∼1 L and this concentrate was extracted three times with 250 mL of water, and then brine, and then dried over magnesium sulfate and concentrated to a dark oil (39.5 g) and stored at -40°C.
   The oil was purified by chromatography on silica using 9/1 to 8/2 hexane:ethyl acetate. Two isomers were recovered, the first with the azido group α to the ketone function was obtained in 13.22 g, 26.6%, yield. The β-isomer was obtained in 15.825 g, 32.0%, yield.
B. Triphenyl phosphine was dissolved in 20 mL of dichloromethane and placed under an argon atmosphere at 20 °C. The β-isomer obtained as described above was added via cannual to the stirred solution and maintained at 20°C for 2 h. As the reaction progressed nitrogen was liberated from the solution, and after 2 h TLC demonstrated there was no starting material remaining. The solution was concentrated and passed through a silica gel column with dichloromethane progressing to 95/5 dichloromethane:methanol as eluent. The amidophosphonate intermediate was obtained in 2.139 g, 65.1%, yield.
C. The amidophosphonate was dissolved in 100 mL of anhydrous o-xylene and then 10% Pd / C was added with stirring. Freshly distilled acrolein was then added to the mixture via syringe and heated to reflux for 4 h, after which time the remaining acrolein was added and heating under reflux was continued for 44 h under a finger condenser and under argon. At that time TLC indicated some intermediate remained, so 0.5g addition Pd/ C was added and the mixture again was heated to reflux for another 8 h. The mixture was cooled to rt, filtered and concentrated on a rotovap to eliminate excess acrolein, until about 100 mL of o-xylene solution remained. This solution was cooled by addition of ice, and was extracted three times with 1N HCl. The combined aqueous fractions were extracted 3X with Et₂O. The aqueous phase was then cooled to 0°C and the pH was adjusted to -10 using concentrated NaOH. The aqueous was then extracted 5X with 100 mL portions of chloroform. The combined chloroform fractions were washed with water and then brined and dried over magnesium sulfate, filtered, and finally concentrated to give 3.51 g of an oil in 84.4% yield of 7,8-dihydro-6H-quinolin-5-one.
D. The 4(5)-imidazole carboxaldehyde was condensed with the quinolinone as described in Step E of Example P and was obtained both Q-1 and Q-2.
E. The exo double bond was then reduced with palladium on carbon as described in Step F of Example P above to yield two products which were separated by chromatography to give Q-3 and A.
F. The keto group was removed by the same hydrazine reduction procedure as that described in Step E of Example O above to give Q4.
G. The fully-reduced quinoline ring product Q-5 was obtained by a standard reduction of A with lithium/ammonia. (Li, 10 equiv., in NH₃ at -78°C for 10 min, quenched with NH₄OH, gradual warming with NH₃ evaporation).

### Example R-1

### Procedure for the preparation of (E)-6-(3H-imidazol-4(5)-ylmethylene)-7, 8-dihydro-6H-quinoxalin-5-one

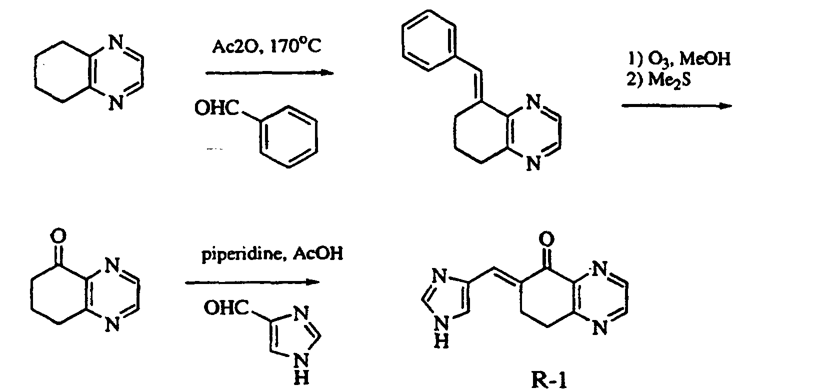

### Procedure:

A. A mixture of 5,6,7,8-tetrahydroquinoxaline (23.75g, 1 equiv.), benzaldehyde (19.81 mL, 1.1 equiv.) and acetic anhydride (33.4 mL, 2.0 equiv.) was stirred at 150°C under argon for 15 hr, after which time TLC indicated mostly desired product with some starting materials remaining. Starting materials were removed by vacuum distillation using a Vigreux column at 170°C. The pot residue was then subjected to Kugelrohr distillation from 170 - 220°C. The first fraction was slightly contaminated with starting materials (4.71g). A second fraction was pure (18.93g). After applying high vacuum to the first fraction it crystallized. Combined fractions yielded 20.1 1g, 51%.
B. The product from A, above, was dissolved in 100 mL of methanol and warmed slightly, then cooled to -35 to -40°C and ozone was bubbled through the solution. After a few minutes the starting material began to crystallize out of solution and the solution was warmed and another 200 mL of methanol was added and then the reaction was resumed. After about 30 minutes the solution turned pale blue. Nitrogen was then introduced by bubbling through the solution for 30 minutes, then methyl sulfide (3.5 mL) was injected into the solution, whereafter the solution was stirred for another 30 min. at -35°C, then allowed to warm to ambient temperature with stirring. After about 48 hr. at 20°C the mixture was steam distilled to remove solvents to provide a residue of 8.4g of a yellow-brown oil. This residue was taken up in diethyl ether and extracted 3x with 25 mL portions of 1N HCl. The combined aqueous fractions were washed with diethyl ether 3x. The aqueous solution was gradually basified to a pH of 8 with concentrated NaOH. The free amine was then extracted from the aqueous phase with chloroform (3x). The combined chloroform extracts were washed twice with brine, dried of MgSO₄ and concentrated to a yellow oil (3.01g) After keeping under high vacuum for 1 hr., 2.97g remained. This was recrystallized from diethyl ether to give 2.35g of a bright yellow solid. Yield 67.5%.
C. The 7,8-dihydroquinoxalin-5-one and 4(5)-imidazolecarboxaldehyde (Aldrich Chemicals) were suspended in 75 mL of anhydrous tetrahydrofuran at 20°C under argon followed by addition of piperidine followed by acetic acid. The mixture was stirred 16 h at 20°C. After 20 h, no traces of the quinoxalone remained as indicated by TLC. The solids were collected by filtration and washed with a small amount of tetrahydrofuran, followed by chloroform. The solid was dried under high vacuum to give 6.85g of R-1. Yield 90.3%.

### Example R-2 and R-3

In a similar manner as R-1, 5,6,7,8-tetrahydroisoquinoline (5.42g, 1 equiv., Aldrich) was stirred with benzaldehyde (5.182 g, 1.2 equiv.) and acetic anhydride (6.309 g, 2.0 g) which was vacuum distilled and used without further purification in the next step. Yield (impure): 8.28 g.
The crude product (7.96 g) from the step above was subjected to ozonolysis as described in Step B above. After work-up and chromatography there was obtained 5.18 g of a pale oil. Yield: 97.8% assuming pure starting material.
The resulting 7,8-dihydro-6H-isoquinolin-5-one (1.692 g, 1 equiv.) was condensed with 4(5)-imidazolecarboxaldehyde as described in Step C above to yield 2.23 g of the unsaturated compound analogous to R-1 in the scheme above in 92.8% yield. This product was treated with palladium on carbon as described in Step F of Example P to reduce the exo double bond to produce 6-(3H-imidazol-4(5)-ylmethyl)-7,8-dihydro-6H-isoquinolin-5-one (R-2) in 52%.
The ketone above was reduced using hydrazine and converted to the fumarate salt as detailed in Example P, Step F. Yield for the reduction: 62%. Yield of fumarate salt after recrystallization: 30.4% of 6-(3H-imidazol-4(5)-ylmethyl)-5,6,7,8-tetrahydroisoquinoline (R-3).

### Example S

A method for measuring α-agonist selectivity comprises the RSAT (Receptor Selection and Amplification Technology) assay as reported in Messier et al. (1995) "High throughput assays of cloned adrenergic, muscarinic, neurokinin and neurotrophin receptors in living mammalian cells", *Pharmacol*. *Toxicol.* **76**:308-11 and adapted for use with alpha₂ receptors. The assay measures a receptor-mediated loss of contact inhibition that results in selective proliferation of receptor-containing cells in a mixed population of confluent cells. The increase in cell number is assessed with an appropriate transfected marker gene such as b-galactosidase, the activity of which can be easily measured in a 96-well format. Receptors that activate the G protein, G_{q}, elicit this response. Alpha₂ receptors, which normally couple to Gᵢ, activate the RSAT response when coexpressed with a hybrid G_{q} protein that has a Gᵢ receptor recognition domain, called G_{q/i5}². See Conklin et al. (1993) "Substitution of three amino acids switches receptor specificity of G_{qa} to that of Gᵢa." *Nature* **363**:274-6.

NIH-3T3 cells are plated at a density of 2x10⁶ cells in 15 cm dishes and maintained in Dulbecco's modified Eagle's medium supplemented with 10% calf serum. One day later, cells are cotransfected by calcium phosphate precipitation with mammalian expression plasmids encoding p-SV-b-galactosidase (5-10 mg), receptor (1-2 mg) and G protein (1-2 mg). 40 mg salmon sperm DNA may also be included in the transfection mixture. Fresh media is added on the following day and 1-2 days later, cells are harvested and frozen in 50 assay aliquots. Cells are thawed and 100 ml added to 100 ml aliquots of various concentrations of drugs in triplicate in 96-well dishes. Incubations continue 72-96 hr at 37°. After washing with phosphate-buffered saline, b-galactosidase enzyme activity is determined by adding 200 ml of the chromogenic substrate (consisting of 3.5 mM o-nitrophenyl-b-D-galactopyranoside and 0.5% nonidet P-40 in phosphate buffered saline), incubating overnight at 30° and measuring optical density at 420 nm. The absorbence is a measure of enzyme activity, which depends on cell number and reflects a receptor-mediated cell proliferation. The EC₅₀ and maximal effect of each drug at each alpha₂ receptor is determined. The efficacy or intrinsic activity is calculated as a ratio of the maximal effect of the drug to the maximal effect of a standard full agonist for each receptor subtype. Brimonidine, also called UK14,304-18, is used as the standard agonist for the alpha_{2A} and alpha_{2C} receptors. Oxymetazoline is the standard agonist used for the alpha_{2B} receptor.

Table 1, below, provides the intrinsic activity values at subtypes of the α2-adrenoreceptor as determined in the RSAT assay for the compounds of above Examples B through R and certain adrenergic compounds not having selective agonist activity at the α2B or α2B /α2C subtype(s). At the α2A subtype, the compounds of the Examples are inactive or exhibit low efficacy (≤0.4). They have greater efficacy at the α2B and the α2C-subtypes than the α2A-subtype. Therefore, unlike ophthalmic α2-adrenoreceptor compounds such as clonidine and brimonidine, the compounds of Examples B through R can selectively activate α2-adrenoreceptor subtypes other than the α2A-subtype.

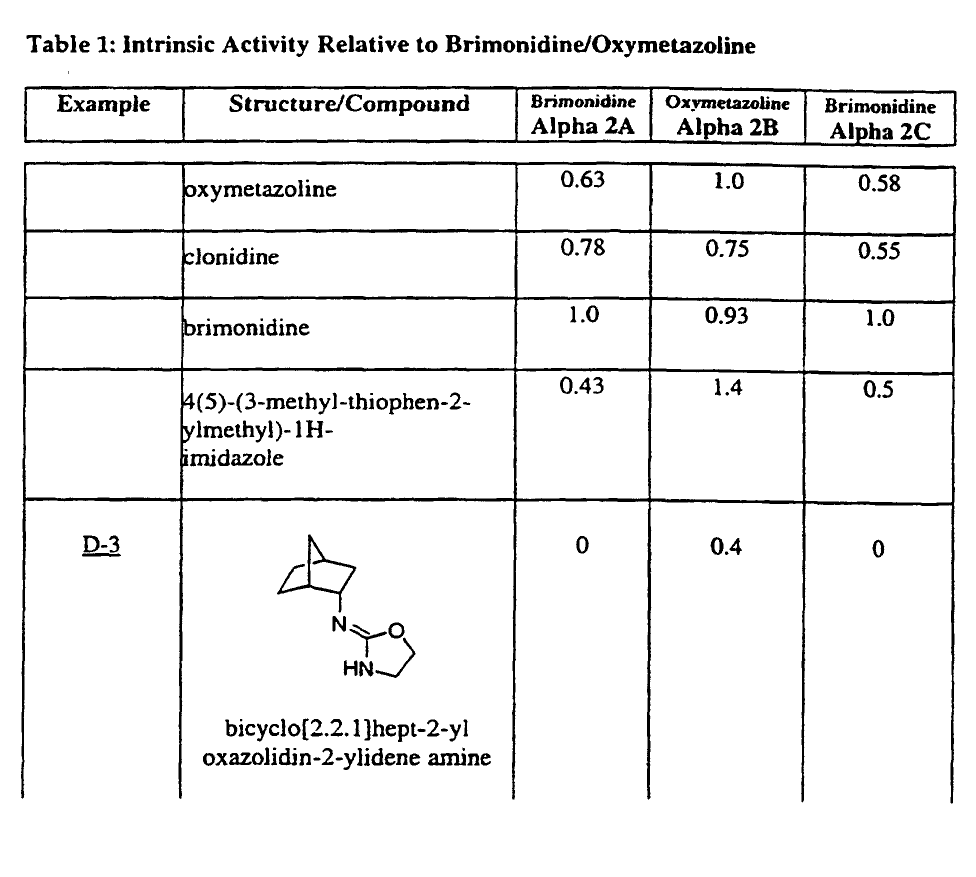

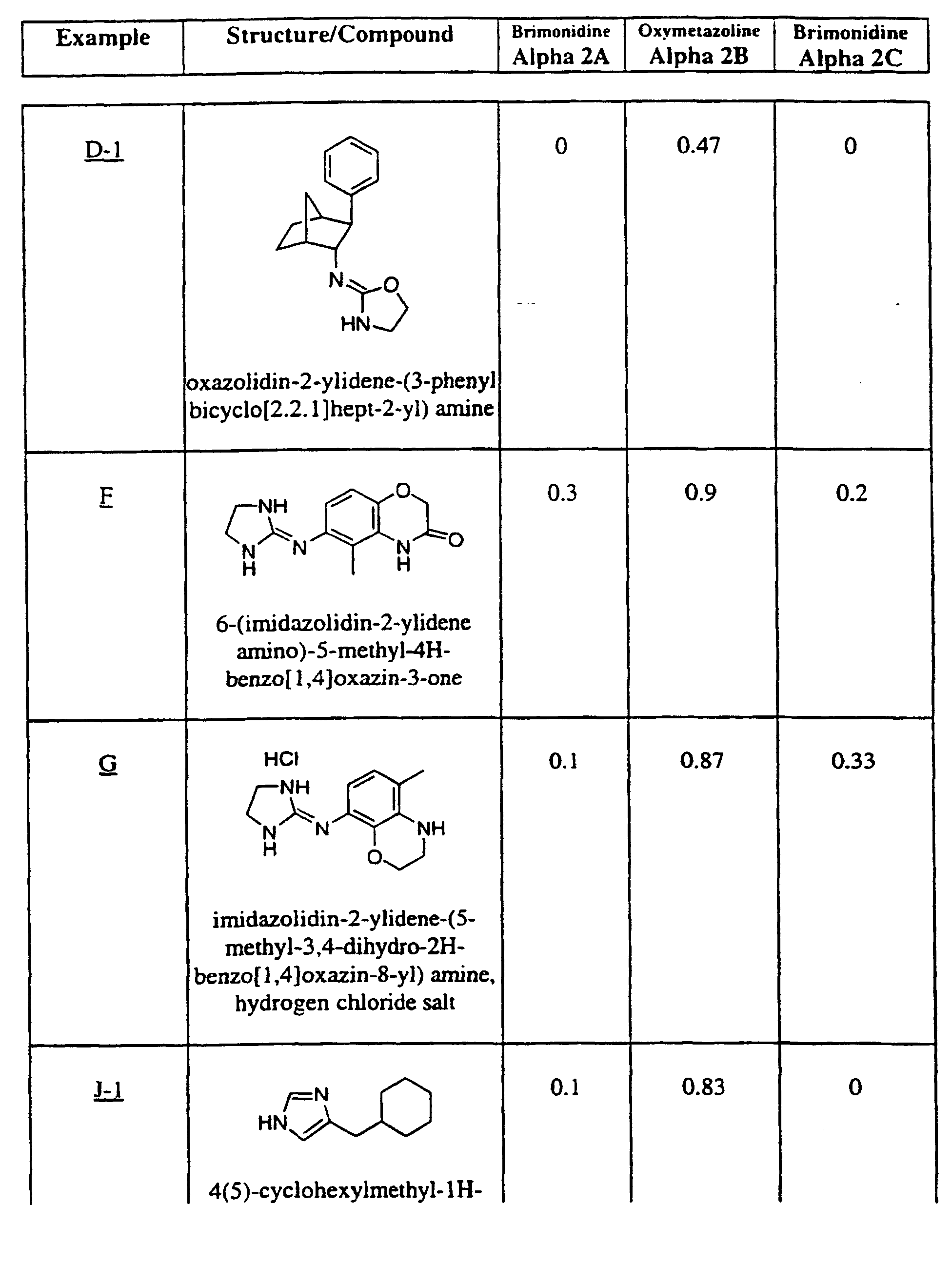

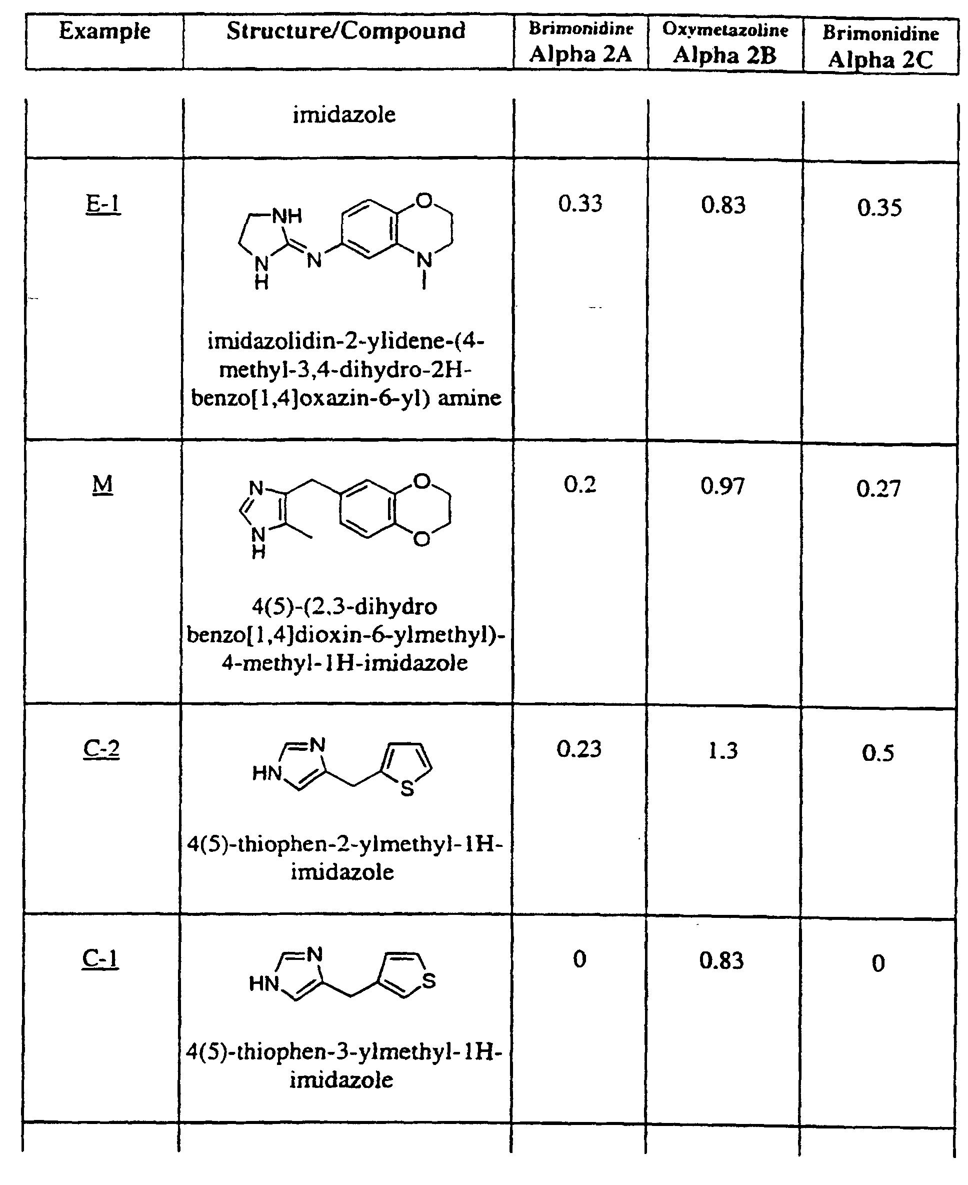

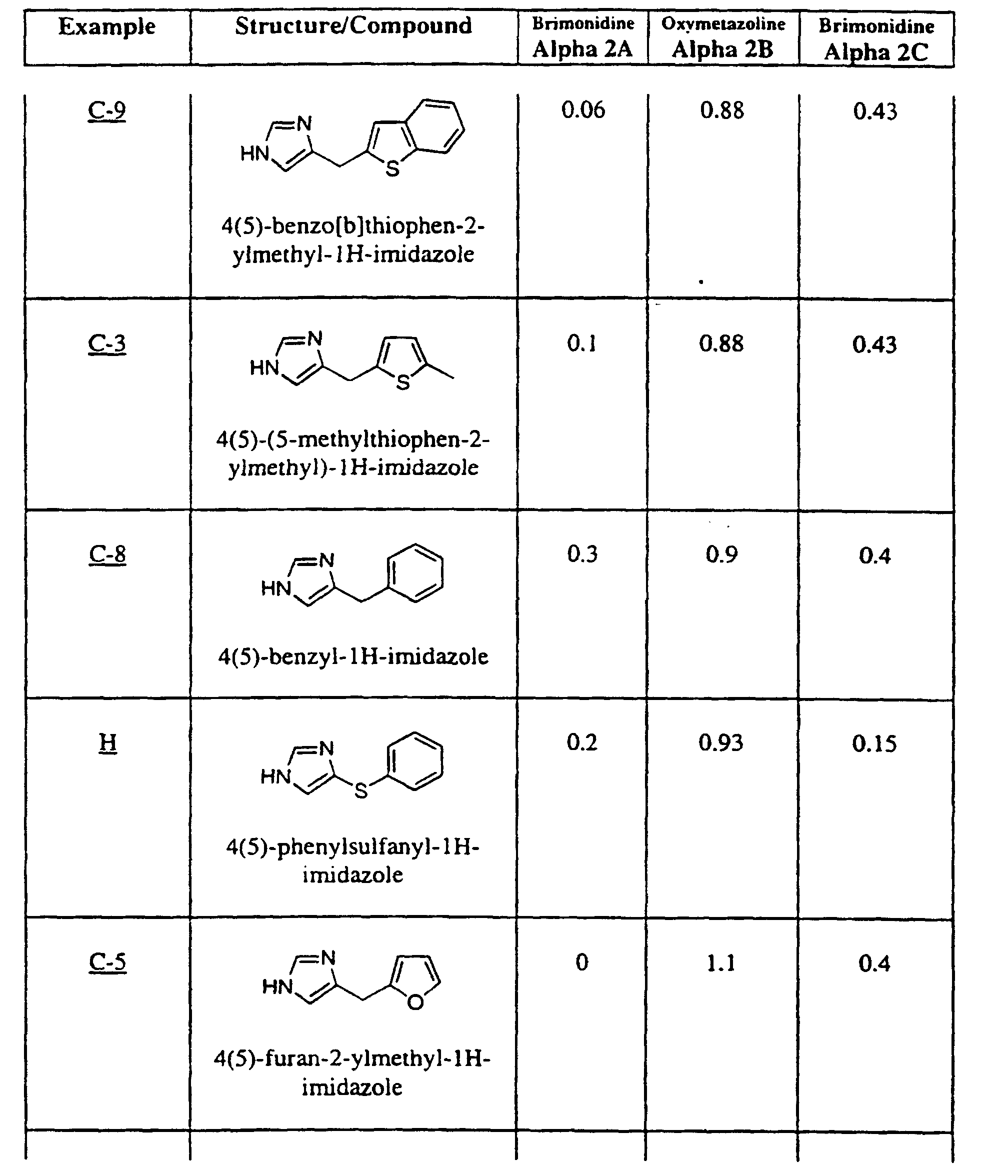

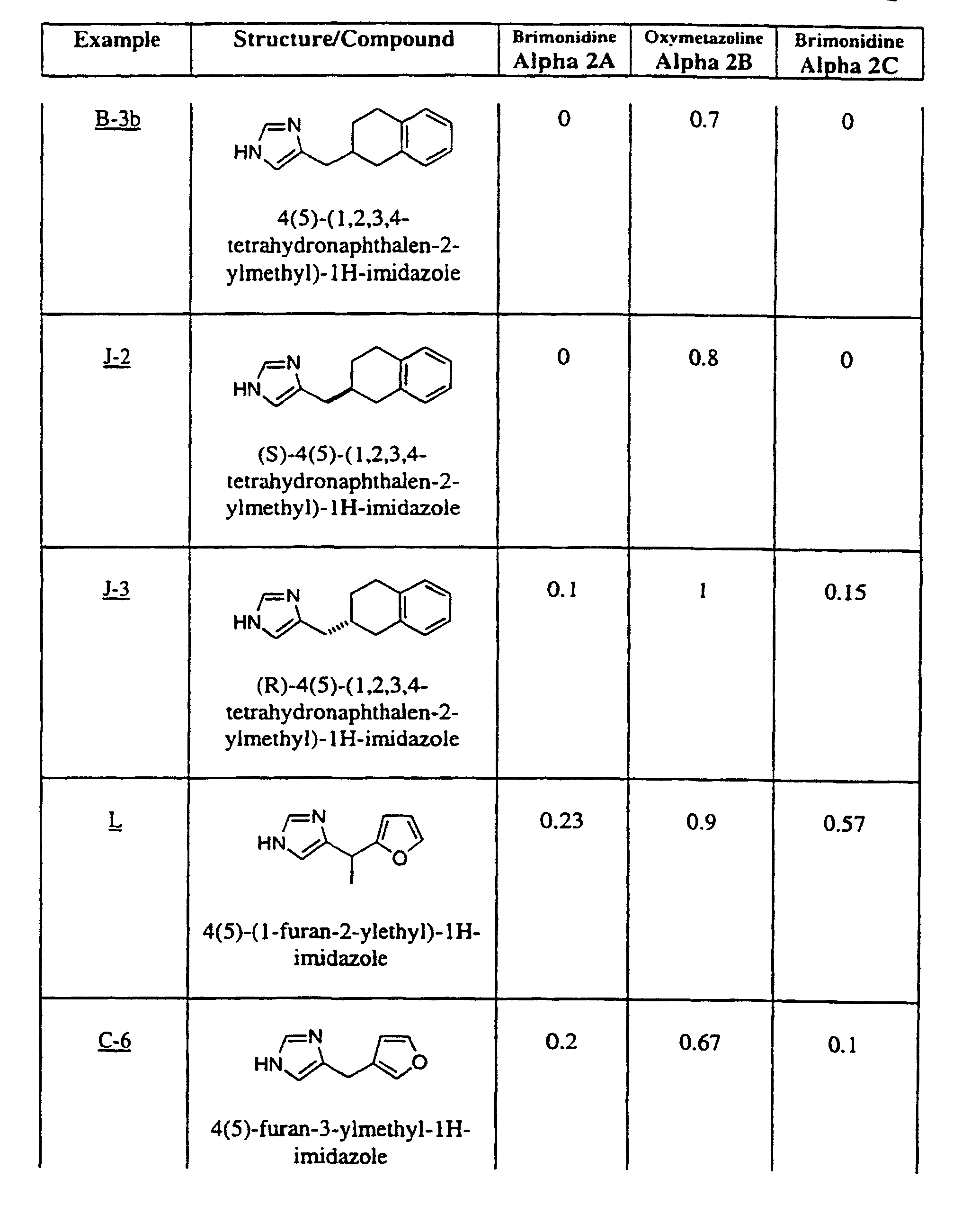

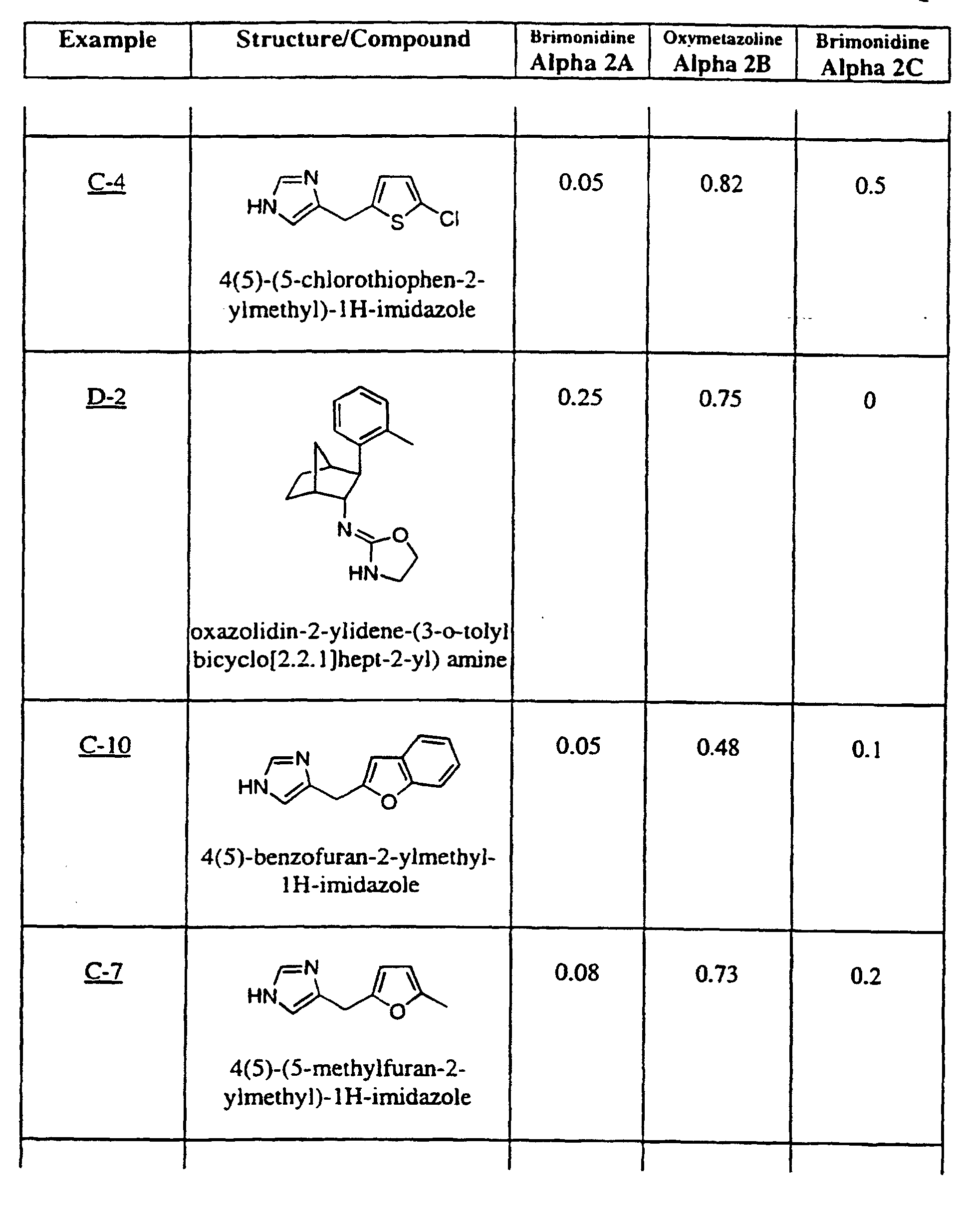

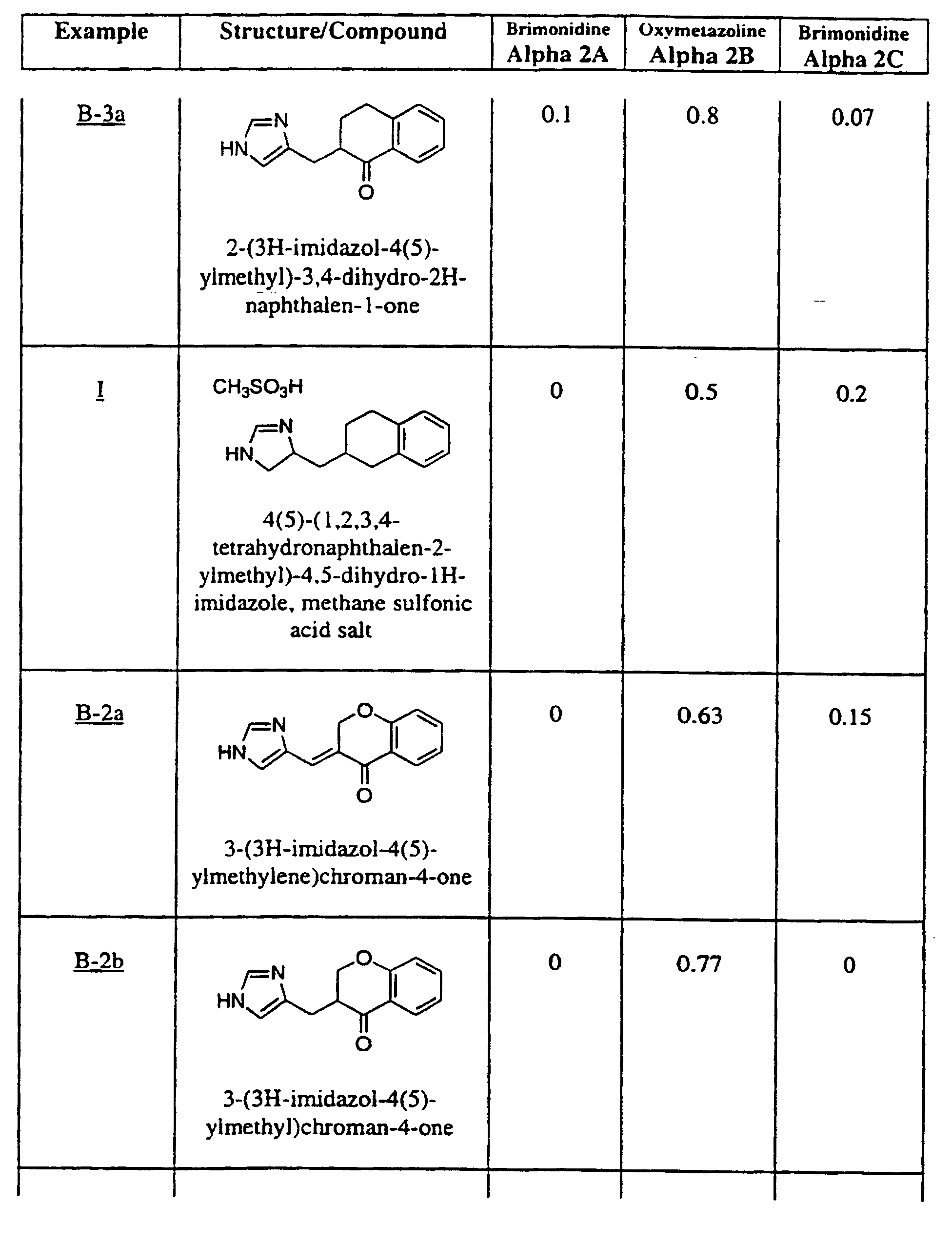

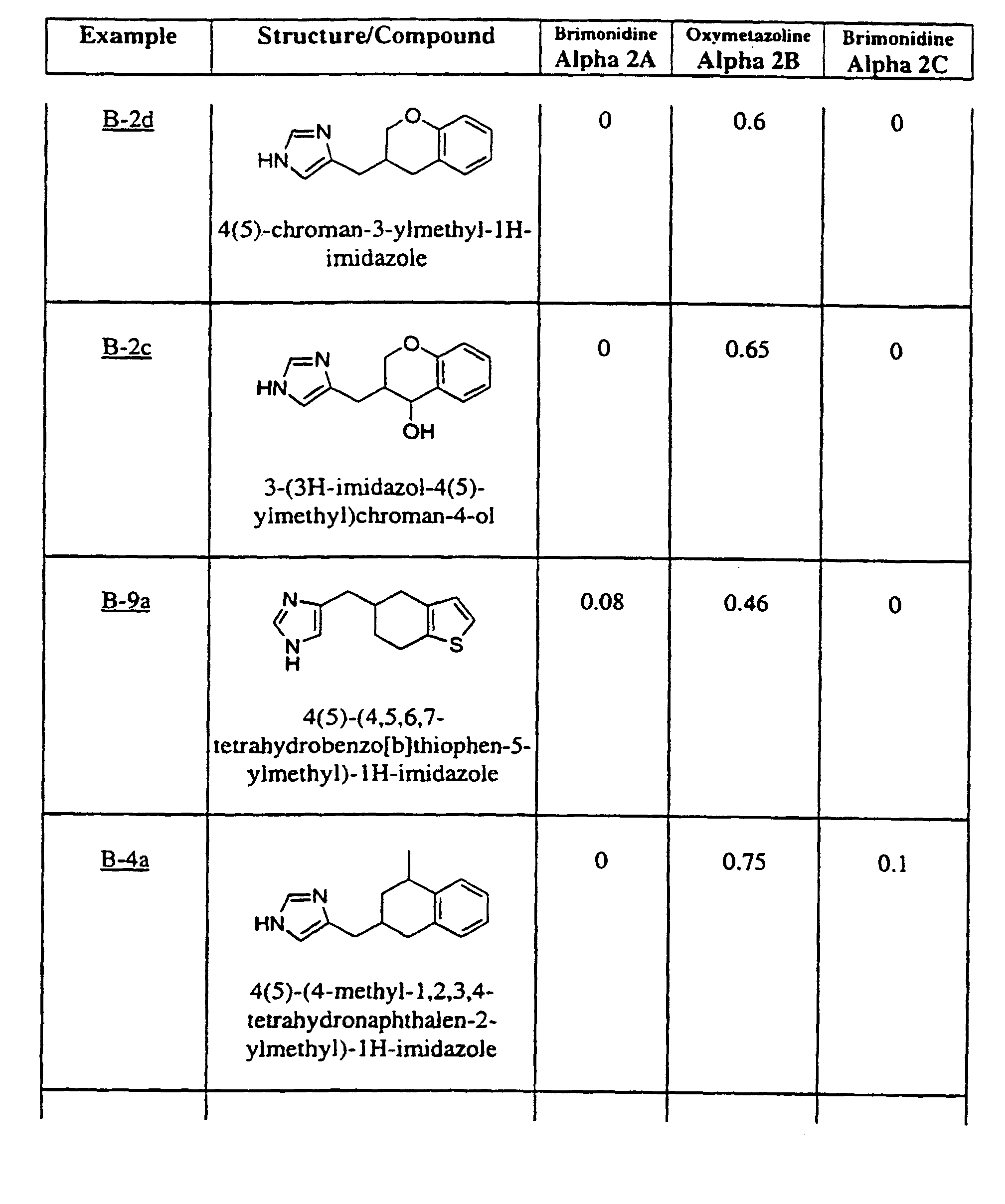

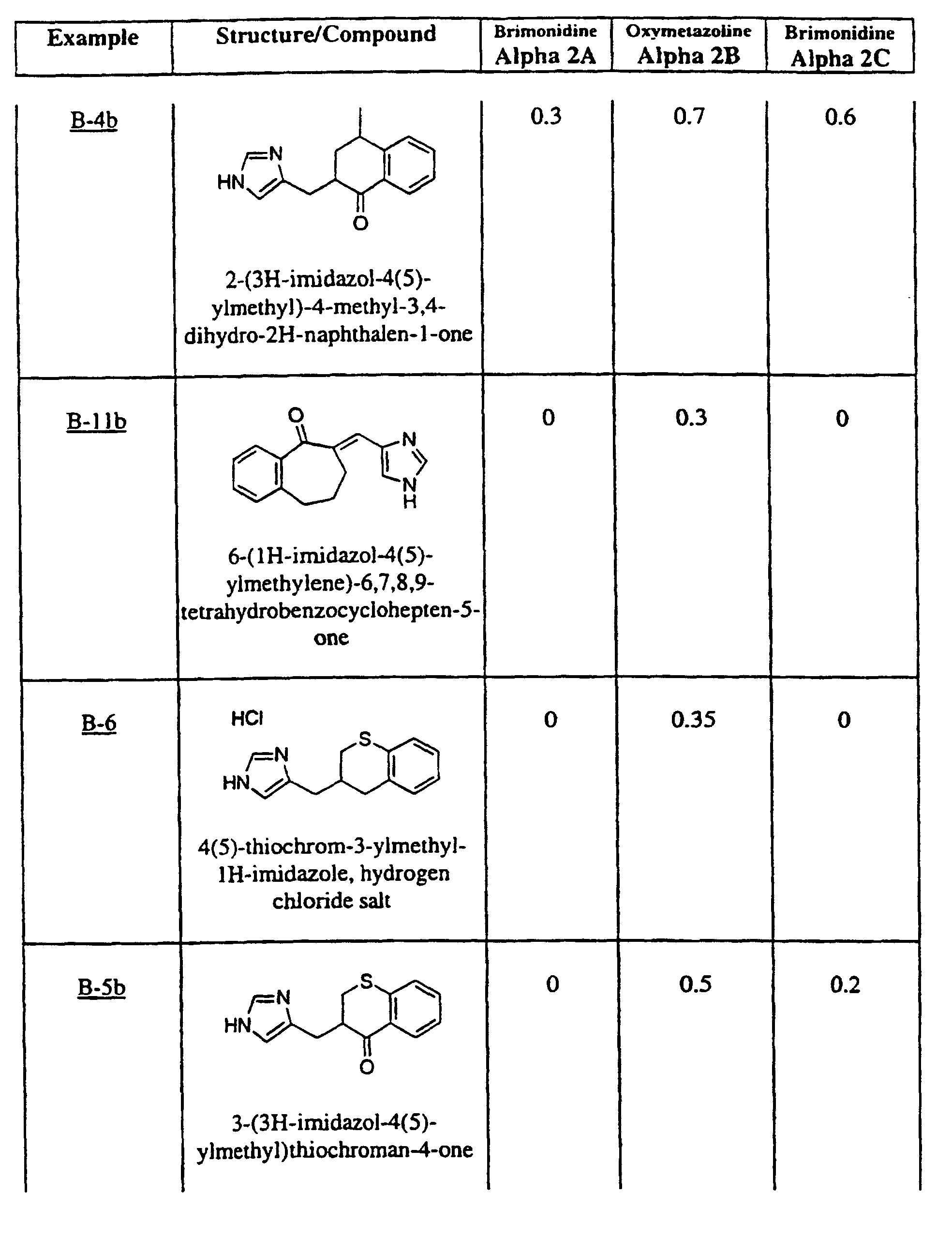

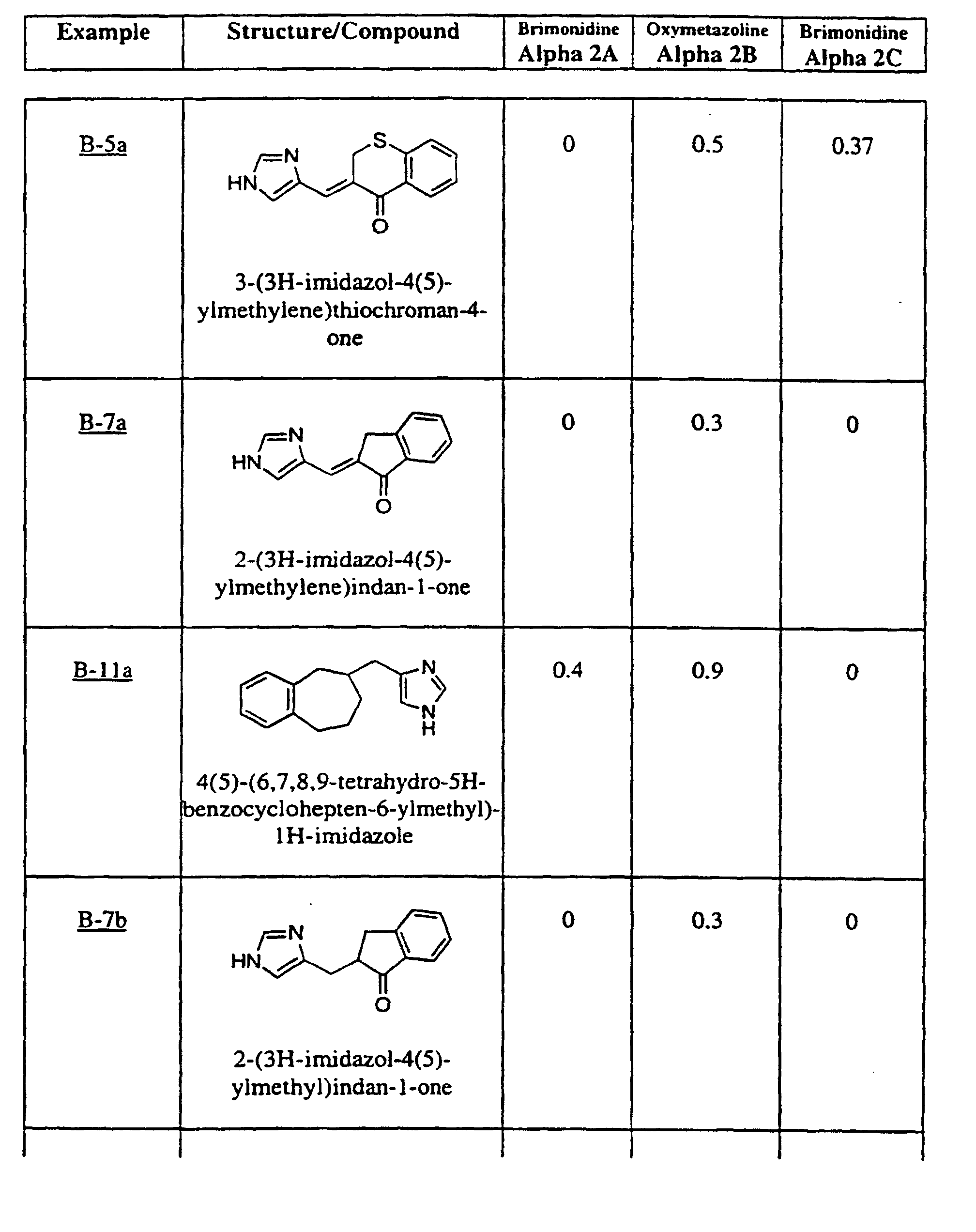

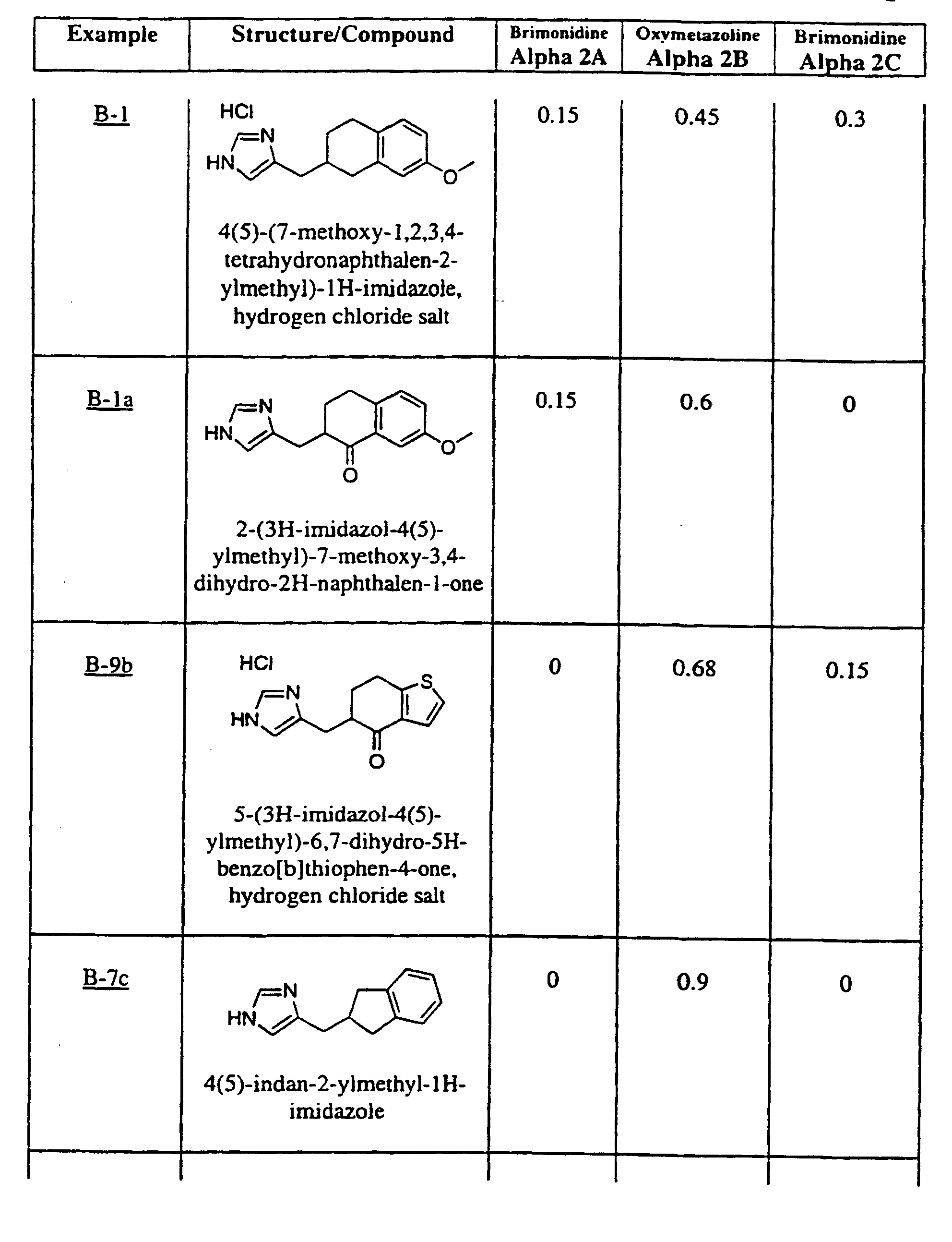

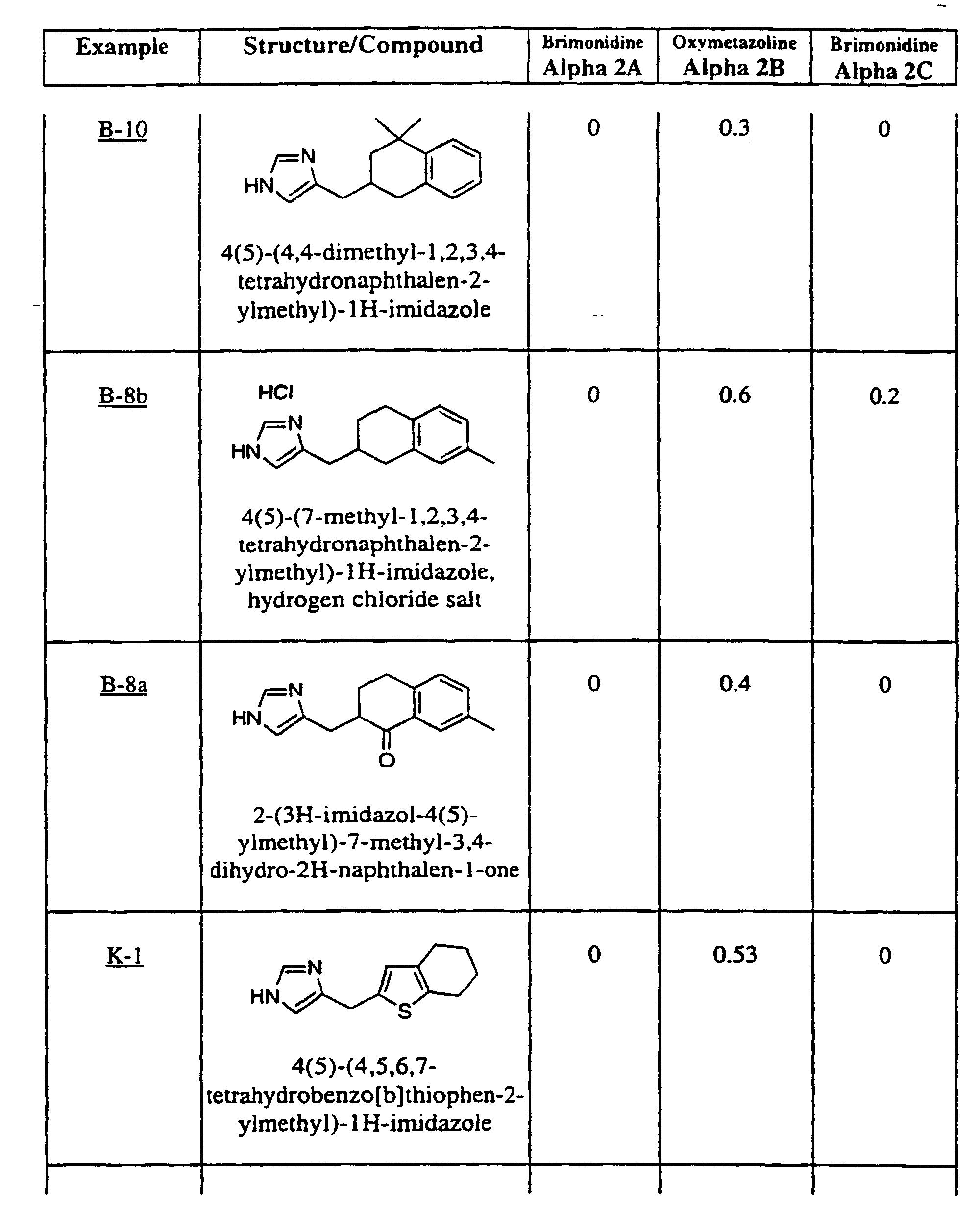

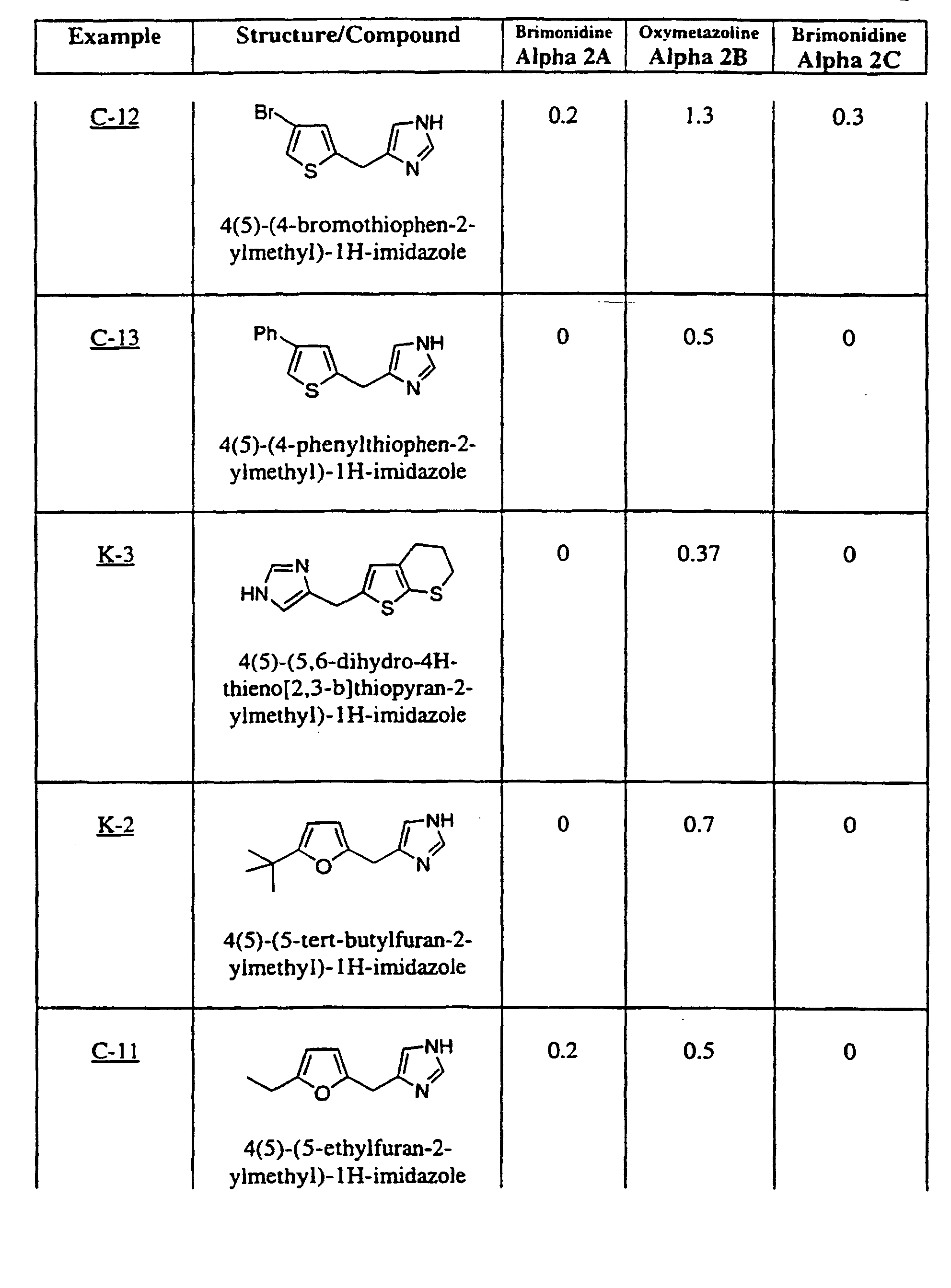

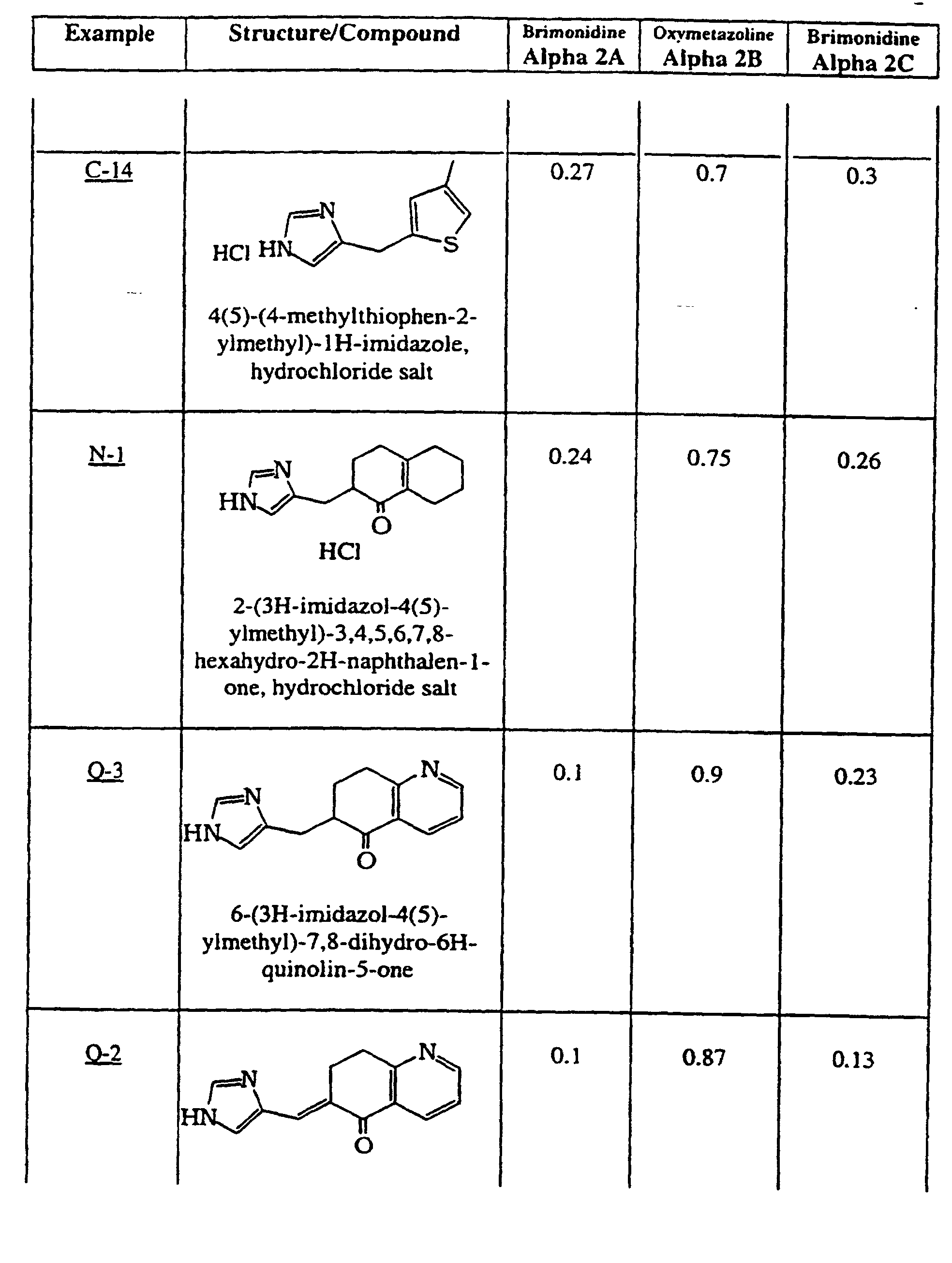

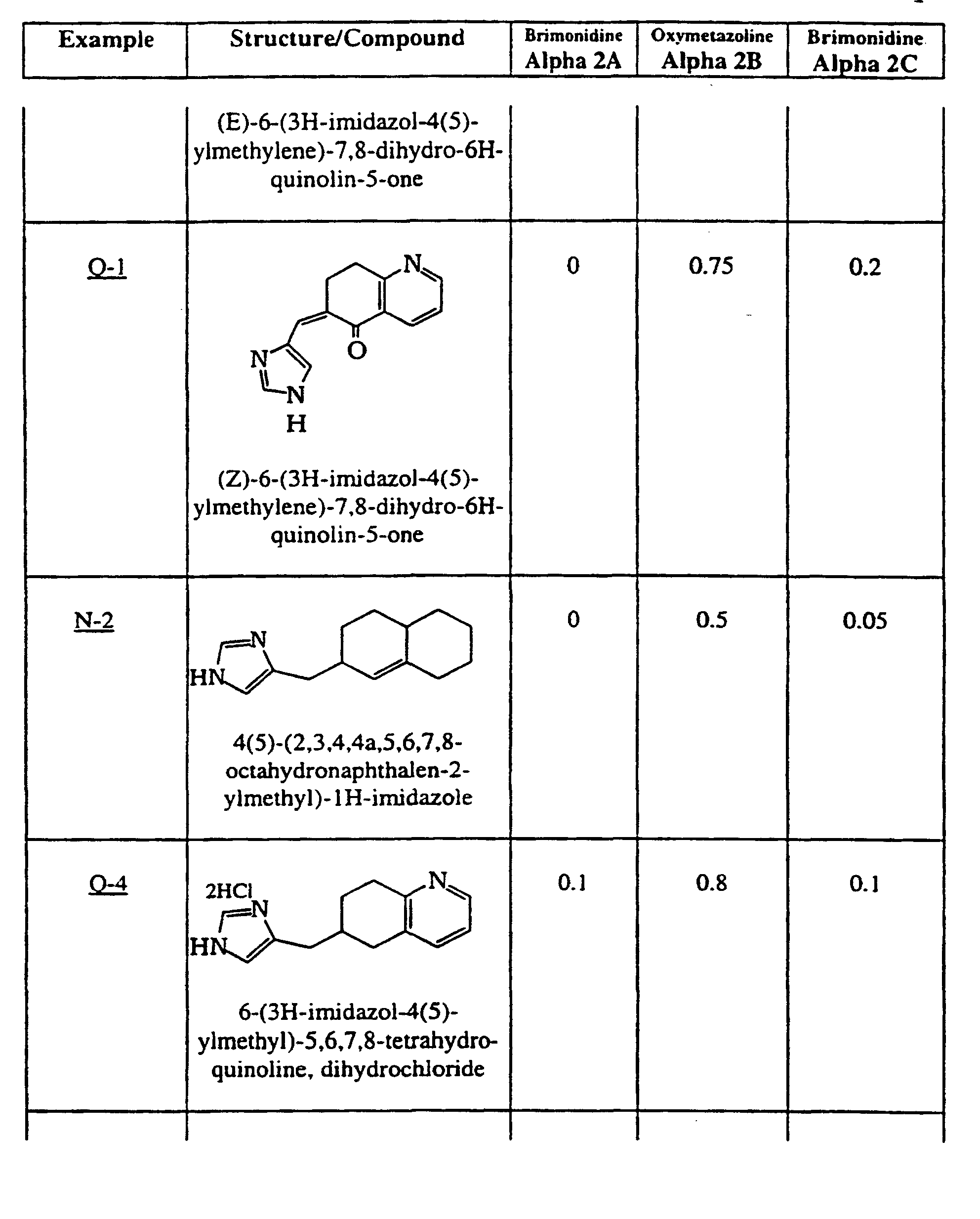

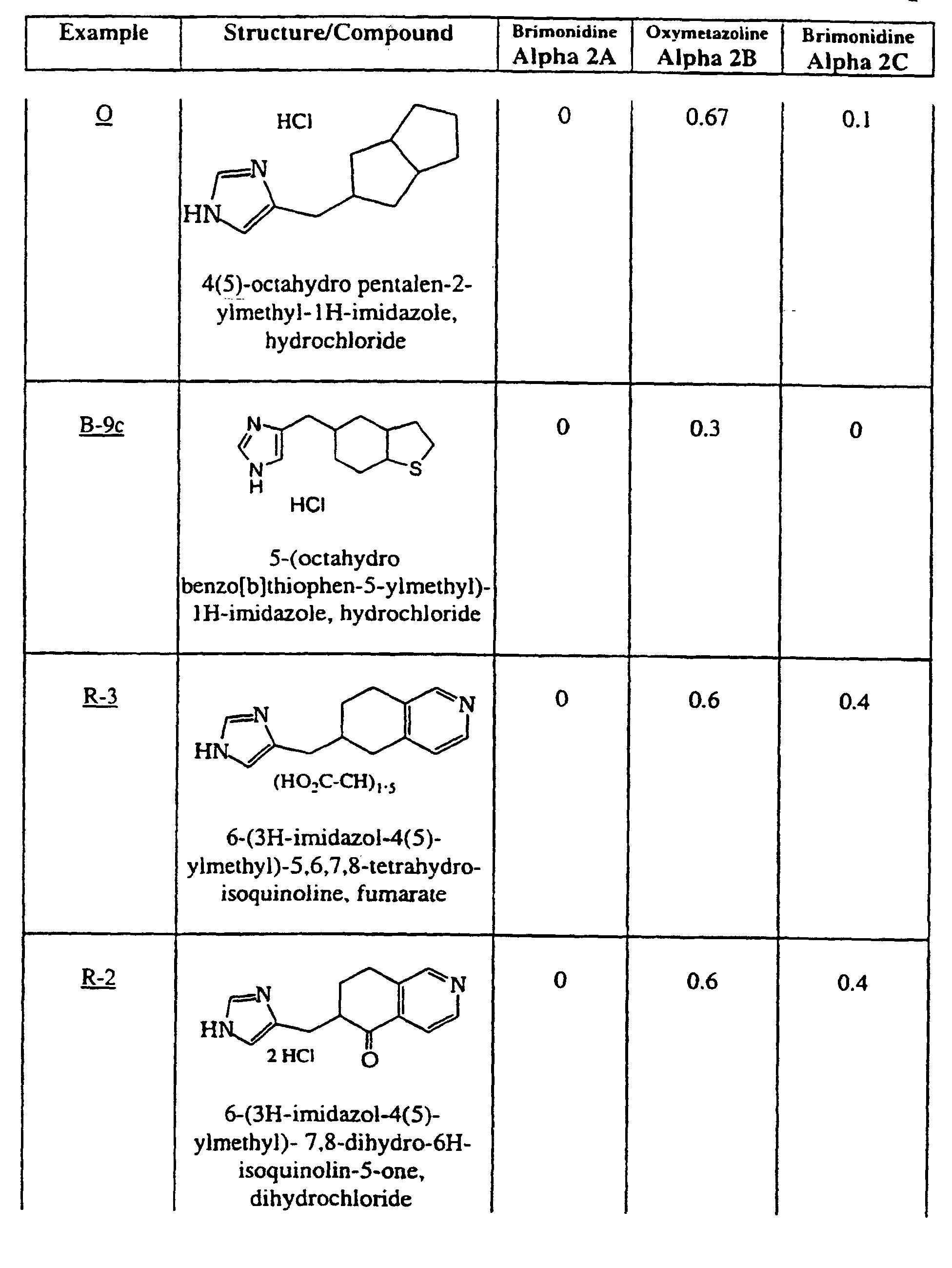

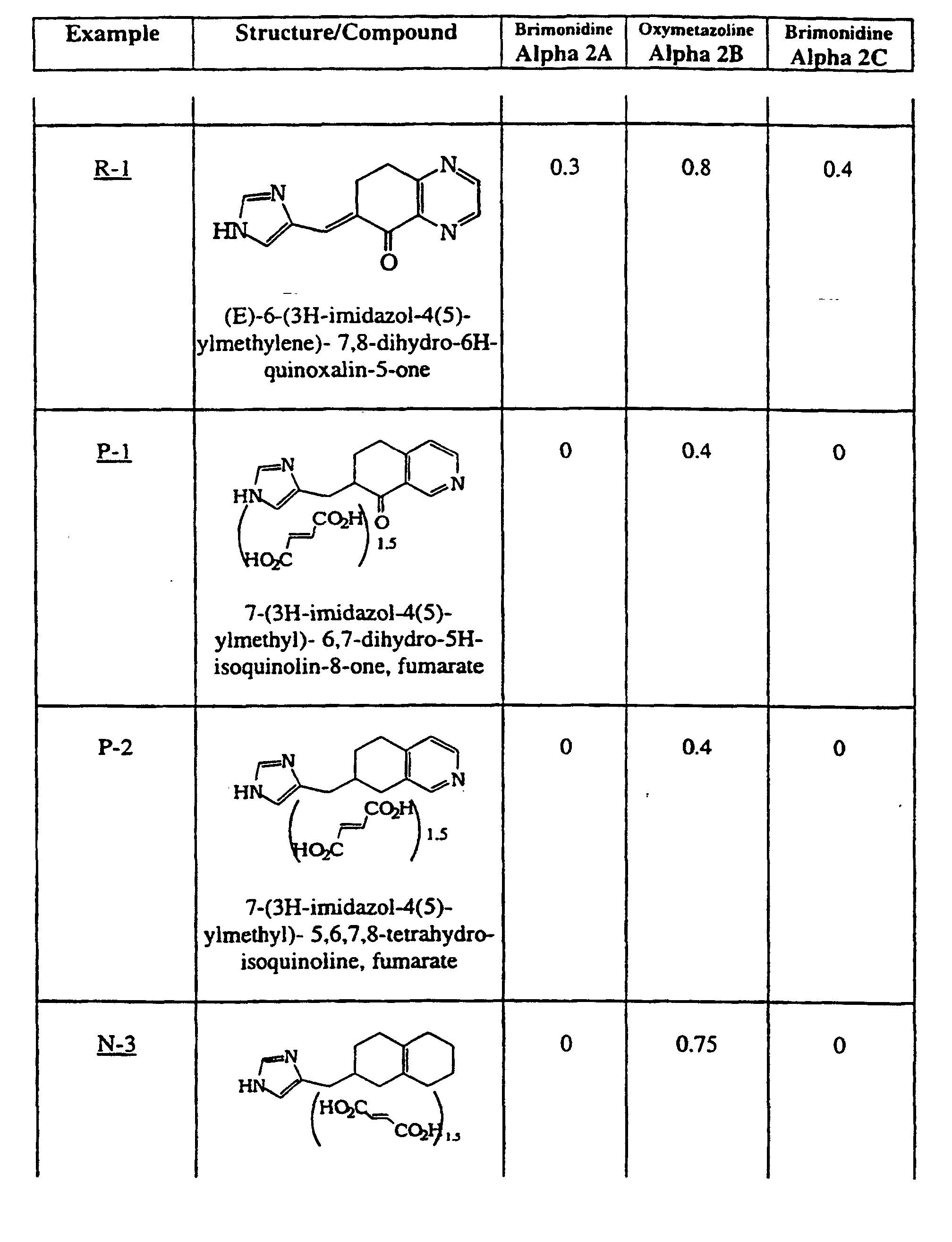

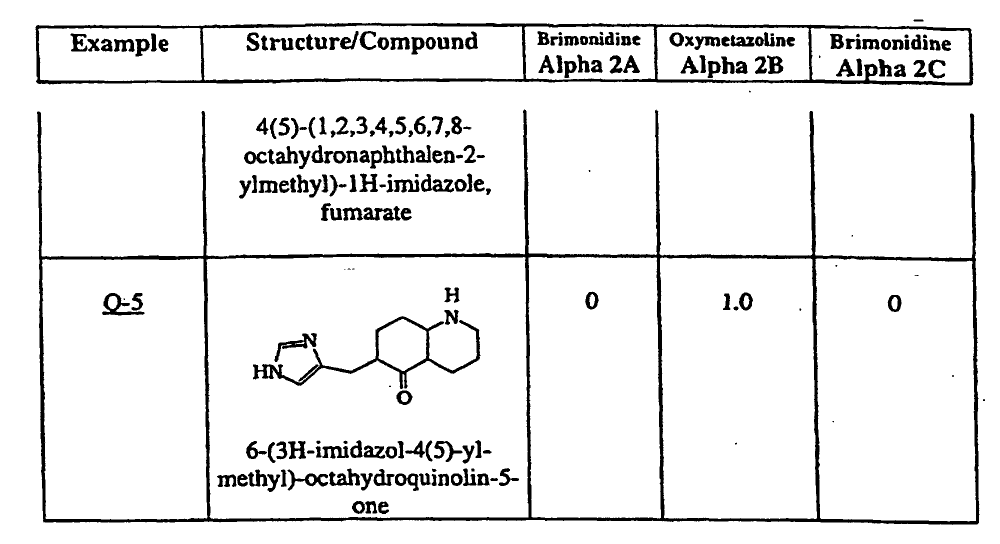

### Example T

### IOP-Lowering and Sedative Side Effects

Measurements of IOP (intra ocular pressure) were made in fully conscious female cynomolgus monkeys weighing 3-4 kg with sustained elevated IOP that was produced in the right eye by argon laser photocoagulation of the trabecular meshwork. Animals were usable for experiments ∼ 2 months following surgery. During the experiments, monkeys sat in specially designed chairs (Primate Products, San Francisco), and were fed orange juice and fruit as needed. A 30R model Digilab pneumatonometer (Alcon, Texas) was used to measure IOP.

Twenty five µl of an anesthetic (proparacaine) was topically applied to each monkey before IOP measurements to minimize ocular discomfort due to tonometry. Two baseline measurements were made prior to instillation of the drugs, followed by periodic measurements up to 6 hours post-instillation. The test compounds were administered unilaterally as a single 50 µl eye drop; the contralateral eyes received an equal volume of saline.

Many of the α2B or α2B/2C selective compounds of the examples were tested in the monkeys. Surprisingly, as Table 2 shows, these structurally diverse compounds all lowered IOP in the treated eye.

At the same time, sedation was measured and assessed according to the following score: 0 = alert, typical vocalization, movement, etc.; 1 = calm, less movement; 2= slightly sedated, some vocalization, responsive to stimulation; 3 = sedated, no vocalization, some response to stimulation; 4 = asleep.

The compounds used in the present invention also did not cause sedation. This contrasts with the action of clonidine and brimonidine, which caused sedation.

**Table 2.** The effects of α₂-adrenoceptor agonists on IOP and sedation in conscious cynomolgus monkeys following ocular administration in eyes made unilaterally hypertensive by argon laser photocoagulation. Measurements were made periodically up to 6 hours. Sedation was assessed subjectively during the IOP experiments using the following scoring: 0 = alert, typical vocalization, movement, etc.; 1 = calm, less movement; 2 = slightly sedated, some vocalization, responsive to stimulation; 3 = sedated, no vocalization, some response to stimulation; 4 = asleep. Number of animals per group = (6-9).

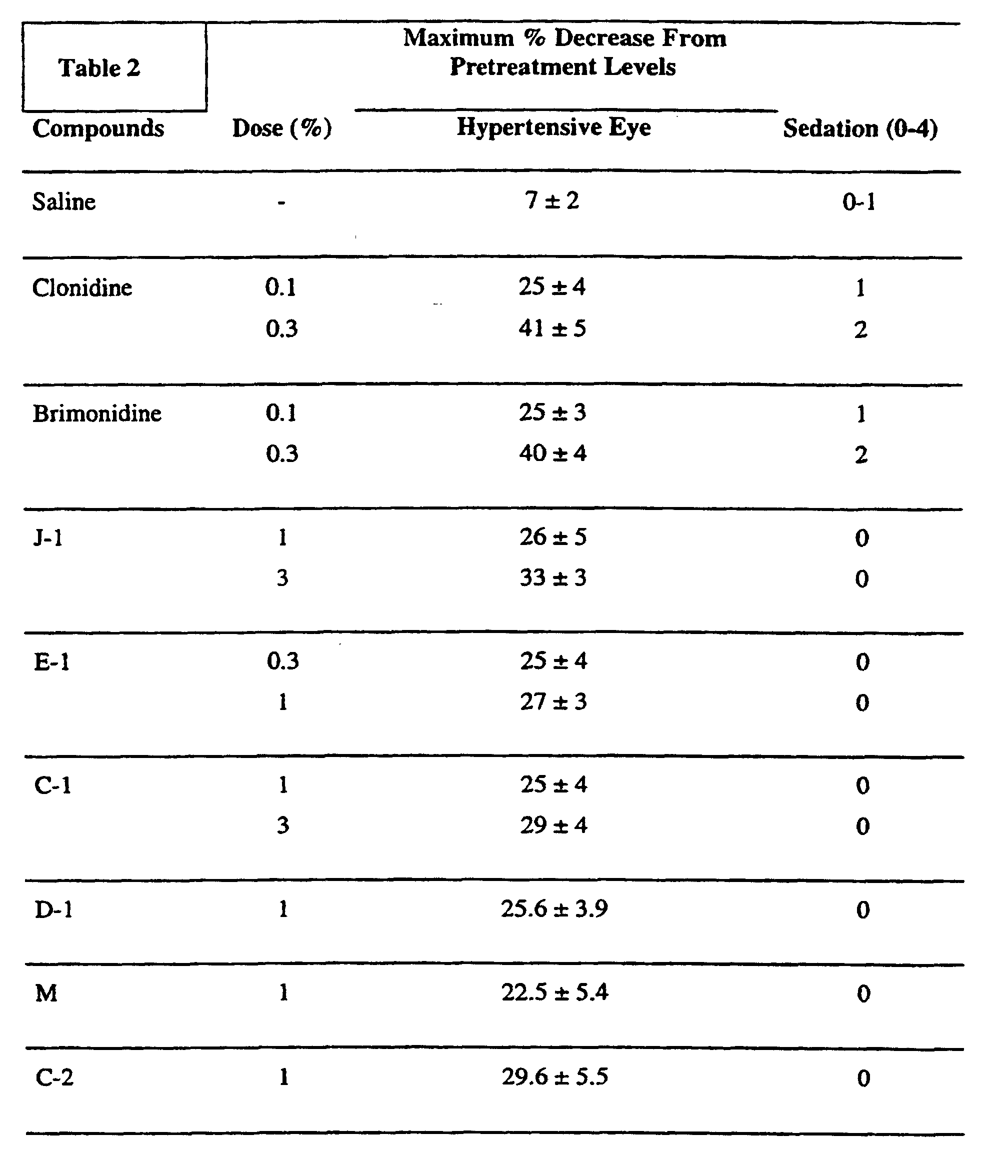

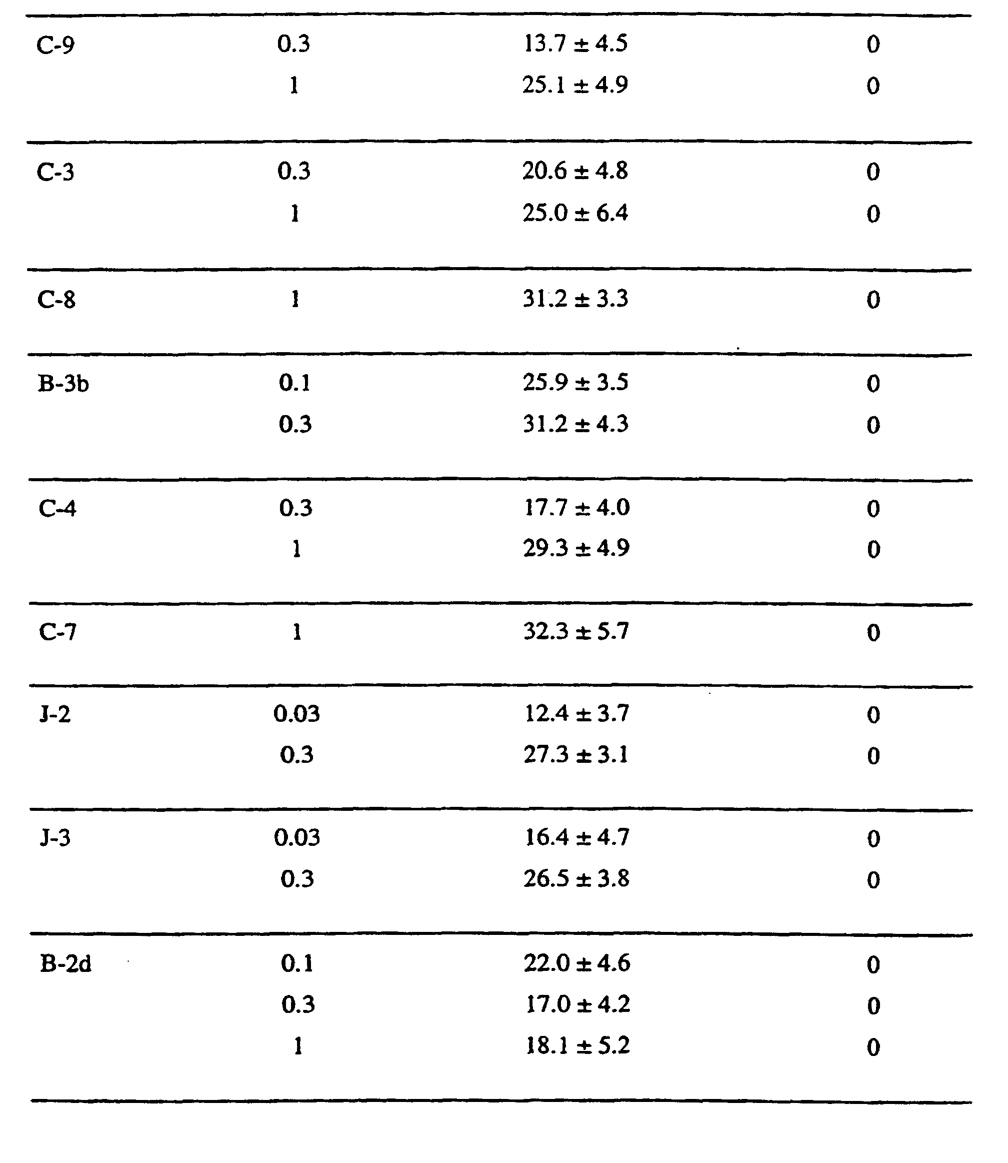

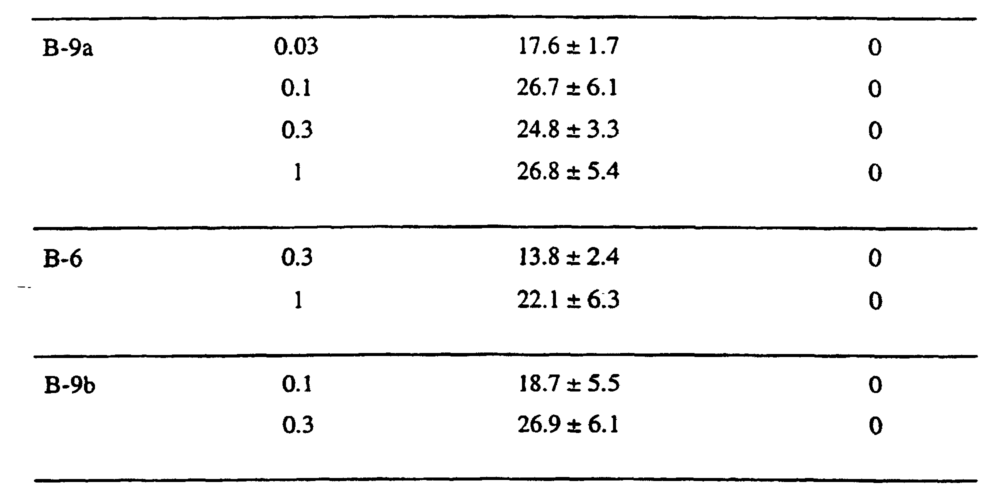

### Example U

### Measurement of Cardiovascular Side Effects

Cardiovascular measurements were made in a different group of monkeys using a BP 100S automated sphygmomanometer (Nippon Colin,. Japan). Intravenous (IV) administration of certain of the compounds of the present invention at doses ten to thirty times higher than the doses for clonidine and brimonidine did not reduce heart rate or lower blood pressure. Interestingly, the compound 4(5)-3-methylthiophen-2-ylmethyl)-1H-imidazole, which has intrinsic activity of 0.43 at the α2A-subtype, exhibited a weak effect on heart rate. Clonidine and brimonidine had even greater effects on heart rate. See Table 3 below.

**Table 3**. The effects of α₂-adrenoceptor agonists on cardiovascular variables in conscious cynomolgus monkeys following i.v. administration. Measurements were made periodically up to 6 hours. Number of animals per group = (6-10).

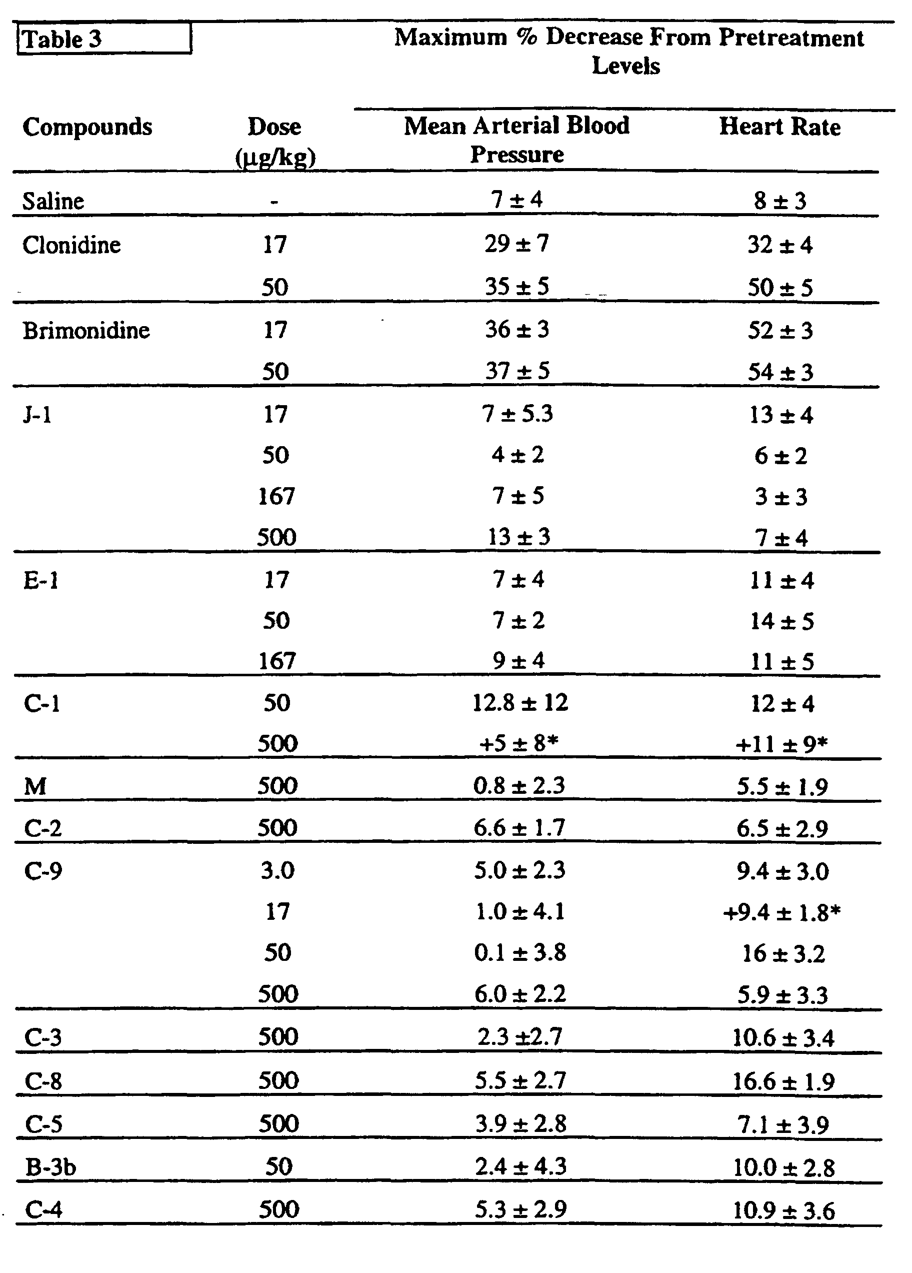

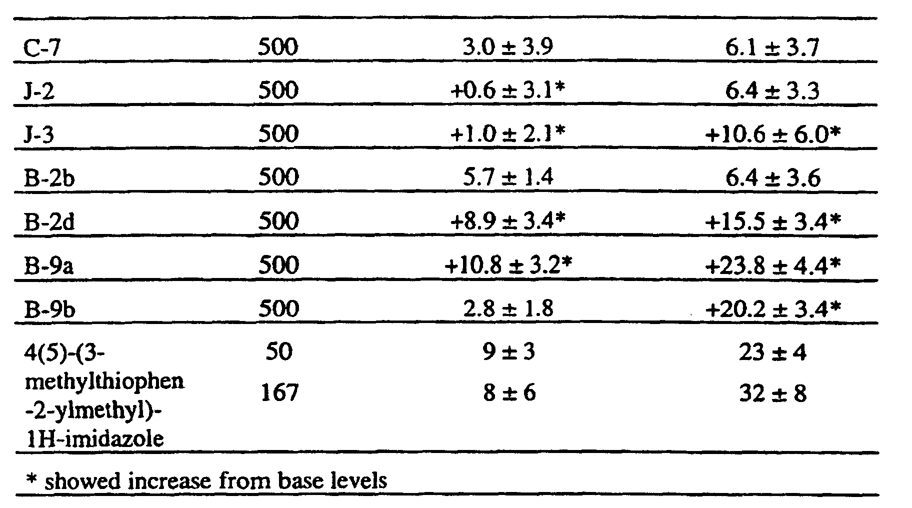

### EXAMPLE V

The studies in the above Examples T and U demonstrate that a therapeutic effect of alpha2 agonists can be separated from sedative and cardiovascular side effects. This separation is accomplished with compounds that share the property of being preferentially active at the alpha2B and alpha2B/alpha2C subtypes relative to the alpha2A subtype.

The prior art alpha2 adrenergic agonists, which activate all three alpha2 receptors, cause sedation, hypotension and bradycardia, preventing or severely limiting their use for treating diseases and disorders that are known to be ameliorated by them. Such diseases and disorders include muscle spasticity including hyperactive micturition, diarrhea, diuresis, withdrawal syndromes, pain including neuropathic pain, neurodegenerative diseases including optic neuropathy, spinal ischemia and stroke, memory and cognition deficits, attention deficit disorder, psychoses including manic disorders, anxiety, depression, hypertension, congestive heart failure, cardiac ischemia and nasal congestion. See, for example, Hieble et al., "Therapeutic applications of agents interacting with alpha-adrenoceptors, in Alphaadrenoceptors: molecular biology, biochemistry and pharmacology". *Prog*. *Basic Clin*. *Pharmacol*. (Basel, Karger) 8, pp. 180-220(1991). For example, clonidine has been shown to be clinically effective in providing pain relief for postoperative, cancer-associated and neurogenic pain. But, as stated in Maze and Tranquilli, Maze MB and Tranquilli, W. "Alpha-2 adrenoceptor agonists: defining the role in clinical anesthesia". *Anesthesiology* **74,** 581-605 (1991), the "full clinical promise" of this and other alpha2 agonists requires the development of compounds that do not cause sedation, hypotension and bradycardia.

The above-listed diseases and disorders are treatable by activation of α2B or α2B/2C receptor subtype(s). Therefore, the alpha2 compounds described above that have been shown above not to elicit sedation and cardiovascular effects, are useful and advantageous in the treatment of these conditions.

Amelioration of neuronal degeneration in glaucomatous neuropathy is another example of the novel utility of the compounds of the invention. Recent studies have demonstrated that clonidine and other alpha2 agonists are neuroprotective of retinal cells in several rat models of neuronal degeneration. These models include light-induced photoreceptor degeneration in albino rat, as described in Wen et al, "Alpha2-adrenergic agonists induce basic fibroblast growth factor expression in photoreceptors in vivo and ameliorate light damage." *J*. *Neurosci.* **16**, 5986-5992 and calibrated rat optic nerve injury resulting in secondary loss of retinal ganglion cells, as described in Yoles et al, "Injury-induced secondary degeneration of rat optic nerve can be attenuated by alpha2-adrenoceptor agonists AGN 191103 and brimonidine". *Invest*. *Ophthalmol*. *Vis*. *Sci*. **37**, 540,S114. However, unlike the compounds of the present invention, the doses used in these studies - 0.1 to >1 mg/kg by intraperitoneal or intramuscular injection- also cause sedation and cardiovascular effects. Induction of the expression of basic fibroblast growth factor (bFGF) is considered a sensitive indicator of alpha2 receptor activation in the retina (Wen et al above) and measurement of bFGF induction following topical administration of alpha2 agonists to rat eyes indicates that approximately a 1% dose is necessary to induce a 2-3 fold increase in bFGF levels that correspond with alpha2 agonist mediated neuroprotection (See Wen et al, above, and Lai et al, "Neuroprotective effect of ocular hypotensive agent brimonidine", in *Proceedings of Xlth Congress of the European Society of Ophthalmology* (Bologna, Monduzzi Editore), 439-444.) These topical doses of current alpha2 agonists such as clonidine are known to result in systemic side effects such as sedation and hypotension that would prevent their use as ocular neuroprotective agents. Additionally commonly assigned and co-pending application, 08/496,292 filed on 28 June, 1995, discloses and claims the use of certain non-selective α2-adrenergic agents in treating neural injury, the contents of which are hereby incorporated by reference in their entirety.

The compounds used in the present invention do not cause sedation and cardiovascular effects following topical administration of doses of at least 3% in monkeys. Thus, neuroprotective concentrations of these compounds can be reached in humans without causing side effects. In fact, as reported below, the compound of Example B-9(b) has been shown to be neuroprotective in the calibrated rat optic nerve injury model of Yoles et al, above. See Table 4, below.

**Table 4:**

| Retinal Ganglion Cell Numbers at 2 Weeks Post-Injury (cells/microscopic field) | |
|---|---|
| Control | Example B-9(b) |
| (vehicle i.p.) | (0.5 mg/kg i.p.) |
| 33±8 | 73±12 |
| n=8 | n=5 |

This level of neuroprotection is comparable to the effect seen in previous studies with the standard alpha 2-adrenoceptor agonist, brimonidine, and the neuroprotective agent, MK801.

### Example W

Alleviation of pain including neuropathic pain is another example of a disorder in which the compounds used in the invention are useful and advantageous since pain is alleviated without undesirable side effects. Clonidine, an agonist that activates all three alpha2 receptors, has been used clinically for treating chronic pain, but its utility for this indication is limited because it causes sedation and cardiovascular side effects. Compounds of the present invention were compared to clonidine and brimonidine in a rodent model of neuropathic pain that is known to be predictive of clinical activity. (See, for example, Kim, S. and Chung, J. "An experimental model for peripheral neuropathy produced by segmental spinal nerve ligation in the rat." *Pain* 50 pp. 355-363 (1992).) Following ligation of two spinal nerves, the animals develop a sensitivity to normally non-painful stimuli such as touch. The ability of alpha2 compounds to reverse this sensitivity, called allodynia, was tested 30 minutes after dosing by either intrathecal or intraperitoneal administration. The sedative activity of each compound was also measured using an activity chamber.

The compounds used in the invention, exemplified by N-1, are able to alleviate the allodynia without causing sedation, even at very high doses. This is in contrast to clonidine and brimonidine, which cause sedation at doses only slightly higher than their anti-allodynic doses. See tables 5 and 6, below.

**Table 5.**

| The anti-allodynic and sedative effects of alpha2-adrenoceptor agonists in rats 30 minutes following intrathecal administration (N=6). | | | |
|---|---|---|---|
| Compound | Dose (µg) | Reversal of Tactile Allodynia (%) | Sedation (%) |
| Clonidine | 0.1 | 20* | ND |
| | 1 | 96* | 15 |
| | 10 | ND | 60* |
| N-1 | 3 | 13 | ND |
| | 30 | 64* | 0 |
| | 300 | ND | 0 |

| | | | |
|---|---|---|---|
| * p<0.05 compared to saline control • ND signifies no data | | | |

**Table 6.**

| The anti-allodynic and sedative effects of alpha2-adrenoceptor agonists in rats 30 minutes following intraperitoneal administration (N=6). | | | |
|---|---|---|---|
| Compound | Dose (mg/kg) | Reversal of Tactile Allodynia (%) | Sedation (%) |
| Brimondine | 3 | 0 | ND |
| | 30 | 37* | 24 |
| | 300 | ND | 67* |
| N-1 | 3 | 3 | ND |
| | 30 | 41* | ND |
| | 10,000 | ND | 0 |

| | | | |
|---|---|---|---|
| * p<0.05 compared to saline control • ND signifies no data | | | |

The results of these Examples demonstrate that the common side effects of α2-adrenoceptor drugs are mediated by the α2A-subtype and that their ocular antihypertensive and other therapeutic actions can be mediated by a subtype other than the α2A-subtype. Thus, α2-adrenoceptor compounds of unrelated structural classes, that have in common low functional activity at the α2A-subtype, lower IOP and elicit other therapeutic actions without dose-limiting side effects.

## Claims

1. Use of a compound with adrenergic activity and which is a selective agonist of the α2B adrenoreceptor subtype or α2B/α2C adrenoreceptor subtype (s)
for the manufacture of a pharmaceutical composition for the treatment or prevention of glaucoma without sedating or cardiovascular side effects in a host mammal, including a human being,
wherein the active compound has a) an efficacy relative to a standard full agonist that is at least approximately 0.3 greater at the α2B or α2C adrenoreceptor subtypes than at the α2A adrenoreceptor subtype and its efficacy at the α2A adrenoreceptor subtype is ≤0.4 or b)
is at least ten times more potent at the α2B or α2C adrenoreceptor subtype than at the α2A adrenoreceptor.

2. The use of claim 1 wherein approximately 0.001% to 5% by weight of the active compound is administered topically to the host mammal in daily or twice daily doses.

3. The use of claim 2 wherein approximately 0.01% to 3% by weight of the active compound is administered topically to the host mammal in daily or twice daily doses.

4. The use of claim 1 wherein said compound has no activity at the α2A adrenoreceptor subtype.

5. The use of claim 1 wherein said compound has no activity at the α2A and α2C adrenoreceptor subtypes.

6. Use of a compound with adrenergic activity and which is a selective agonist of the α2B or α2B/α2C adrenoreceptor subtype(s)
as active ingredient for the manufacture of a pharmaceutical composition for the treatment of elevated intraocular pressure without sedating or cardiovascular side effects in a host mammal, including a human being,
wherein the active compound has a) an efficacy relative to a standard full agonist that is at least approximately 0.3 greater at the α2B or 2C adrenoreceptor subtypes than at the α2A adrenoreceptor subtype and its efficacy at the α2A adrenoreceptor subtype is ≤0.4 or b) is at least ten times more potent at the α2B or α2C adrenoreceptor subtype than at the α2A adrenoreceptor.

7. The use of claim 6 wherein approximately 0.001% to 5% by weight of the active compound is administered topically to the host mammal in daily or twice daily doses.

8. The use of claim 7 wherein approximately 0.01% to 3% by weight of the active compound is administered topically to the host mammal in daily or twice daily doses.

9. The use of claim 6 wherein said compound has no activity at the α2A adrenoreceptor subtypes.

10. The use of claim 6 wherein said compound has no activity at the α2A and α2C adrenoreceptor subtypes.

11. Use of a compound which selectively agonizes the α2B adrenoreceptor subtype or a α2B/α2C adrenoreceptor subtype(s)
as an active ingredient for the manufacture of a pharmaceutical composition for treating a mammal to lower intraocular pressure without having cardiovascular and sedative side effects, wherein the active compound a) has an efficacy relative to a standard full agonist that is at least approximately 0.3 greater at the α2B or α2C adrenoreceptor subtypes that at the α2A adrenoreceptor subtype and its efficacy at the α2A adrenoreceptor subtype is ≤0.4 or b) is at least ten times more potent at the α2B or α2C adrenoreceptor subtype than at the α2A adrenoreceptor.

12. The use according to claims 1, 6 or 11 , wherein the active compound is selected from the group consisting of compounds having the formula 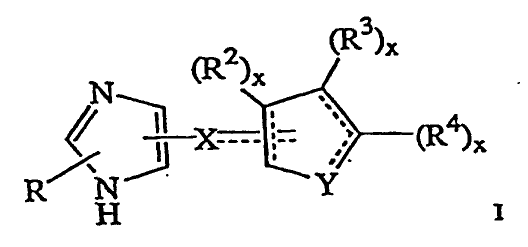 wherein the dotted lines represent optional double bonds; R is H or C₁₋₄ alkyl; X is S or C(H)R¹, wherein R¹ is H or C₁₋₄ alkyl or R¹ is absent when X is S or when the bond between X and the ring represented by 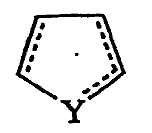 is a double bond, Y is O, N, S, (CR¹ₓ)_{y}, wherein y is an integer of from 1 to 3, -CH=CH- or -Y¹CH₂-, wherein Y¹ is O, N or S; x is an integer of 1 or 2, wherein x is 1 when R², R³ or R⁴ is bound to an unsaturated carbon atom and x is 2 when R², R³ or R⁴ is bonded to a saturated carbon atom, R² is H, C₁₋₄ alkyl, halogen, hydroxy or C₁₋₄ alkoxy, or, when attached to a saturated carbon atom, R² may be oxo; R³ and R⁴ are, each H, C₁₋₄ alkyl, hydroxy, C₁₋₄ alkoxy, or phenyl or, together, are -(C(R²)x)z-; -Y¹(C(R²)x)z'-; -Y¹(C(R²)x)yY¹-; -(C(R²)x)-Y¹-(C(R²)x)-; -(C(R²)x)-Y¹-(C(R²)x)-(C(R²)x)- and -Y¹-(C(R²)x)-Y¹-(C(R²)x)- wherein z is an integer of from 3 to 5, z' is an integer of from 2 to 4 and x and y are as defined above, and further either end of each of these divalent moieties may attach at either R³ or R⁴ to form the condensed ring structure 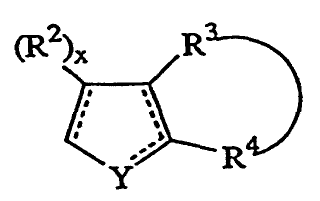 and the ring thus formed may be totally unsaturated, partially unsaturated, or totally saturated provided that a ring carbon has no more than 4 valences, nitrogen no more than three and O and S have no more than two; or 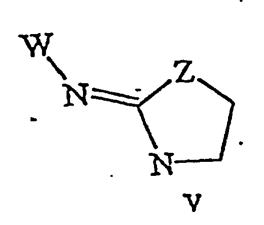 wherein W is a bicyclic radical selected from the group consisting of 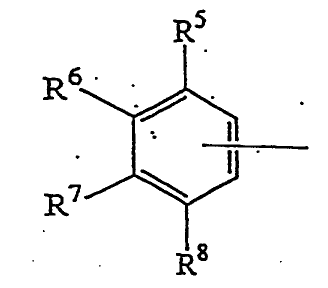 wherein R⁵, R⁶, R⁷ and R⁸ are selected from the group consisting of H and C₁₋₄ alkyl provided that at least one of R⁵ and R⁶ or R⁶ and R⁷ are OC(R⁹)C(R⁹)N(R) to form a condensed ring with 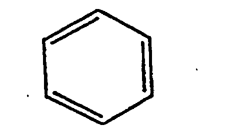 wherein R⁹ is H, C₁₋₄ alkyl or oxo,
and 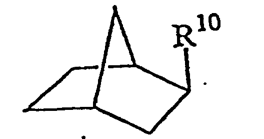 wherein R¹⁰ is H, C₁₋₄ alkyl, phenyl or C₁₋₄ alkyl substituted phenyl, and Z is O or NH.

13. Use of a compound as an active ingredient for the manufacture of a pharmaceutical composition for the treatment of prevention of glaucoma in a host mammal, including a human being, wherein.the compound, having adrenergic activity, has the biological property that the compound is a selective agonist of α2B or α2B/α2C receptor subtype(s)
said selectivity being measured in an assay using cells that naturally express the individual α2 subtypes or have had one of the subtypes introduced, the receptors being human or from a species that has been shown to have a similar pharmacology, and in which assay the efficacy relative to a standard compound of the active compound at the α2B or α2C receptor subtype is measured to be at least 0.3 greater than the efficacy relative to the standard compound of the active compound at the α2A receptor subtype and its efficacy at the α2A receptor subtype is ≤0.4, and/or the active compound is at least approximately 10 times more potent at the α2B or α2C receptor subtypes than at the α2A receptor subtype.

14. The use of claim 13 wherein approximately 0.001% to 5% by weight of tne active compound is administered topically to the host mammal per day.

15. Use of a compound as an active ingredient for the manufacture of a pharmaceutical composition for the treatment or prevention of muscle spasticity including hyperactive micturition, diarrhea, diuresis, withdrawal syndromes, pain including neuropathic pain, neurodegenerative diseases including optic neuropathy, spinal ischemia and stroke, memory and cognition deficits, attention deficit disorder, psychoses including manic disorders, anxiety, depression, hypertension, congestive heart failure, cardiac ischemia and nasal congestion without sedating or cardiovascular side effects, in a host mammal including a human being, wherein said compound has adrenergic activity and is a selective agonist of the α2B or α2B/α2C adrenoreceptor subtype (s), wherein said compound a) has an efficacy relative to a standard full agonist that is at least approximately 0.3 greater at the α2B or α2C adrenoreceptor subtype them at the α2A adrenoreceptor subtype and its efficacy at the α2A adrenoreceptor subtype is ≤ 0.4 or b) is at least ten times more potent at the α2B or α2C adrenoreceptor subtype than at the α2A adrenoreceptor.

16. Use of a compound as an active ingredient for the manufacture of a pharmaceutical composition for the treatment of muscle spasticity including hyperactive micturition, diarrhea, diuresis, withdrawal syndromes, pain including neuropathy, spinal ischemia and stroke, memory and cognition deficits, attention deficit disorder, psychoses including manic disorders, anxiety, depression, hypertension, congestive heart failure, cardiac ischemia and nasal congestion without sedating or cardiovascular side effects, in a host mammal, including a human being, wherein the active compound having adrenergic activity has the biological property that the compound is a selective agonist of α2B or α2B/α2C receptor subtype(s)
; said selectivity being measured in an assay using cells that naturally express the individual α2 subtypes or have had one of the subtypes introduced, the receptors being human or from a species that has been shown to have a similar pharmacology, and in which assay the efficacy relative to a standard compound of the active compound at the α2B or the α2C receptor subtype is measured to be at least 0.3 greater than the efficacy relative to the standard compound of the active compound at the α2A receptor subtype and its efficacy at the α2A receptor is ≤0.4, and/or the active compound is at least approximately 10 times more potent at the α2B or α2C receptor subtypes than at the α2A receptor subtype.

17. Use of a compound as an active ingredient for the manufacture of a pharmaceutical composition for the treatment of muscle spasticity including hyperactive micturition, diarrhea, diuresis, withdrawal syndromes, pain including neuropathy, spinal ischemia and stroke, memory and cognition deficits, attention deficit disorder, psychoses including manic disorders, anxiety, depression, hypertension, congestive heart failure, cardiac ischemia and nasal congestion without sedating or cardiovascular side effects, in a host mammal, including a human being, wherein the active compound having adrenergic activity has the biological property that the compound is a selective agonist of α2B or α2B/α2C receptor subtype(s)
said selectivity being measured in an RSAT assay in which activation of the α2A and α2C receptor subtype by the test compound is compared to brimonidine and the α2B receptor subtype is compared to oxymetazoline and wherein the respective α2A, α2B and α2C receptor subtypes are expressed in NIH-3T3 cells, and in which assay the efficacy relative to brimonidine of the active compound at the α2C receptor subtype or the efficacy relative to oxymetazoline of the active compound at the α2B receptor subtype is measured to be at least 0.3 greater than the efficacy relative to brimonidine of the active compound at the α2A receptor subtype, and its efficacy at the α2A receptor subtype is ≤0.4 and/or the active compound is at least approximately 10 times more potent at the α2B or α2C receptor subtypes than at the α2A receptor subtype.

18. The use of claim 1, 6, 11, 15, 16 or 17 wherein the active compound is at least one hundred times more potent at the α2B or α2C adrenoreceptor subtype than at the α2A adrenoreceptor subtype.

19. The use of claim 15 wherein approximately 0.001% to 5% by weight of the active compound is administered topically to the host mammal in daily or twice daily doses.

20. The use of claim 20 wherein approximately 0.01% to 3% by weight of the active compound is administered topically to the host mammal in daily or twice daily doses.

21. The use of claim 15 wherein said compound has no activity at the α2A adrenoreceptor subtype.

22. The use of claim 15 wherein said compound has no activity at the α2A and α2C adrenoreceptor subtypes.

## Patentansprüche

1. Verwendung einer Verbindung mit adrenergischer Wirkung, die ein selektiver Agonist des α2B-Adrenorezeptor-Sybtyps oder der α2B/α2C-Adrenorezeptor-Subtypen ist, zur Herstellung einer pharmazeutischen Zusammensetzung zur Behandlung oder Prävention von Glaukomen ohne sedierende oder kardiovaskuläre Nebenwirkungen in einem Säugetier-Wirt, einschliesslich des Menschen, wobei die aktive Verbindung
(a) eine Wirksamkeit in bezug auf einen Standard-Totalagonisten hat, die um mindestens etwa 0,3 grösser an den α2B- oder α2C-Adrenorezeptor-Subtypen als am α2A-Adrenorezeptor-Subtyp ist und seine Wirksamkeit am α2A-Adrenorezeptor-Subtyp ≤ 0,4 ist, oder
(b) mindestens zehnmal wirksamer am α2B- oder α2C-Adrenorezeptor-Subtyp als am α2A-Adrenorezeptor ist.

2. Verwendung gemäss Anspruch 1, wobei etwa 0,001 bis 5 Gew.% der wirksamen Verbindung topisch an einen Säugetier-Wirt in täglichen oder zweimal täglichen Dosen verabreicht werden.

3. Verwendung gemäss Anspruch 2, wobei mindestens 0,01 bis 3 Gew.% der wirksamen Verbindung topisch an einen Säugetier-Wirt in täglichen oder zweimal täglichen Dosen verabreicht werden.

4. Verwendung gemäss Anspruch 1, wobei diese Verbindung keine Wirkung auf den α2A-Adrenorezeptor-Subtyp hat.

5. Verwendung gemäss Anspruch 1, wobei diese Verbindung keine Wirkung auf die α2A- oder α2C-Adrenorezeptor-Subtypen hat.

6. Verwendung einer Verbindung mit adrenergischer Wirkung, welche ein selektiver Agonist der α2B- oder α2B/α2C-Adrenorezeptor-Subtypen ist, als Wirkstoff zur Herstellung einer pharmazeutischen Zusammensetzung zur Behandlung eines erhöhten intraokularen Drucks ohne zu sedieren oder kardiovaskuläre Nebenwirkungen in einem Säugetier-Wirt, einschliesslich des Menschen, wobei die aktive Verbindung aufweist:
(a) eine Wirksamkeit in bezug auf einen Standard-Totalagonisten, die mindestens etwa 0,3 grösser auf die α2B- oder α2C-Adrenorezeptor-Subtypen als auf den α2A-Adrenorezeptor-Subtyp ist und dessen Wirksamkeit auf den α2A-Adrenorezeptor-Subtyp ≤ 0,4 ist, oder
(b) mindestens zehnmal stärker für den α2Boder α2C-Adrenorezeptor-Subtyp ist als am α2A-Adrenorezeptor.

7. Verwendung gemäss Anspruch 6, wobei etwa 0,001 bis 5 Gew.% des Wirkstoffs topisch in täglichen oder zweimal täglichen Dosen an den Säugetier-Wirt verabreicht werden.

8. Verwendung gemäss Anspruch 7, wobei etwa 0,01 bis 3 Gew.% des Wirkstoffs topisch in täglichen oder zweimal täglichen Dosen an den Säugetier-Wirt verabreicht werden.

9. Verwendung gemäss Anspruch 6, wobei diese Verbindung keine Wirkung auf die α2A-Adrenorezeptor-Subtypen hat.

10. Verwendung gemäss Anspruch 6, wobei diese Verbindung keine Wirkung auf die α2A- und α2C-Adrenorezeptor-Subtypen hat.

11. Verwendung einer Verbindung, die den α2B-Adrenorezeptor-Subtyp oder einen α2B/α2C-Adrenorezeptor-Subtyp(en) selektiv agonisiert, als Wirkstoff zur Herstellung einer pharmazeutischen Zusammensetzung zur Behandlung eines Säugetiers, um den intraokularen Druck zu senken, ohne kardiovaskuläre oder sedierende Nebenwirkungen zu haben, wobei der Wirkstoff
(a) eine Wirksamkeit, bezogen auf einen Standard-Totalagonisten, hat, die um mindestens etwa 0,3 grösser auf die α2B- oder α2C-Adrenorezeptor-Subtypen als auf den α2A-Adrenorezeptor-Subtyp ist, und dessen Wirksamkeit auf den α2A-Adrenorezeptor-Subtyp ≤ 0,4 ist, oder
(b) mindestens zehnmal stärker auf den α2Boder α2C-Adrenorezeptor-Subtyp ist als auf den α2A-Adrenorezeptor.

12. Verwendung gemäss Anspruch 1, 6 oder 11, wobei der Wirkstoff ausgewählt ist aus der Gruppe bestehend aus Verbindungen der Formel: 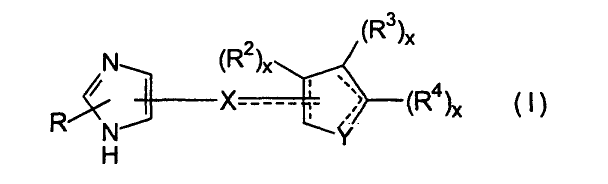 wobei die gepunkteten Linien gegebenenfalls Doppelbindungen darstellen; R H oder C₁₋₄-Alkyl ist; X S oder C(H)R¹ ist, wobei R¹ H oder C₁₋₄-Alkyl ist oder R¹ nicht vorhanden ist, wenn X S ist oder wenn die Bindung zwischen X und dem Ring, der durch 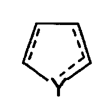 dargestellt wird, eine Doppelbindung ist, Y O, N, S, (CR¹ₓ)_{y} ist, wobei y eine ganze Zahl von 1 bis 3 ist, -CH=CH- oder -Y¹CH₂-, wobei Y¹ O, N oder S ist; x ist eine ganze Zahl von 1 oder 2, wobei x 1 ist, wenn R², R³ oder R⁴ an ein nicht-gesättigtes Kohlenstoffatom gebunden sind, und x 2 ist, wenn R², R³ oder R⁴ an ein gesättigtes Kohlenstoffatom gebunden ist, R² ist H, C₁₋₄-Alkyl, Halogen, Hydroxy oder C₁₋₄-Alkoxy, oder wenn es an ein gesättigtes Kohlenstoffatom gebunden ist, kann R² auch Oxo sein; R³ und R⁴ sind jeweils H, C₁₋₄-Alkyl, Hydroxy, C₁₋₄-Alkoxy oder Phenyl, oder zusammen sind sie -(C(R²)ₓ)_{z}-;
-Y¹(C(R²)ₓ)_{z'}-; -Y¹(C(R²)ₓ)_{y}Y¹-;
- (C(R²)ₓ)-Y¹-(C(R²)ₓ)-;
- (C(R²)ₓ)-Y¹-(C(R²)ₓ)-(C(R²)ₓ)- und
-Y¹-(C(R²)ₓ)-Y¹-(C(R²)ₓ)-, wobei z eine ganze Zahl von 3 bis 5 ist, z' eine ganze Zahl von 2 bis 4 ist und x und y wie oben definiert sind, und ausserdem jedes Ende dieser divalenten Reste sich entweder an R³ oder R⁴ binden kann, um die kondensierte Ringstruktur zu bilden: 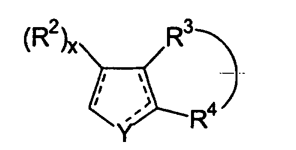 und der so gebildete Ring vollständig ungesättigt, teilweise ungesättigt oder vollkommen gesättigt sein kann, vorausgesetzt, dass ein Ringkohlenstoff nicht mehr als 4 Valenzen hat, Stickstoff nicht mehr als 3 und O und S nicht mehr als 2 haben; oder 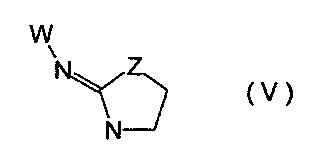 wobei W ein bicyclischer Rest ist, der ausgewählt ist aus der Gruppe bestehend aus: 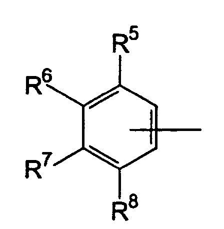 wobei R⁵, R⁶, R⁷ und R⁸ ausgewählt sind aus der Gruppe bestehend aus H und C₁₋₄-Alkyl, vorausgesetzt, dass mindestens eines von R⁵ und R⁶ oder R⁶ und R⁷ OC(R⁹)C(R⁹)N(R) ist, um einen kondensierten Ring zu bilden, mit: 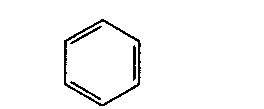 wobei R⁹ H, C₁₋₄-Alkyl oder Oxo ist, und 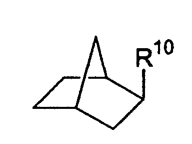 wobei R¹⁰ H, C₁₋₄-Alkyl, Phenyl oder C₁₋₄-Alkylsubstituiertes Phenyl ist, und Z oder NH ist.

13. Verwendung einer Verbindung als Wirkstoff zur Herstellung einer pharmazeutischen Zusammensetzung zur Behandlung oder Prävention von Glaukomen in einem Säugetier-Wirt, einschliesslich des Menschen, wobei die Verbindung mit adrenergischer Wirkung die biologische Eigenschaft hat, dass die Verbindung ein selektiver Agonist der α2B- oder α2B/α2C-Rezeptor-Subtypen ist, wobei die Selektivität in einem Test unter Verwendung von Zellen gemessen wird, die natürlicherweise die individuellen α2-Subtypen exprimieren oder einen der Subtypen eingeschleust haben, wobei die Rezeptoren menschlich sind oder von einer Spezies, von der gezeigt wurde, dass sie eine ähnliche Pharmakologie hat, und wobei in dem Test die relative Wirksamkeit gegenüber einer Standardverbindung der aktiven Verbindung auf den α2B- oder α2C-Rezeptor-Subtyp in einer Messung um mindestens 0,3 grösser als die relative Wirksamkeit der Standardverbindung auf den Wirkstoff am α2A-Rezeptor-Subtyp ist, und deren Wirksamkeit auf den α2A-Rezeptor-Subtyp ≤ 0,4 ist, und/oder der Wirkstoff ist mindestens etwa zehnmal stärker an den α2B- oder α2C-Rezeptor-Subtypen als am α2A-Rezeptor-Subtyp.

14. Verwendung gemäss Anspruch 13, wobei etwa 0,001 bis 5 Gew.% des Wirkstoffs täglich topisch an den Säugetier-Wirt verabreicht werden.

15. Verwendung einer Verbindung als Wirkstoff zur Herstellung einer pharmazeutischen Zusammensetzung zur Behandlung oder Verhinderung von Muskelspasmen, einschliesslich hyperaktiver Blasenleerung, Diarrhoe, Diurese, Entzugssyndromen, Schmerzen, einschliesslich neuropathischen Schmerzen, neurodegenerativen Erkrankungen, einschliesslich optischer Neuropathie, spinaler Ischämie und Schlaganfall, Erinnerungs- und kognitiven Defiziten, Aufmerksamkeitsdefizitstörungen, Psychosen, einschliesslich manischer Störungen, Ängstlichkeit, Depression, Hypertension, kongestivem Herzversagen, Herzischämie und Nasenverstopfung ohne sedierende oder kardiovaskuläre Nebenwirkungen, in einen Säugetier-Wirt, einschliesslich des Menschen, wobei die Verbindung adrenergische Wirkung hat und ein selektiver Agonist des/der α2B- oder α2B/α2C-Adrenorezeptor-Subtyp(s/en) ist, wobei die Verbindung
(a) eine Wirksamkeit, bezogen auf einen Standard-Totalagonisten, hat, die mindestens etwa 0,3 grösser auf den α2B- oder α2C-Adrenorezeptor-Subtyp als auf den α2A-Adrenorezeptor-Subtypen ist, und dessen Wirkung auf den α2A-Adrenorezeptor-Subtyp ≤ 0,4 ist, oder
(b) mindestens zehnmal stärker auf den α2Boder α2C-Adrenorezeptor-Subtyp ist als auf den α2A-Adrenorezeptor.

16. Verwendung einer Verbindung als Wirkstoff zur Herstellung einer pharmazeutischen Zusammensetzung zur Behandlung von Muskelspasmen, einschliesslich hyperaktiver Blasenleerung, Diarrhoe, Diurese, Entzugssyndromen, Schmerzen, einschliesslich neuropathischen Schmerzen, neurodegenerativen Erkrankungen, einschliesslich optischer Neuropathie, spinaler Ischämie und Schlaganfall, Erinnerungs- und kognitiven Defiziten, Aufmerksamkeitsdefizitstörungen, Psychosen, einschliesslich manischer Störungen, Ängstlichkeit, Depression, Hypertension, kongestivem Herzversagen, Herzischämie und Nasenverstopfung ohne sedierende oder kardiovaskuläre Nebenwirkungen in einem Säugetier-Wirt, einschliesslich des Menschen, wobei der Wirkstoff mit adrenergischer Wirkung die biologische Eigenschaft hat, dass die Verbindung ein selektiver Agonist der α2B- oder α2B/α2C-Rezeptor-Subtypen ist, wobei diese Selektivität in einem Test gemessen wird, unter Verwendung von Zellen die natürlicherweise die einzelnen α2-Subtypen exprimieren oder in die einer der Subtypen eingeschleust wurde, wobei die Rezeptoren menschlich sind oder von einer Spezies, von der gezeigt wurde, dass sie eine ähnliche Pharmakologie hat, und wobei in dem Test die Wirksamkeit, bezogen auf eine Standardverbindung des Wirkstoffs auf den α2B- oder den α2C-Rezeptor-Subtyp in einer Messung mindestens 0,3 grösser als die Wirksamkeit, bezogen auf die Standardverbindung des Wirkstoffs auf den α2A-Rezeptor-Subtyp ist, und dessen Wirksamkeit auf den α2A-Rezeptor ≤ 0,4 ist, und/oder der Wirkstoff ist mindestens etwa zehnmal stärker am α2B- oder α2C-Rezeptor-Subtyp als am α2A-Rezeptor-Subtyp.

17. Verwendung einer Verbindung als Wirkstoff zur Herstellung einer pharmazeutischen Zusammensetzung zur Behandlung von Muskelspasmen, einschliesslich hyperaktiver Blasenleerung, Diarrhoe, Diurese, Entzugssyndromen, Schmerzen, einschliesslich neuropathischen Schmerzen, neurodegenerativen Erkrankungen, einschliesslich optischer Neuropathie, spinaler Ischämie und Schlaganfall, Erinnerungs- und kognitiven Defiziten, Aufmerksamkeitsdefizitstörungen, Psychosen, einschliesslich manischer Störungen, Ängstlichkeit, Depression, Hypertension, kongestivem Herzversagen, Herzischämie und Nasenverstopfung ohne sedierende oder kardiovaskuläre Nebenwirkungen in einem Säugetier-Wirt, einschliesslich des Menschen, wobei der Wirkstoff mit adrenergischer Wirkung die biologische Eigenschaft hat, dass die Verbindung ein selektiver Agonist der α2B- oder α2B/α2C-Rezeptor-Subtypen ist, wobei die Selektivität in einem RSAT-Test gemessen wird, bei dem die Aktivierung des α2Aund α2C-Rezeptor-Subtyps durch die Testverbindung mit Brimonidin verglichen wird, und der α2B-Rezeptor-Subtyp verglichen wird mit Oxymetazolin, und wobei die entsprechenden α2A-, α2B- und α2C-Rezeptor-Subtypen in NIH-3T3-Zellen exprimiert werden und wobei in dem Test die Wirksamkeit, bezogen auf Brimonidin des Wirkstoffs auf den α2C-Rezeptor-Subtyp oder die Wirksamkeit des Wirkstoffs, bezogen auf Oxymetacolin auf den α2B-Rezeptor-Subtyp in einer Messung mindestens 0,3 grösser als die Wirksamkeit des Wirkstoffs auf den α2A-Rezeptor-Subtyp, bezogen auf Brimonidin, und die Wirksamkeit auf den α2A-Rezeptor-Subtyp ≤ 0,4 ist, und/oder der Wirkstoff mindestens etwa zehnmal stärker am α2B- oder α2C-Rezeptor-Subtyp als am α2A-Rezeptor-Subtyp ist.

18. Verwendung gemäss Anspruch 1, 6, 11, 15, 16 oder 17, wobei der Wirkstoff mindestens hundertmal stärker auf den α2B- oder α2C-Adrenorezeptor-Subtyp wirkt als am α2A-Adrenorezeptor-Subtyp.

19. Verwendung gemäss Anspruch 15, wobei etwa 0,001 bis 5 Gew.% des Wirkstoffs topisch in täglichen oder zweimal täglichen Dosen an den Säugetier-Wirt verabreicht werden.

20. Verwendung gemäss Anspruch 20, wobei etwa 0,01 bis 3 Gew.% des Wirkstoffs topisch in täglichen oder zweimal täglichen Dosen an den Säugetier-Wirt verabreicht werden.

21. Verwendung gemäss Anspruch 15, wobei die Verbindung keine Wirkung auf den α2A-Adrenorezeptor-Subtyp hat.

22. Verwendung gemäss Anspruch 15, wobei die Verbindung keine Wirkung auf die α2A- und α2C-Adrenorezeptor-Subtypen hat.

## Revendications

1. Utilisation d'un composé ayant une activité adrénergique et qui est un agoniste sélectif du sous-type de récepteur adrénergique α2B ou du ou des sous-type(s) de récepteur adrénergique α2B/α2C
pour la fabrication d'une composition pharmaceutique pour le traitement ou la prévention du glaucome sans effets secondaires sédatifs ou cardiovasculaires chez un mammifère hôte, y compris l'homme,
dans laquelle le composé actif a
a) une efficacité par rapport à un agoniste entier standard qui est au moins approximativement 0,3 fois plus élevée vis à vis des sous-types de récepteur adrénergique α2B ou α2C que vis à vis du sous-type de récepteur adrénergique α2A et son efficacité vis à vis du sous-type de récepteur adrénergique α2A est ≤ 0,4 ou
b) est au moins dix fois plus puissant vis à vis du sous-type de récepteur adrénergique α2B ou α2C que vis à vis du sous-type de récepteur adrénergique α2A.

2. Utilisation selon la revendication 1, dans laquelle approximativement 0,001 % à 5 % en poids du composé actif sont administrés par voie topique à un mammifère hôte à raison d'une ou de deux doses journalières.

3. Utilisation selon la revendication 2, dans laquelle approximativement 0,01 % à 3 % en poids du composé actif sont administrés par voie topique à un mammifère hôte à raison d'une ou de deux doses journalières.

4. Utilisation selon la revendication 1, dans laquelle ledit composé est dépourvu d'activité vis à via du sous-type de récepteur adrénergique α2A.

5. Utilisation selon la revendication 1, dans laquelle ledit composé est dépourvu d'activité vis à vis des sous-types de récepteur adrénergique α2A et α2C.

6. Utilisation d'un composé ayant une activité adrénergique et qui est un agoniste sélectif du ou des sous-type (s) de récepteur adrénergique α2B ou α2B/α2C
comme principe actif pour la fabrication d'une composition pharmaceutique pour le traitement d'une tension intra-oculaire élevée sans effets secondaires sédatifs ou cardiovasculaires chez un mammifère hôte, y compris l'homme,
dans laquelle le composé actif a
a) une efficacité par rapport à un agoniste entier standard qui est au moins approximativement 0,3 fois plus élevée vis à vis des sous-types de récepteur adrénergique α2B ou α2C que vis à vis du sous-type de récepteur adrénergique α2A et son efficacité vis à vis du sous-type de récepteur adrénergique α2A est ≤ 0,4 ou
b) est au moins dix fois plus puissant vis à vis des sous-types de récepteur adrénergique α2B ou α2C que vis à vis du sous-type de récepteur adrénergique α2A.

7. Utilisation selon la revendication 6, dans laquelle approximativement 0,001 % à 5 % en poids du composé actif sont administrés par voie topique à un mammifère hôte à raison d'une ou de deux doses journalières.

8. Utilisation selon la revendication. 7, dans laquelle approximativement 0,01 % à 3 % en poids du composé actif sont administrés par voie topique à un mammifère hôte à raison d'une ou de deux doses journalières.

9. Utilisation selon la revendication 6, dans laquelle ledit composé est dépourvu d'activité vis à vis des sous-types de récepteur adrénergique α2A.

10. Utilisation selon la revendication 6, dans laquelle ledit composé est dépourvu d'activité vis à vis des sous-types de récepteurs adrénergiques α2A et α2C.

11. Utilisation d'un composé qui stimule de manière sélective le sous-type de récepteur adrénergique α2B ou un ou des sous-type(s) de récepteur adrénergique α2B/α2C
comme principe actif pour la fabrication d'une composition pharmaceutique pour le traitement d'un mammifère pour réduire sa tension intra-oculaire sans avoir d'effets secondaires sédatifs ou cardiovasculaires, dans laquelle le composé actif a
a) une efficacité par rapport à un agoniste entier standard qui est au moins approximativement 0,3 fois plus élevée vis à vis des sous-types de récepteur adrénergique α2B ou α2C que vis à vis du sous-type de récepteur adrénergique α2A et son efficacité vis à vis du sous-type de récepteur adrénergique α2A est ≤ 0,4 ou
b) est au moins dix fois plus puissant vis à vis du sous-type de récepteur adrénergique α2B ou α2C que vis à vis du sous-type de récepteur adrénergique α2A.

12. Utilisation selon la revendication 1, 6, ou 11, dans laquelle le composé actif est choisi dans le groupe comprenant les composés ayant la formule 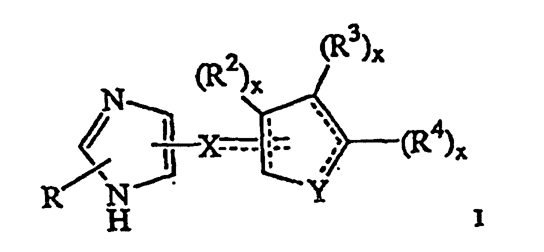 dans laquelle les lignes pointillées représentent des doubles liaisons optionnelles ; R est H ou un groupe alkyle en C₁ à C₄ ; X est S ou C(H)R¹, où R¹ est H ou un groupe alkyle en C₁ à C₄ ou R¹ est absent lorsque X est S ou lorsque la liaison entre X et le cycle représenté par 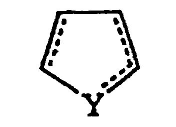 est une double liaison, Y est O, N, S, (CR¹ₓ)_{y}, dans laquelle y est un nombre entier valant de 1 à 3, -CH=CH-ou -Y¹CH₂-, dans laquelle Y¹ est O, N ou S ; x est un nombre entier valant 1 ou 2, dans laquelle x vaut 1 lorsque R², R³ ou R⁴ est lié à un atome de carbone insaturé et x vaut 2 lorsque R², R³ ou R⁴ est lié à un atome de carbone saturé, R² est H, un groupe alkyle en C₁ à C₄, un halogène, un hydroxy ou un groupe alcoxy en C₁ à C₄, ou, lorsqu'il est lié à un atome de carbone saturé, R² peut être un groupe oxo ; R³ et R⁴ sont chacun H, un groupe alkyle en C₁ à C₄, un hydroxy, un groupe alcoxy en C₁ à C₄, ou un groupe phényle ou, ensemble, sont - (C(R²)x) z- ; - Y¹ (C(R²)x) z'- ;
-Y¹ (C(R²)x) yY¹ - ; - (C(R²)x) - Y¹-(C(R²)x) - ;
- (C(R²)x) - Y¹ - (C(R²)x) - (C(R²)x) - et
- Y¹ - (C(R²)x)
- Y¹ - (C(R²)x) - dans lesquelles z est un nombre entier valant de 3 à 5, z' est un nombre entier valant de 2 à 4 et x et y sont tels que définis ci-dessus, et en outre, l'une ou l'autre extrémité de chacune de ces fractions divalentes peut être liée à R³ ou à R⁴ pour former une structure cyclique condensée 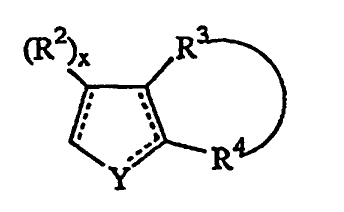 et le cycle ainsi formé peut être totalement insaturé, partiellement insaturé, ou totalement saturé à condition qu'un carbone du cycle n'ait pas plus de quatre valences, un azote n'en ait pas plus de trois et que O et S n'en aient pas plus de deux ; ou 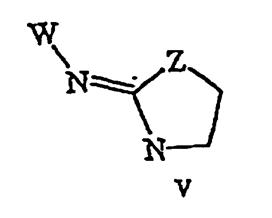 dans laquelle W est un radical bicyclique choisi dans le groupe comprenant 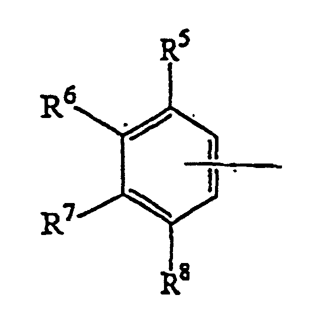 dans laquelle R⁵, R⁶, R⁷ et R⁸ sont choisis dans le groupe comprenant H et un groupe alkyle en C₁ à C₄ à condition qu'au moins l'un de R⁵ et de R⁶ ou bien de R⁶ et de R⁷ soit OC(R⁹)C(R⁹)N(R) pour former un cycle condensé avec 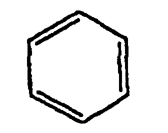 dans laquelle R⁹ est H, un groupe alkyle en C₁ à C₄, un groupe oxo,
et 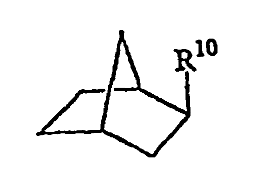 dans laquelle R¹⁰ est H, un groupe alkyle en C₁ à C₄, un groupe phényl ou un groupe phényle substitué par un groupe alkyle en C₁ à C₄, et Z est O ou NH.

13. Utilisation d'un composé comme principe actif pour la fabrication d'une composition pharmaceutique pour le traitement ou la prévention du glaucome chez un mammifère hôte, y compris chez l'homme, dans laquelle le composé, ayant une activité adrénergique, a la propriété biologique que ce composé est un agoniste sélectif du ou des sous-type(s) de récepteur α2B ou α2B/α2C
ladite sélectivité étant mesurée dans un essai utilisant des cellules qui expriment naturellement les sous-types α2 individuels ou dans lesquelles on a introduit l'un de ces sous-types, les récepteurs étant des récepteurs humains ou issus d'une espèce dont on a démontré qu'elle a une pharmacologie similaire, et dans lequel essai l'efficacité du composé actif vis à vis du sous-type de récepteur α2B ou α2C par rapport à un composé standard est évaluée comme étant au moins 0,3 fois plus élevée que l'efficacité, par rapport à un composé standard, du composé actif vis à vis du sous-type de récepteur α2A et son efficacité vis à vis du sous-type de récepteur α2A est ≤ 0,4, et/ou le composé actif est au moins approximativement 10 fois plus puissant vis à vis des sous-types de récepteur α2B ou α2C que vis à vis du sous-type de récepteur α2A.

14. Utilisation selon la revendication 13, dans laquelle approximativement 0,001 % à 5 % en poids du composé actif sont administrés quotidiennement par voie topique à un mammifère hôte.

15. Utilisation d'un composé comme principe actif pour la fabrication d'une composition pharmaceutique pour le traitement ou la prévention de la spasticité musculaire, y compris de la miction hyperactive, de la diarrhée, de la hausse de la diurèse, des syndromes de sevrage, de la douleur y compris de la douleur neuropathique, des maladies neurodégénératives y compris de la neuropathie optique, de l'ischémie spinale et des accidents cérébro-vasculaires, des déficits de la mémoire et de la cognition, des troubles déficitaires de l'attention, des psychoses y compris des troubles maniaques, de l'anxiété, de la dépression, de l'hypertension, de l'insuffisance cardiaque congestive, de l'ischémie cardiaque et de la congestion nasale, sans effets secondaires sédatifs ou cardiovasculaires, chez un mammifère hôte, y compris chez l'homme, dans laquelle ledit composé a une activité adrénergique et est un agoniste sélectif du ou des sous-type(s) de récepteur adrénergique α2B ou α2B/α2C, dans laquelle ledit composé a) a une efficacité relative par rapport à un agoniste entier standard qui est au moins approximativement 0,3 fois plus élevée vis à vis du sous-type de récepteur adrénergique α2B ou α2C que vis à vis du sous-type de récepteur adrénergique α2A et son efficacité vis à vis du sous-type de récepteur adrénergique α2A est ≤ 0,4 ou b) est au moins dix fois plus puissant vis à vis du sous-type de récepteur adrénergique α2B ou α2C que vis à vis du sous-type de récepteur adrénergique α2A.

16. Utilisation d'un composé comme principe actif pour la fabrication d'une composition pharmaceutique pour le traitement de la spasticité musculaire, y compris de la miction hyperactive, de la diarrhée, de la hausse de la diurèse, des syndromes de sevrage, de la douleur y compris de la neuropathie, de l'ischémie spinale et des accidents cérébro-vasculaires, des déficits de la mémoire et de la cognition, des troubles déficitaires de l'attention, des psychoses y compris des troubles maniaques, de l'anxiété, de la dépression, de l'hypertension, de l'insuffisance cardiaque congestive, de l'ischémie cardiaque et de la congestion nasale, sans effets secondaires sédatifs ou cardiovasculaires, chez un mammifère hôte, y compris chez l'homme, dans laquelle le composé actif ayant une activité adrénergique a la propriété biologique que ce composé est un agoniste sélectif du ou des sous-type(s) de récepteur α2B ou α2B/α2C ;
ladite sélectivité étant mesurée dans un essai utilisant des cellules qui expriment naturellement les sous-types α2 individuels ou dans lesquelles on a introduit l'un de ces sous-types, les récepteurs étant des récepteurs humains ou issus d'une espèce dont on a démontré qu'elle a une pharmacologie similaire, et dans lequel essai l'efficacité du composé actif vis à vis du sous-type de récepteur α2B ou α2C par rapport à un composé standard est évaluée comme étant au moins 0,3 fois plus élevée que l'efficacité, par rapport à un composé standard, du composé actif vis à vis du sous-type de récepteur α2A et son efficacité vis à vis du récepteur α2A est ≤ 0,4, et /ou le composé actif est au moins approximativement 10 fois plus puissant vis à vis des sous-types de récepteur α2B ou α2C que vis à vis du sous-type de récepteur α2A.

17. Utilisation d'un composé comme principe actif pour la fabrication d'une composition pharmaceutique pour le traitement de la spasticité musculaire, y compris de la miction hyperactive, de la diarrhée, de la hausse de la diurèse, des syndromes de sevrage, de la douleur y compris de la neuropathie, de l'ischémie spinale et des accidents cérébro-vasculaires, des déficits de la mémoire et de la cognition, des troubles déficitaires de l'attention, des psychoses y compris des troubles maniaques, de l'anxiété, de la dépression, de l'hypertension, de l'insuffisance cardiaque congestive, de l'ischémie cardiaque et de la congestion nasale, sans effets secondaires sédatifs ou cardiovasculaires, chez un mammifère hôte, y compris chez l'homme, dans laquelle le composé actif ayant une activité adrénergique a la propriété biologique que ce composé est un agoniste sélectif du ou des sous-type(s) de récepteur α2B ou α2B/α2C ;
ladite sélectivité étant mesurée dans un essai RSAT
dans lequel l'activation des sous-types de récepteur α2A et α2C par le composé de l'essai est comparée à la brimonidine et celle du sous-type de récepteur α2B est comparée à l'oxymétazoline et dans lequel les sous-types de récepteur α2A, α2B et α2C respectifs sont exprimés dans des cellules NIH-3T3, et dans lequel essai l'efficacité, par rapport à la brimonidine, du composé actif vis à vis du sous-type de récepteur α2C ou l'efficacité, par rapport à l'oxymétazoline, du composé actif vis à vis du sous-type de récepteur α2B est mesurée comme étant au moins 0,3 fois plus élevée que l'efficacité, par rapport à la brimonidine, du composé actif vis à vis du sous-type de récepteur α2A et son efficacité vis à vis du sous-type de récepteur α2A est s 0,4 et/ou le composé actif est au moins approximativement dix fois plus puissant vis à vis des sous-types de récepteur α2B ou α2C que vis à vis du sous-type de récepteur α2A.

18. Utilisation selon la revendication 1, 6, 11, 15, 16 ou 17, dans laquelle le composé actif est au moins cent fois plus puissant vis à vis du sous-type de récepteur α2B ou α2C que vis à vis du sous-type de récepteur α2A.

19. Utilisation selon la revendication 15, dans laquelle approximativement 0,001 % à 5 % en poids du composé actif sont administrés par voie topique à un mammifère hôte à raison d'une ou de deux doses journalières.

20. Utilisation selon la revendication 20, dans laquelle approximativement 0,01 % à 3 % en poids du composé actif sont administrés par voie topique à un mammifère hôte à raison d'une ou de deux doses journalières.

21. Utilisation selon la revendication 15, dans laquelle ledit composé est dépourvu d'activité vis à vis du sous-type de récepteur adrénergique α2A.

22. Utilisation selon la revendication 15, dans laquelle ledit composé est dépourvu d'activité vis à vis des sous-types de récepteur adrénergique α2A et α2C.
